# EUROPÄISCHE PATENTSCHRIFT

(11) **EP 2 903 668 B1**
(45) Veröffentlichungstag und Bekanntmachung des Hinweises auf die Patenterteilung: **25.04.2018**
(21) Anmeldenummer: 13795412.9
(22) Anmeldetag: 08.10.2013
(51) Int. Cl.: A61M 5/315, A61M 5/24, A61M 5/20, A61M 5/31

(54) **INJEKTIONSVORRICHTUNG ZUR AUFDOSIERUNG UND ZUR AUSSCHÜTTUNG EINER FESTGELEGTEN DOSIS EINES FLUIDEN PRODUKTS**
INJECTION DEVICE FOR DOSING AND ADMINISTRATION OF A SET DOSE OF A FLUID PRODUCT
DISPOSITIF D'INJECTION DE DOSAGE ET ADMINISTRATION D'UN DOSE FIXE DE PRODUIT FLUIDE

(30) Priorität: 08.10.2012 CH 18742012
(43) Veröffentlichungstag der Anmeldung: 12.08.2015
(73) Patentinhaber: TecPharma Licensing AG, 3401 Burgdorf (CH)
(72) Erfinder: MOSER, Ulrich, CH-3412 Heimiswil (CH); SCHRUL, Christian, CH-3400 Burgdorf (CH); TSCHIRREN, Markus, CH-3400 Burgdorf (CH); URBANEK, Leos, CH-3012 Bern (CH)
(86) Internationale Anmeldenummer: PCT/CH2013/000176
(87) Internationale Veröffentlichungsnummer: WO 2014/056117

(56) Entgegenhaltungen:
- EP-A1- 2 047 877
- WO-A1-01/60311
- WO-A1-01/72361
- WO-A1-2009/080775
- WO-A2-03/020347

## Beschreibung

Die Erfindung betrifft eine Injektionsvorrichtung zur Aufdosierung und zur Ausschüttung einer festgelegten Dosis eines fluiden Produkts gemäss dem Oberbegriff des Anspruchs 1.

Aus dem Stand der Technik sind Vorrichtungen bekannt, mit welchen eine festgelegte Dosis eines fluiden Produkts aufdosiert und ausgeschüttet werden können.

Unter einer festgelegten Dosis wird allgemein verstanden, dass der Benutzer eine Dosis nicht frei wählen kann, sondern dass die Dosis vorbestimmt beziehungsweise fixiert ist.

WO2009/080775A1 beschreibt eine Injektionsvorrichtung umfassend ein erstes hülsenförmiges Element und ein zweites hülsenförmiges Element, wobei die beiden Elemente derart miteinander gekoppelt sind, dass die Aufdosierbewegung und die Ausschüttbewegung in zwei Schritten durchgeführt werden können. Das erste und das zweite Element werden während der Aufdosierbewegung und der Ausschüttbewegung in einem Führungseingriff geführt. Während der Ausschüttbewegung wird eine Gewindestange zur Ausschüttung eines fluiden Produkts aufgrund einer drehfesten Verbindung zwischen der Gewindestange und dem zweiten Element in die Injektionsvorrichtung geschraubt.

Im Folgenden bedeutet die distale Position bei einer Injektionsvorrichtung zur Aufdosierung und zur Ausschüttung einer festgelegten Dosis eines fluiden Produkts die Anfangsposition der Injektionsvorrichtung, in welcher die Injektionsvorrichtung ausgeliefert wird, wobei ein Dosierelement oder ein Einstellknopf zur Einstellung und zur Ausschüttung einer festgelegten Dosis sich in seiner Anfangsposition befindet, und die proximale Position die Position, in welcher das Dosierelement oder der Einstellknopf in einer aufgezogenen und injektionsbereiten Position ist. Allgemeiner definiert distal eine Richtung zum nadelseitigen Ende der Injektionsvorrichfung oder darüber hinaus weisend und proximal zum einstellknopfseitigen Ende oder darüber hinaus weisend.

Es ist eine Aufgabe der Erfindung eine alternative Injektionsvorrichtung zur Aufdosierung und zur Ausschüttung einer festgelegten Dosis eines fluiden Produkts bereitzustellen.

Diese Aufgabe wird durch den Gegenstand des Anspruchs 1 gelöst.

Vorteilhafte Ausführungsformen der Erfindung sind in den abhängigen Ansprüchen definiert.

Die Erfindung betrifft eine Injektionsvorrichtung zur Aufdosierung und zur Ausschüttung einer festgelegten Dosis eines fluiden Produkts mit einem Dosierelement oder einem Einstellknopf zur Einstellung und zur Ausschüttung der festgelegten Dosis. Zur Einstellung oder zum Aufziehen der festgelegten Dosis kann das Dosierelement oder der Einstellknopf von einer distalen Position, einer Anfangsposition des Dosierelements oder des Einstellknopfs, in eine proximale Position, einer aufgezogenen Position des Dosierelements oder des Einstellknopfs, bewegt und zur Ausschüttung der eingestellten Dosis kann das Dosierelement oder der Einstellknopf in eine distale Position, einer ausgeschütteten Position des Dosierelements oder des Einstellknopfs, zurück bewegt werden. Das Dosierelement oder der Einstellknopf weist ein Führungselement auf. Ferner umfasst die Injektionsvorrichtung ein Halteelement mit einem Führungsgegenelement, wobei das Führungselement des Dosierelements oder des Einstellknopfs mit dem an einem Halteelement vorgesehenen Führungsgegenelement in einem Führungseingriff oder in einer Kulissenführung in Eingriff stehen kann. Der Führungseingriff oder die Kulissenführung kann derart ausgelegt sein, dass das Dosierelement oder der Einstellknopf während dem Einstellen oder Aufziehen der festgelegten Dosis in die proximale Richtung bewegbar ist. Vorzugsweise kann während der Aufdosierbewegung oder der Aufziehbewegung, welche eine Dreh- und/oder Axialbewegung sein kann, bei welcher das Dosierelement oder der Einstellknopf in die proximale Richtung bewegt wird, und während der Ausschüttbewegung, welche eine Dreh- und/oder Axialbewegung sein kann, bei welcher das Dosierelement oder der Einstellknopf in die distale Richtung bewegt wird, das Führungselement im Führungseingriff oder in der Kulissenführung mit dem Führungsgegenelement stehen. Das Führungselement des Dosierelements oder des Einstellknopfs kann somit in der Anfangsposition, in der aufgezogenen Position und in der ausgeschütteten Position des Dosierelements oder des Einstellknopfs im Führungseingriff oder in der Kulissenführung mit dem Führungsgegenelement des Halteelements stehen.

Das Injektionsgerät umfasst ferner ein Ausschüttelement zur Ausschüttung der festgelegten Dosis mit einer ersten und einer zweiten Ausnehmung. Das Halteelement weist ferner ein erstes und ein zweites Halteelement auf, wobei die erste Ausnehmung des Ausschüttelements zur Aufnahme des ersten Halteglieds des Halteelementes oder zumindest eines Teils des ersten Halteglieds des Halteelements und die zweite Ausnehmung des Ausschüttelements zur Aufnahme des zweiten Halteglieds oder zumindest eines Teils des zweiten Halteglieds des Halteelements geeignet sind. Vorteilhaft sind diese zusammenwirkenden Elemente jeweils paarweise oder mehrfach, insbesondere symmetrisch angeordnet vorgesehen.

Die erste Ausnehmung des Ausschüttelements wirkt mit dem ersten Halteglied oder mit zumindest einem Teil des ersten Halteglieds des Haltelements derart sowie die zweite Ausnehmung des Ausschüttelements wirkt mit dem zweiten Halteglied oder mit zumindest einem Teil des zweiten Halteglieds des Halteelements derart zusammen, dass in einer Anfangsposition des Dosierelements oder des Einstellknopfs, in welcher das Dosierelement oder der Einstellknopf in einer distalen Position ist, das erste Halteglied des Halteelements oder zumindest ein Teil des ersten Haltglieds des Halteelements mit der ersten Ausnehmung des Ausschüttelement in Eingriff steht, und in einer aufgezogenen Position des Dosierelements oder des Einstellknopfs, in welcher das Dosierelement oder der Einstellknopf in einer proximalen Position ist, das erste Halteglied oder zumindest ein Teil des ersten Halteglieds des Halteelements mit der ersten Ausnehmung des Ausschüttelements in Eingriff steht, und in einer ausgeschütteten Position des Dosierelements oder des Einstellknopfs, in welcher das Dosierelement oder der Einstellknopf in einer distalen Position ist, das zweite Halteglied oder zumindest ein Teil des zweiten Halteglieds des Halteelements mit der zweiten Ausnehmung des Ausschüttelements in Eingriff steht.

Diese Anordnung der Injektionsvorrichtung dient der Aufdosierung und der Ausschüttung der festgelegten Dosis eines fluiden Produkts aus der Injektionsvorrichtung. Besonders bevorzugt, kann die Anordnung der Injektionsvorrichtung derart ausgerichtet sein, dass aus der Injektionsvorrichtung mehrere, insbesondere gleiche festgelegte Dosen des fluiden Produkts ausgeschüttet werden können.

Bevorzugt kann die Injektionsvorrichtung ein Dosierelement und ein Einstellknopf umfassen, wobei das Dosierelement und der Einstellknopf mittelbar oder unmittelbar miteinander gekoppelt sind.

Bevorzugt weist das Dosierelement ferner eine Aussparung auf, welche derart ausgebildet ist, dass in der ausgeschütteten Position des Dosierelements oder des Einstellknopfs das erste Halteglied oder zumindest ein Teil des ersten Halteglieds des Halteelements mit der Aussparung des Dosierelements in Eingriff steht. Zudem kann das erste Halteglied oder zumindest ein Teil des ersten Halteglieds des Halteelements derart ausgebildet sein, dass es in der ausgeschütteten Position des Dosierelements oder des Einstellknopfs mit der ersten Ausnehmung des Ausschüttelements ausser Eingriff ist.

Alternativ kann das Dosierelement je mehrere Aussparungen umfassen. Bevorzugt können die Aussparungen in Umfangsrichtung und vorzugsweise in axialer Richtung versetzt zueinander in dem Dosierelement angeordnet sein. Vorteilhaft sind die Aussparungen paarweise oder mehrfach, insbesondere symmetrisch angeordnet. Der axiale Abstand aufeinander folgender Aussparungen können die festgelegte Dosis definiert.

Zudem kann vorzugsweise das Dosierelement in der Anfangsposition des Dosierelements oder des Einstellknopfs mit dem ersten Halteglied oder zumindest mit einem Teil des ersten Halteglieds des Halteelements in Gleitkontakt stehen. Durch diesen Kontakt kann sichergestellt werden, dass das erste Halteglied oder zumindest ein Teil des ersten Halteglieds des Halteelements in der Anfangsposition des Dosierelements oder des Einstellknopfs mit der ersten Ausnehmung des Ausschüttelements in Eingriff stehen kann. Die Gleitfläche des Dosierelements kann derart auf das erste Halteglied oder auf zumindest einen Teil des ersten Halteglieds des Halteelements wirken, dass das erste Halteglied oder zumindest ein Teil des ersten Halteglieds des Halteelements elastisch radial nach innen vorgespannt oder eingelenkt wird, wobei das erste Halteglied oder zumindest ein Teil des ersten Halteglieds des Haltelements mit der ersten Ausnehmung des Ausschüttelements in Eingriff gehalten werden kann. Alternativ kann die Gleitfläche des Dosierelements derart auf das erste Halteglied oder auf zumindest einen Teil des ersten Halteglieds des Halteelements wirken, dass das erste Halteglied oder zumindest ein Teil des ersten Halteglieds des Halteelements daran gehindert wird, sich elastisch radial nach aussen vorzuspannen oder auszulenken, sodass das erste Halteglied oder zumindest ein Teil des ersten Halteglieds des Halteelements mit der ersten Ausnehmung des Ausschüttelements in Eingriff verbleiben kann. Wie beschrieben sind die einzelnen an dieser Zusammenwirkung beteiligten Elemente vorteilhaft je mehrfach, insbesondere symmetrisch angeordnet vorgesehen, stehen sich insbesondere beispielsweise paarweise gegenüber.

Zudem kann das Dosierelement in der aufgezogenen Position des Dosierelements oder des Einstellknopfs mit dem ersten Halteglied oder mit zumindest einem Teil des ersten Halteglieds des Halteelements in Gleitkontakt sein. Dabei kann die Gleitfläche des Dosierelements analog zu der oben erwähnten Anfangsposition des Dosierelements oder des Einstellknopfs derart auf das erste Halteglied oder auf zumindest einen Teil des ersten Halteglieds des Halteelements wirken, dass das erste Halteglied oder zumindest ein Teil des ersten Halteglieds des Haltelements elastisch radial nach innen vorgespannt oder eingelenkt wird oder daran gehindert wird, sich elastisch nach radial nach aussen vorzuspannen oder auszulenken. Somit wird sichergestellt, dass während dem Aufziehvorgang der Dosis, um die festgelegte Dosis einzustellen, das erste Halteglied oder zumindest ein Teil des ersten Halteglieds des Halteelements mit dem Ausschüttelement in Eingriff verbleiben kann, sodass zumindest keine relative axiale Bewegung zwischen dem ersten Halteelement und dem Ausschüttelement stattfinden kann.

Das Dosierelement oder der Einstellknopf kann relativ zu dem Halteelement drehbar sein. Das Führungselement des Dosierelements oder des Einstellknopfs steht in Eingriff, insbesondere in einem Führungseingriff oder in einer Kulissenführung mit dem Führungsgegenelement des Halteelements, wobei das Dosierelement oder der Einstellknopf relativ zu dem Halteelement drehbar ist. Besonders bevorzugt kann das Dosierelement oder der Einstellknopf durch diese relative Drehbewegung relativ zu dem Halteelement in die proximale Richtung bewegt werden, sodass eine rotative und axiale Relativbewegung zwischen dem Dosierelement oder dem Einstellknopf und dem Halteelement vollzogen werden kann.

Ferner umfasst die Injektionsvorrichtung ein Antriebsmittel welches derart ausgebildet ist, dass es auf das Ausschüttelement wirkt Das Antriebsmittel ist eine vorgespannte Feder. Das heisst, dass der Benutzer die Injektionsvorrichtung nicht vorspannen muss, sondem die Injektionsvorrichtung mit einem vorgespannten Antriebsmittel, insbesondere der Feder ausgeliefert bekommt.

Das Ausschüttelement kann hülsenförmig ausgebildet sein. Zudem kann das vorgespannte Antriebsmittel von dem hülsenförmigen Ausschüttelement zumindest teilweise aufgenommen sein. Dadurch kann die Bauweise der Injektionsvorrichtung verkleinert werden und/oder das Antriebsmittel kann geführt und/oder stabilisiert werden.

Die erste und die zweite Ausnehmung des Ausschüttelements sind derart relativ zu dem ersten und dem zweiten Halteglied oder zu zumindest einem Teil des ersten oder des zweiten Halteglieds des Halteelements angeordnet, dass die festgelegte Dosis aus der Vorrichtung ausschüttbar ist. Die relative Anordnung ist insbesondere derart ausgelegt, dass mehrere immer gleiche festgelegte Dosen aus der Injektionsvorrichtung ausschüttbar sind.

Besonders bevorzugt ist die erste und die zweite Ausnehmung des Ausschüttelements in Umfangsrichtung und vorzugsweise in axialer Richtung versetzt zueinander angeordnet. Die Aussparungen der ersten und der zweiten Ausnehmung sind bevorzugt teilweise auf gleicher axialer Höhe angeordnet derart, dass sie sich in Umfangsrichtung überlappen. Diese Überlappung der beiden Ausnehmungen kann derart ausgelegt sein, dass sowohl das erste wie auch das zweite Halteglied oder zumindest ein Teil des ersten oder des zweiten Halteglieds des Halteelements in zumindest einer relativen Position des Dosierelements oder des Einstellknopfs zu dem Halteelement in den entsprechenden Ausnehmungen des Ausschüttelements in Eingriff sein können. Wie beschrieben sind die einzelnen an dieser Zusammenwirkung beteiligten Elemente vorteilhaft je mehrfach, insbesondere symmetrisch angeordnet vorgesehen, stehen sich insbesondere beispielsweise paarweise gegenüber.

Alternativ können die erste und die zweite Ausnehmung des Ausschüttelements in Umfangsrichtung versetzt zueinander angeordnet sein und in axialer Richtung auf gleicher Höhe vorgesehen sein.

Alternativ können das erste und das zweite Halteglied oder zumindest ein Teil des ersten oder des zweiten Halteglieds des Halteelements oder die Aussparung des Dosierelements oder die mehreren Aussparungen des Dosierelements derart ausgelegt sein, dass in zumindest einer relativen Position des Dosierelements oder des Einstellknopfs zu der entsprechenden Ausnehmung des Ausschüttelements, sowohl das erste wie auch das zweite Halteglied oder zumindest ein Teil des ersten oder des zweiten Halteglieds des Halteelements in die entsprechende Ausnehmung des Ausschüttelements in Eingriff stehen. Wie beschrieben sind die einzelnen an dieser Zusammenwirkung beteiligten Elemente vorteilhaft je mehrfach, insbesondere symmetrisch angeordnet vorgesehen, stehen sich insbesondere beispielsweise paarweise gegenüber.

Das Halteelement kann hülsenförmig ausgebildet sein. Das Halteelement kann zumindest teilweise das Ausschüttelement mit dem darin angeordneten Antriebsmittel, insbesondere der Feder aufnehmen.

Das erste und das zweite Halteglied oder zumindest ein Teil des ersten oder des zweiten Halteglieds des Halteelements können auf gleicher axialen Höhe und in Umfangsrichtung versetzt zueinander angeordnet sein. Alternativ können das erste und das zweite Halteglied oder zumindest ein Teil des ersten oder des zweiten Halteglieds des Halteelements in Umfangsrichtung und in axialer Richtung versetzt zueinander angeordnet sein. Zudem können die Halteelemente, die Halteglieder oder zumindest ein Teil der Halteglieder elastisch ausgebildet sein. Besonders bevorzugt können die Halteelemente, Halteglieder oder zumindest ein Teil der Halteglieder radial nach innen und/oder radial nach aussen bewegbar, insbesondere nach radial innen und/oder aussen elastisch spannbar oder lenkbar sein. Die Halteelemente, Halteglieder oder zumindest ein Teil der Halteglieder sind derart ausgebildet, dass sie in die entsprechende Ausnehmung des Ausschüttelements eingreifbar sind und eine axiale Verschiebung, insbesondere in distaler Richtung des Ausschüttelements verhindern können.

Die Halteelemente, die Halteglieder oder zumindest ein Teil der Halteglieder können als Schnapparm oder Schnappzunge ausgebildet sein. Die Schnapparme oder Schnappzungen sind derart ausgelegt, dass sie gleichzeitig oder alternierend in die entsprechende Ausnehmung des Ausschüttelements eingreifen können, derart dass eine axiale Verschiebung, insbesondere in die distale Richtung des Ausschüttelements ermöglicht werden kann. Insbesondere können die Schnapparme oder die Schnappzungen einen Schnapphaken oder eine Schnappnocke oder einen sonstigen Vorsprung umfassen, welche mit den entsprechenden Ausnehmungen des Ausschüttelements in Eingriff kommen können.

Zudem kann das Führungselement des Dosierelements oder des Einstellknopfs als Führungsnocke und das Führungsgegenelement des Halteelements als Kulissenführung ausgebildet sein. Alternativ kann das Führungselement des Dosierelements oder des Einstellknopfs als Kulissenführung und das Führungsgegenelement des Halteelements als Führungsnocke ausgebildet sein.

Die Führungsnocke und die Kulissenführung sind derart im Führungseingriff oder in Eingriff, dass die Aussparung des Dosierelements in der Anfangsposition, in der aufgezogenen Position und in der ausgeschütteten Position des Dosiereleements oder des Einstellknopfs relativ zu dem ersten und dem zweiten Halteglied oder zu einem Teil des ersten oder des zweiten Halteglieds des Halteelements derart ausgelegt ist, dass die festgelegte Dosis ausgeschüttet werden kann. Dazu kann in der entsprechenden Position des Dosierelements die Aussparung des Dosierelements derart mit einem der Halteglieder oder mit zumindest einem Teil der Halteglieder des Halteelements zusammenwirken, dass das eine Halteglied oder zumindest eine Teil des Halteglieds des Halteelements zumindest teilweise in die Aussparung oder in die zumindest eine Aussparung ragen kann und sich elastisch entspannen oder elastisch spannen kann oder elastisch in die Aussparung oder in die zumindest eine Aussparung radial ein- oder auslenken kann, wobei das andere Halteglied oder zumindest eine Teil des anderen Halteglieds des Haltelements gehindert wird, in die Aussparung oder in die zumindest eine Aussparung zu ragen. Alternativ kann das andere Halteglied oder zumindest eine Teil des anderen Halteglieds des Halteelements analog zu dem ersten Halteglied oder zu zumindest einem Teil des ersten Halteglieds des Halteelements in die Aussparung ragen.

Die Injektionsvorrichtung kann ferner ein Gehäuse umfassen, welches mittelbar oder unmittelbar mit dem Halteelement axial und rotativ fest verbunden ist. Das hülsenförmige Gehäuse kann das hülsenförmige Halteelement zumindest teilweise aufnehmen, wobei zwischen dem Gehäuse und dem Halteelement das hülsenförmige Dosierelement angeordnet sein kann. Das Dosierelement kann relativ zu dem Gehäuse und dem Halteelement rotativ und vorzugsweise axial bewegbar sein. Durch eine rotative und vorzugsweise axiale Bewegung des Dosierelements relativ zu dem Halteelement können das erste und/oder das zweite Halteglied oder zumindest ein Teil des ersten und/oder des zweiten Halteglieds derart in die Aussparung oder in die entsprechende Aussparung des Dosierelement gelangen, dass das erste und/oder zweite Halteelement oder zumindest ein Teil des ersten und/oder zweiten Halteelements entsprechend aus dem Eingriff mit der ersten und/oder zweiten Ausnehmung des Ausschüttelements gelangen und das Ausschüttelement in die distale Richtung bewegbar ist, um aus der Injektionsvorrichtung die festgelegte Dosis abzugeben. Alternativ kann nur durch eine rotative Bewegung des Dosierelements relativ zu dem Halteelement die entsprechende festgelegte Dosis abgegeben werden.

Alternativ kann das Dosierelement mittelbar oder unmittelbar axial fest zu dem Gehäuse und/oder dem Halteelement angeordnet sein. Ferner kann das Dosierelement durch einen Rückdrehsperrmechanismus nur in eine Richtung relativ zu dem Gehäuse und/oder dem Halteelement drehbar sein. Durch eine rotative Bewegung des Dosierelements relativ zu dem Halteelement können folglich das erste und/oder das zweite Halteglied oder zumindest ein Teil des ersten und/oder zweiten Halteglieds derart in die Aussparung des Dosierelements gelangen, dass das erste und/oder zweite Halteglied oder zumindest ein Teil des ersten und/oder zweiten Halteglieds des Halteelements entsprechend aus dem Eingriff mit der ersten und/oder zweiten Ausnehmung des Ausschüttelements gelangt. Dadurch kann erzielt werden, dass das Ausschüttelement in die distale Richtung bewegbar ist, um aus der Injektionsvorrichtung die festgelegte Dosis abzugeben.

Die Injektionsvorrichtung weist ferner eine Karpule auf, in welcher sich das fluide abzugebende Produkt befindet. Die Karpule kann zudem von einem Karpulenhalter aufgenommen sein. Die Karpule oder der Karpulenhalter kann mittelbar oder unmittelbar axial- und drehfest mit dem Gehäuse oder mit dem Halteelement verbunden sein. Die Karpule oder der Karpulenhalter sind vorzugsweise an dem distalen Ende des Gehäuses oder an dem distalen Ende des Halteelements angebracht. Um das fluide Produkt abzugeben, kann an die Karpule oder an den Karpulenhalter eine Injektionsnadel über eine Nadelverbindung, insbesondere eine Drehverbindung, Bajonettverbindung oder Schnappverbindung in Gestalt eines Verbindungselements an der Karpule oder an dem Karpulenhalter und einen Verbindungsgegenelements an der Injektionsnadel angebracht werden. In der Karpule ist ein Stopfen verschiebbar aufgenommen, sodass das fluide Produkt aus der Karpule durch die Injektionsnadel ausgeschüttet werden kann. Das Ausschüttelement kann derart auf den Stopfen wirken, dass der Stopfen relativ zu einer Wandung der Karpule bewegt werden kann. Das Ausschüttelement kann direkt oder indirekt, insbesondere über ein Verschiebeelement auf den Stopfen wirken.

Die Injektionsvorrichtung kann einen Einstellknopf umfassen. Der Einstellknopf kann zum Einstellen und/oder Ausschütten der festgelegten Dosis aus der Injektionsvorrichtung dienen. Der Einstellknopf kann derart mit dem Dosierelement mittelbar oder unmittelbar gekoppelt sein, dass durch die Betätigung des Einstellknopfs das Dosierelement gesteuert werden kann. Zudem kann die Injektionsvorrichtung ein Mechanismus aufweisen, mit welchem eine Dosiskorrektur, insbesondere ein Rückwärtsdrehen entgegen einer Aufziehrichtung, verhindert werden kann.

Der Einstellknopf kann mit dem Dosierelement mittelbar oder unmittelbar axial- und drehfest verbunden sein und zumindest teilweise ein Gehäuse der Injektionsvorrichtung umgeben, sodass der Benutzer den Einstellknopf greifen kann. Zudem kann der Einstellknopf eine Greifvorrichtung umfassen, welche dazu dient den Einstellknopf besser zu greifen. Durch Drehung des Einstellknopfs durch den Benutzer relativ zu dem Gehäuse kann die rotative Bewegung des Einstellknopfs auf das Dosierelement übertragen werden. Zudem kann während dem Einstellen oder dem Aufziehen der festgelegten Dosis aufgrund eines Führungseingriffs oder einer Kulissenführung zwischen dem Dosierelement und dem gehäusefesten Halteelement und der mittelbaren oder unmittelbaren axial festen Verbindung zwischen dem Einstellknopf und dem Dosierelement das Dosierelement und der Einstellknopf relativ zu dem Gehäuse in die proximale Richtung bewegt werden.

Alternativ kann der Einstellknopf mit dem Gehäuse mittelbar oder unmittelbar axialfest und relativ zu dem Gehäuse drehbar verbunden sein. Der Einstellknopf kann mit dem Dosierelement mittelbar oder unmittelbar drehfest verbunden sein, wobei das Dosierelement relativ zu dem Einstellknopf axial bewegbar ist. Durch Drehung des Einstellknopfs durch den Benutzer relativ zu dem Gehäuse kann somit ebenfalls die rotative Bewegung des Einstellknopfs auf das Dosierelement übertragen werden. Somit kann bei einer relativen Drehbewegung zwischen dem Einstellknopf und dem Gehäuse während dem Einstellen oder Aufziehen der festgelegten Dosis und aufgrund eines Führungseingriffs und einer Kulissenführung zwischen dem Dosierelement und dem Halteelement das Dosierelement relativ zu dem Gehäuse in die proximale Richtung gelangen.

Alternativ kann der Einstellknopf mit dem Dosierelement mittelbar oder unmittelbar drehfest verbunden sein, wobei der Einstellknopf relativ zu dem Dosierelement axial bewegbar ist. Zudem kann der Einstellknopf relativ zu dem Gehäuse drehbar verbunden sein. Durch eine relative Drehbewegung des Einstellknopfs durch den Benutzer kann folglich während dem Einstellen oder Aufziehen der festgelegten Dosis und aufgrund eines Führungseingriffs oder einer Kulissenführung zwischen dem Einstellknopf und dem Halteelement der Einstellknopf relativ zu dem Gehäuse in die proximale Richtung bewegt werden.

Ferner kann die Injektionsvorrichtung eine Anzeigevorrichtung umfassen, welche die Anfangsposition, die aufgezogene Position und/oder die ausgeschüttete Position des Dosierelements oder des Einstellknopfs anzeigen kann. Die Anzeigevorrichtung kann als eine visuelle, akustische und/oder taktile Anzeige ausgebildet sein. Die visuelle Anzeigevorrichtung kann eine Markierung und/oder eine Anzeigeziffer und/oder einen Zwischenraum zwischen zwei Anzeigeziffern umfassen.

Dazu kann die Injektionsvorrichtung eine Anzeigehülse mit einer Anzeigevorrichtung aufweisen. Die Anzeigehülse kann über eine Gewindeverbindung mit dem Gehäuse und über eine drehfeste Verbindung mit dem Dosierelement oder dem Einstellknopf mittelbar oder unmittelbar verbunden sein. Somit kann die Anzeigehülse während der Aufziehbewegung oder der Drehbewegung des Dosierelements oder des Einstellknopfs relativ zu dem Gehäuse eine rotative und axiale Bewegung durchführen. Bevorzugt kann die Anzeigehülse während der Aufziehbewegung oder der Drehbewegung des Dosierelements oder des Einstellknopfs in die distale Richtung geschraubt werden. Zudem kann ein Rückdrehsperrmechanismus vorgesehen sein, welcher dazu dient, dass das Dosierelement oder der Einstellknopf nur in eine Richtung relativ zu dem Gehäuse drehbar ist. Dazu kann am Dosierelement, am Einstellknopf oder an der Anzeigehülse eine Rückdrehsicherung oder zumindest ein Teil einer Rückdrehsicherung vorgesehen sein, welche mit einem anderen Teil der Injektionsvorrichtung zusammenwirken kann.

Alternativ kann eine Karpule oder eine Karpulenhülse eine Anzeigevorrichtung aufweisen, welche die relative Position des Stopfens bei der Ausschüttung des fluiden Produkts anzeigen kann. Während der Ausschüttung wirkt ein Ausschüttelement auf einen Stopfen in der Karpule, wodurch der Stopfen relativ zu der Wandung der Karpule axial bewegbar ist. Durch eine zumindest teilweise transparente Karpule und/oder Karpulenhalter kann somit die relative Position des Stopfens festgestellt werden, wobei die Anzeigevorrichtung auf der Karpule oder der Karpulenhülse diese relative Position des Stopfens, insbesondere visuell anzeigen kann.

Alternativ kann die Anzeigehülse mit der Anzeigevorrichtung eine Gewindeverbindung zu dem Gehäuse und eine andere Gewindeverbindung zu einem Dosierelement aufweisen. Bevorzugt sind die beiden Gewindeverbindung gegenläufig und mit unterschiedlichen Gewindesteigungen ausgebildet. Möglich wäre auch, wenn die beiden Gewindeverbindungen gleichläufig und mit unterschiedlichen Gewindesteigungen ausgebildet sind. Die Anzeigehülse kann über ein Innengewinde mit einem Aussengewinde des Dosierelements und über ein Aussengewinde mit einem Innengewinde des Gehäuses verbunden sein. Bevorzugt kann die Gewindesteigung der Gewindeverbindung zwischen der Anzeigehülse und dem Gehäuse flacher ausgebildet sein, als die Gewindesteigung der Gewindeverbindung zwischen der Anzeigehülse und dem Dosierelement. Besonders bevorzugt kann die Gewindeverbindung zwischen der Anzeigehülse und dem Dosierelement nicht selbsthemmend und die Gewindeverbindung zwischen der Anzeigehülse und dem Gehäuse selbsthemmend oder nicht selbsthemmend ausgebildet sein. Durch Drehung des Dosierelements während der Dosierbewegung, wobei das Dosierelement alternativ über ein mit dem Dosierelement drehfest verbundenen Einstellknopf durch den Benutzer gedreht werden kann, wird die Anzeigehülse über die beiden Gewindeverbindungen in die distale Richtung relativ zu dem Gehäuse axial verschoben. Bevorzugt kann die Steigung eines Führungsgegenelements, insbesondere der Kulissenführung des Halteelements kleiner sein, als die Steigung der Gewindeverbindung zwischen dem Dosierelement und der Anzeigehülse. Somit kann beim Einstellen oder Aufziehen der festgelegten Dosis die Anzeigehülse relativ zu dem Gehäuse in die distale Richtung geschraubt werden, wobei eine rotative und axiale Bewegung der Anzeigehülse relativ zu dem Gehäuse durchgeführt werden kann. Beim Ausschütten der festgelegten Dosis kann das Dosierelement, welches alternativ mit dem Einstellknopf axialfest verbunden ist, relativ zu dem Gehäuse in die axiale Richtung, insbesondere in die distale Richtung verschoben werden. Die Anzeigehülse kann somit aufgrund der beiden Gewindeverbindungen und dem axialen Antrieb des Dosierelements relativ zu dem Gehäuse rotativ und axial in die distale Richtung bewegt werden. Bevorzugt kann bei der Einstellung oder bei dem Aufziehen der festgelegten Dosis die Anzeigehülse den gleichen Drehwinkel oder die gleiche axiale Verschiebung wie bei der Ausschüttung der festgelegten Dosis vollführen.

Alternativ kann die Anzeigehülse mit der Anzeigevorrichtung eine Gewindeverbindung zu dem Gehäuse und eine Gewindeverbindung zu dem Einstellknopf aufweisen. Die Anzeigehülse kann ein Aussengewinde umfassen, welches im Gewindeeingriff mit einem Innengewinde des Gehäuses steht, und ein Innengewinde aufweisen, welches im Gewindeeingriff mit einem Aussengewinde des Einstellknopfs steht Bevorzugt sind die beiden Gewindeverbindung gegenläufig und mit unterschiedlichen Gewindesteigungen ausgebildet. Möglich wäre auch, wenn die beiden Gewindeverbindungen gleichläufig und mit unterschiedlichen Gewindesteigungen ausgebildet sind. Bevorzugt ist die Gewindesteigung der Gewindeverbindung zwischen der Anzeigehülse und dem Einstellknopf steiler ausgebildet als die Gewindesteigung der Gewindeverbindung zwischen der Anzeigehülse und dem Gehäuse. Besonders bevorzugt kann die Gewindeverbindung zwischen der Anzeigehülse und dem Einstellknopf nicht selbsthemmend und die Gewindeverbindung zwischen der Anzeigehülse und dem Gehäuse selbsthemmend oder nicht selbsthemmend ausgebildet sein. Zwischen der Anzeigehülse und dem Dosierelement kann eine mittelbare oder unmittelbare drehfeste Verbindung vorgesehen sein. Während der Ausschüttbewegung, wobei der Einstellknopf axial in die distale Richtung bewegt werden kann, kann die Anzeigehülse aufgrund der axialen Kraft, welche auf die Anzeigehülse wirkt, rotativ in dem Gehäuse in die distale Richtung bewegt werden, wobei das Dosierelement über die drehfeste Verbindung mitgedreht werden kann. Bevorzugt kann die Steigung eines Führungsgegenelements, insbesondere der Kulissenführung des Halteelements gleich gross sein, wie die Steigung der Gewindeverbindung zwischen der Anzeigehülse und dem Einstellknopf.

Ferner kann die Injektionsvorrichtung einen Mechanismus umfassen, welcher das Ende einer Abgabe der festgelegten Dosis anzeigen kann. Die Anzeigevorrichtung zur Anzeige des Endes der Abgabe der festgelegten Dosis kann visuell, akustisch und/oder taktil ausgebildet sind. Die visuelle Anzeigevorrichtung kann eine Markierung und/oder eine Anzeigeziffer und/oder einen Zwischenraum zwischen zwei Anzeigeziffern umfassen.

Dazu kann die Injektionsvorrichtung ein weiteres Antriebsmittel, insbesondere eine vorzugsweise vorgespannte Feder umfassen. Diese Feder kann zwischen der Karpule und einem proximalen Spannelement oder zwischen einem distalen und proximalen Spannelement vorgespannt sein. Das distale Spannelement kann auf der Karpule abgestützt sein. Die Feder kann unmittelbar oder über das distale Spannelement derart auf die Karpule wirken, dass die Karpule in die distale Richtung gegen einen mit einem Gehäuse mittelbar oder unmittelbar axiale- und drehfest verbundenen Karpulenhalter gedrückt werden kann, um ein axiales Spiel zwischen der Karpule und dem Karpulenhalter zu verhindern. Das proximale Spannelement oder alternativ das distale und das proximale Spannelement können gleitbar auf dem Ausschüttelement gelagert sein. In der Anfangsposition des Dosierelements oder des Einstellelements kann sich das proximale Spannelement mit einer Stirnseite eines Anschlags gegen einen Anschlag des Halteelements abstützen, wobei ein radial nach innen ragenden Schnapper, welcher an dem proximalen Spannelement angebracht ist, in eine distale Ausnehmung des Ausschüttelements ragt. Während dem Ausschüttvorgang der festgelegten Dosis aus der Injektionsvorrichtung bewegt sich das Ausschüttelement in die distale Richtung, wobei eine Stirnseite des Schnappers des proximalen Spannelements gegen eine Stirnseite eines Verbindungsstegs zweier in axialer Richtung versetzt zueinander angeordneten Ausnehmungen des Ausschüttelements stossen kann. Dieser Anschlagkontakt kann bewirken, dass das proximale Spannelement in die distale Richtung bewegt werden kann, wobei die Feder gespannt oder weiter gespannt werden kann. Das proximale Spannelement kann so weit in die distale Richtung bewegt werden, bis eine auf einer Haltehülse vorgesehene Nut oder bis ein auf einer Haltehülse vorgesehener Absatz in die Flucht von dem Schnapper des proximalen Spannelements gelangt und der Schnapper elastisch radial nach aussen ausgelenkt werden kann, wobei die Feder aufgrund eines Anschlags zwischen einer Stirnseite der Nut oder des Absatzes und einer Stirnseite des Schnappers des proximalen Spannelements vorgespannt gehalten wird. Bei weiterem Ausschüttvorgang der festgelegten Dosis aus der Injektionsvorrichtung gleitet der Verbindungsstegs des Ausschüttelements, der zwischen zwei in axialer Richtung versetzt zueinander angeordneten Ausnehmungen des Ausschüttelements, nämlich der distalen und einer proximalen Ausnehmung, vorgesehen ist, entlang des Schnappers des proximalen Spannelements bis der Schnapper des proximalen Spannelements in die proximale Ausnehmung einschnappt. Am Ende des Ausschüttvorgangs der festgelegten Dosis aus der Injektionsvorrichtung entspannt sich die Feder zwischen der Karpule und dem proximalen Spannelement oder zwischen dem distalen und proximalen Spannelement, wobei aufgrund der Federkraft der Feder, welche auf das proximale Spannelement wirkt, das proximale Spannelement mit der Stirnseite des Anschlags gegen die Stirnseite des Anschlags des Halteelements anstossen kann. Dieser Anschlagkontakt kann ein akustisches Signal, insbesondere eine Klick-Geräusch auslösen.

Alternativ kann ein akustische Signal, insbesondere ein Klick-Geräusch auch durch das elastische radial nach innen Einlenken oder Entspannen des Schnappers des proximalen Spannelements in die proximale Ausnehmung erzeugt werden. Dabei kann der Schnapper des proximalen Spannelement gegen einen Verbindungssteg des Ausschüttelements, der zwischen zwei in axialer Richtung versetzt zueinander angeordneten Ausnehmungen des Ausschüttelements vorgesehen ist, anschlagen, sodass ein akustisches Signal, insbesondere das Klick-Geräusch ausgelöst werden kann.

Alternativ kann ein weiteres Antriebsmittel, insbesondere eine vorzugsweise vorgespannte Feder, die distal an einem gehäusefesten Halteelement und proximal an einem proximalen Spannelement abgestützt ist, vorgesehen sein, um das Ende einer Abgabe der festgelegten Dosis anzuzeigen. Das proximale Spannelement ist drehfest mit dem Halteelement verbunden, wobei das proximale Spannelement auf dem Halteelement axial verschiebbar angeordnet ist. Das proximale Spannelement wird durch die Federkraft gegen eine distale Stirnseite des Dosierelements vorgespannt. Eine proximale Stirnseite des proximalen Spannelements und die distale Stirnseite des Dosierelements sind komplementär zueinander sägezahnförmig ausgebildet und können ineinander greifen. In der Anfangsposition des Dosierelements oder des Einstellknopfs sind die beiden sägezahnförmigen Anschlagsflächen des proximale Spannelements und des Dosierelements miteinander in Eingriff. Während dem Ausschüttvorgang der festgelegten Dosis aus der Injektionsvorrichtung kann das Dosierelement relativ zu dem Halteelement drehen. Aufgrund des sägezahnförmigen Eingriffs zwischen dem proximalen Spannelement und dem Dosierelement und der mittelbaren oder unmittelbaren drehfesten Verbindung zwischen dem proximalen Spannelement und dem Halteelement kann das proximale Spannelement in die distale Richtung bewegt werden, sodass die Feder gespannt oder weiter gespannt wird. Ein Halteglied des Halteelements gelangt durch die relative Drehbewegung des Dosierelements in eine Aussparung des Dosierelements, wobei eine axiale Bewegung in die proximale Richtung des proximalen Spannelements durch den Anschlagkontakt zwischen einer proximalen Stirnfläche des proximalen Spannelements und einer Stirnfläche des Halteglieds verhindert werden kann. Das Halteglied des Halteelements ist aufgrund des axialen Drucks, welcher von der Feder in dem Ausschüttelement über den Verbindungssteg des Ausschüttelements, der zwischen zwei in axialer Richtung versetzt zueinander angeordneten Ausnehmungen des Ausschüttelements, nämlich einer distalen und einer proximalen Ausnehmung des Ausschüttelements, auf das Halteglied des Halteelements wirkt, radial nach aussen in die Aussparung des Dosierelements elastisch vorgespannt oder ausgelenkt. Bei weiterem Ausschüttvorgang der festgelegten Dosis aus der Injektionsvorrichtung gleitet der Verbindungsstegs des Ausschüttelements entlang des Halteglieds des Halteelements, bis das Halteglied des Halteelements in die proximale Ausnehmung gelangt und sich entspannt oder elastisch einlenkt. Das proximale Spannelement kann durch die Kraft der vorgespannten Feder mit der sägezahnförmigen Anschlagsfläche gegen die sägezahnförmige Anschlagsfläche des Dosierelements anstossen. Dieser Anschlag kann ein akustisches Signal, insbesondere eine Klick-Geräusch auslösen.

Die Injektionsvorrichtung kann zudem einen Mechanismus vorsehen, der ein Einstellen einer weiteren Dosis verhindern kann, wenn die letzte Dosis ausgeschüttet worden ist.

Dazu kann eine Anzeigehülse der Injektionsvorrichtung einen Anschlag aufweisen, welcher gegen einen im Gehäuse vorgesehenen Anschlag anstösst. Somit kann durch einen axialen und/oder radialen Anschlagkontakt zwischen der Anzeigehülse und dem Gehäuse das Einstellen oder Aufziehen einer weiteren Dosis verhindert werden, wenn die letzte Dosis ausgeschüttet worden ist.

Alternativ kann ein axialer und/oder radialer Anschlagkontakt zwischen einem Gehäuse und einem Dosierelement oder einem Einstellknopf vorgesehen sein, welcher das Einstellen oder Aufziehen einer weiteren Dosis verhindert, wenn die letzte Dosis ausgeschüttet worden ist.

Alternativ kann ein Halteglied oder zumindest ein Teil des Halteglieds des Halteelements in eine Ausnehmung des Dosierelements elastisch vorgespannt ragen oder elastisch radial nach aussen in eine Ausnehmung ausgelenkt werden, wobei das Ausschüttelement keine weitere Ausnehmung für die Entspannung des Halteglieds aufweist. Das Halteglieds kann durch das Ausschüttelement radial in die Ausnehmung des Dosierelements gedrückt werden. Das Halteglied oder zumindest ein Teil des Halteglieds des Halteelements kommt in Anschlagkontakt mit einer Seite der Ausnehmung des Dosierelements, sodass eine relative Drehung des Dosierelements zu dem Halteelement verhindert werden kann. Somit kann das Einstellen oder Aufziehen einer weiteren Dosis verhindert werden, wenn die letzte Dosis ausgeschüttet worden ist.

Die Erfindung wird im Folgenden an mehreren Figuren beschrieben. Die hierbei offenbarten Merkmale bilden je einzeln und in Kombination die Erfindung vorteilhaft weiter. Es zeigen:
Figur 1 eine Explosionsansicht einer ersten Ausführungsform einer Injektionsvorrichtung
Figur 2a eine Längsschnittansicht der ersten Ausführungsform der Injektionsvorrichtung in einer Anfangsposition eines Dosierelements (9)
Figur 2b die Injektionsvorrichtung gemäss Figur 2a um die Längsachse B-B um 90° gedreht im Längsschnitt
Figur 3a eine Längsschnittansicht der ersten Ausführungsform der Injektionsvorrichtung in einer aufgezogenen Position des Dosierelements (9)
Figur 3b die Injektionsvorrichtung gemäss Figur 3a um die Längsachse B-B um 90° gedreht im Längsschnitt
Figur 4 eine Detailansicht des proximalen Endes der Injektionsvorrichtung gemäss Figur 3a und Figur 3b, wobei ein Teil des Gehäuses (10) und ein Teil des Einstellknopfs (11) ausgespart sind, sodass das Innere der Injektionsvorrichtung ersichtlich ist
Figur 5a eine Längsschnittansicht der ersten Ausführungsform der Injektionsvorrichtung in einer injektionsausgelösten Position des Dosierelements (9)
Figur 5b die Injektionsvorrichtung gemäss Figur 5a um die Längsachse B-B um 90° gedreht im Längsschnitt
Figur 6a eine Detailansicht der Injektionsvorrichtung gemäss der Figur 5b, wobei der Mechanismus zur Anzeige des Injektionsendes in der injektionsausgelösten Position des Dosierelements (9) dargestellt ist
Figur 6b die Detailansicht der Injektionsvorrichtung gemäss Figur 6a, wobei der Mechanismus zur Anzeige des Injektionsendes in der Zwischenposition zwischen der injektionsausgelösten und der injektionsbeendeten Position des Dosierelements (9) dargestellt ist
Figur 6c die Detailansicht der Injektionsvorrichtung gemäss Figur 6c, wobei der Mechanismus zur Anzeige des Injektionsendes in der injektionsbeendeten Position des Dosierelements (9) dargestellt ist
Figur 7a eine Längsschnittansicht der ersten Ausführungsform der Injektionsvorrichtung in einer komplett ausgeschütteten Position des Dosierelements (9)
Figur 7b die Injektionsvorrichtung gemäss Figur 7a um die Längsachse B-B um 90° gedreht im Längsschnitt
Figur 8 eine Explosionsansicht einer zweiten Ausführungsform der Injektionsvorrichtung
Figur 9a eine Längsschnittansicht der zweiten Ausführungsform der Injektionsvorrichtung in einer Anfangsposition eines Dosierelements (90)
Figur 9b die Injektionsvorrichtung gemäss Figur 9a um die Längsachse B-B um 90° gedreht im Längsschnitt
Figur 10a eine Längsschnittansicht der zweiten Ausführungsform der Injektionsvorrichtung in einer aufgezogenen Position des Dosierelements (90)
Figur 10b die Injektionsvorrichtung gemäss Figur 10a um die Längsachse B-B um 90° gedreht im Längsschnitt
Figur 11 eine Detailansicht des proximalen Endes der Injektionsvorrichtung gemäss Figur 10a und Figur 10b, wobei ein Teil des Gehäuses (100) und ein Teil des Einstellknopfs (110) ausgespart sind, sodass das Innere der Injektionsvorrichtung ersichtlich ist
Figur 11a eine Längsschnittansicht der zweiten Ausführungsform der Injektionsvorrichtung in einer injektionsausgelösten Position des Dosierelements (90)
Figur 11b die Injektionsvorrichtung gemäss Figur 11a um die Längsachse B-B um 90° gedreht im Längsschnitt
Figur 12 eine Detailansicht des proximalen Endes der Injektionsvorrichtung der zweiten Ausführungsform in einer komplett ausgeschütteten Position des Dosierelements (90), wobei ein Teil des Gehäuses (100) und ein Teil des Einstellknopfs (110) ausgespart sind, sodass das Innere der Injektionsvorrichtung ersichtlich ist Figur 13 eine Explosionsansicht einer dritten Ausführungsform der Injektionsvorrichtung
Figur 14a eine Längsschnittansicht der dritten Ausführungsform der Injektionsvorrichtung in einer Anfangsposition eines Einstellknopfs (1100)
Figur 14b die Injektionsvorrichtung gemäss Figur 14a um die Längsachse B-B um 90° gedreht im Längsschnitt
Figur 15a eine Längsschnittansicht der dritten Ausführungsform der Injektionsvorrichtung in einer aufgezogenen Position des Einstellknopfs (1100)
Figur 15b die Injektionsvorrichtung gemäss Figur 15a um die Längsachse B-B um 90° gedreht im Längsschnitt
Figur 16 eine Detailansicht des proximalen Endes der Injektionsvorrichtung gemäss Figur 15a und Figur 15b, wobei ein Teil des Gehäuses (1000) und ein Teil des Einstellknopfs (1100) ausgespart sind, sodass das Innere der Injektionsvorrichtung ersichtlich ist
Figur 17a eine Längsschnittansicht der dritten Ausführungsform der Injektionsvorrichtung in einer injektionsausgelösten Position des Einstellknopfs (1100)
Figur 17b die Injektionsvorrichtung gemäss Figur 17a um die Längsachse B-B um 90° gedreht im Längsschnitt
Figur 18 eine Detailansicht des proximalen Endes der Injektionsvorrichtung der zweiten Ausführungsform in einer komplett ausgeschütteten Position des Einstellknopfs (1100), wobei ein Teil des Gehäuses (1000) und ein Teil des Einstellknopfs (1100) ausgespart sind, sodass das Innere der Injektionsvorrichtung ersichtlich ist
Figur 19a eine Detailansicht einer Injektionsvorrichtung, wobei der Mechanismus zur Anzeige des Injektionsendes in einer Anfangsposition eines Einstellknopfs dargestellt ist
Figur 19b eine Längsschnittansicht der Injektionsvorrichtung gemäss Figur 19a
Figur 20a die Detailansicht der Injektionsvorrichtung gemäss Figur 19a, wobei der Mechanismus zur Anzeige des Injektionsendes in der Zwischenposition zwischen der injektionsausgelösten und der injektionsbeendeten Position des Einstellknopfs dargestellt ist
Figur 20b die Längsschnittansicht der Injektionsvorrichtung gemäss Figur 20a
Figur 21a die Detailansicht der Injektionsvorrichtung gemäss Figur 19a, wobei der Mechanismus zur Anzeige des Injektionsendes in der injektionsbeendeten Position des Einstellknopfs dargestellt ist.
Figur 21b die Längsschnittansicht der Injektionsvorrichtung gemäss Figur 21a
Figur 22 eine Explosionsansicht einer vierten Ausführungsform einer Injektionsvorrichtung
Figur 23a eine Längsschnittansicht der vierten Ausführungsform der Injektionsvorrichtung in einer Anfangsposition eines Dosierelements (9000)
Figur 23b die Injektionsvorrichtung gemäss Figur 23a um die Längsachse B-B um 90° gedreht im Längsschnitt
Figur 24a eine Längsschnittansicht der vierten Ausführungsform der Injektionsvorrichtung in einer aufgezogenen Position des Dosierelements (9000)
Figur 24b die Injektionsvorrichtung gemäss Figur 24a um die Längsachse B-B um 90° gedreht im Längsschnitt
Figur 25a eine Längsschnittansicht der vierten Ausführungsform der Injektionsvorrichtung in einer injektionsausgelösten Position des Dosierelements (9000)
Figur 25b die Injektionsvorrichtung gemäss Figur 25a um die Längsachse B-B um 90° gedreht im Längsschnitt

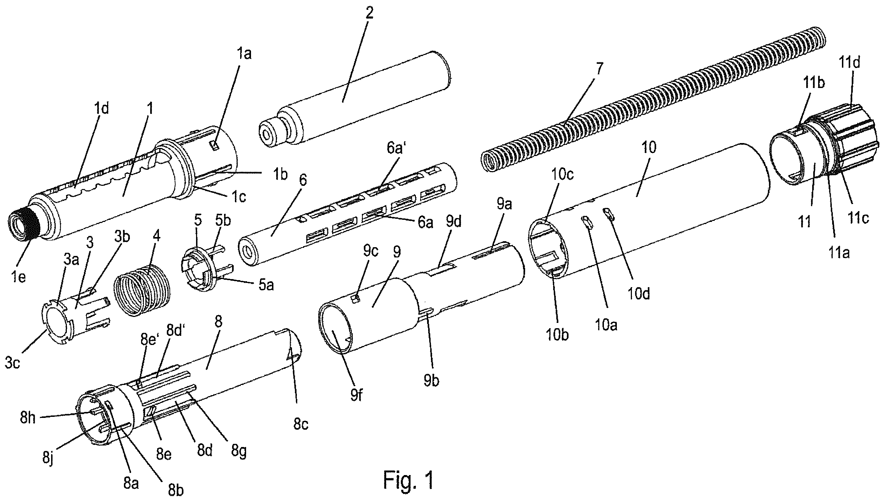

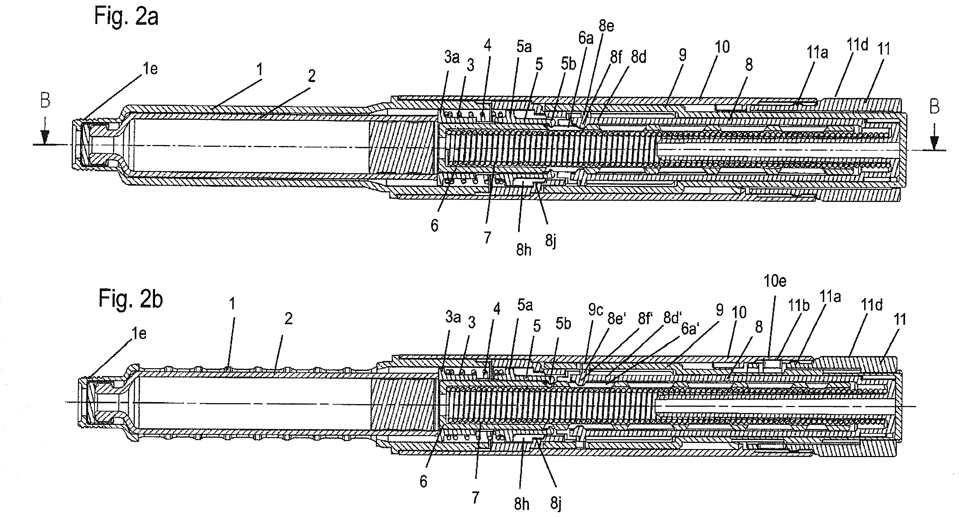

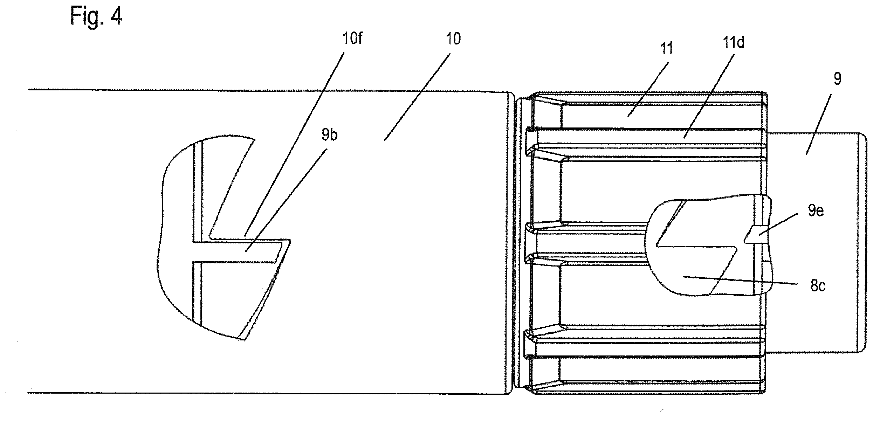

In der Figur 1 ist eine Explosionsansicht einer ersten Ausführungsform einer erfindungsgemässen Injektionsvorrichtung dargestellt. Die Injektionsvorrichtung umfasst eine Karpule (2), welche von einem Karpulenhalter (1) aufgenommen ist, wobei der Karpulenhalter (1) über eine Karpulenhalternase (1a) mit einer distalen Gehäusenasenaussparung (10a) eines Gehäuses (10) axialfest verbunden ist. Ferner ist an dem Karpulenhalter (1) ein Karpulenhaltersteg (1b) zur drehfesten Verbindung über eine korrespondierende Gehäusenut (10b) mit dem Gehäuse (10) vorgesehen. Ein Karpulenhalteranschlag (1c) kann axial an eine distale Gehäusekante (10c) des Gehäuses (10) anschlagen. Eine Karpulenhalteraussparung (1d) in dem Karpulenhalter (1) dient zur Anzeige der relativen Position eines in der Karpule (2) axial bewegbaren Stopfens. An dem distalen Ende des Karpulenhalters (1) ist eine Nadelverbindungselement (1e) vorgesehen, welches zur lösbaren Befestigung einer Injektionsnadel vorgesehen ist. Die Injektionsvorrichtung umfasst ferner ein Dosierelement (9), welches radial zwischen dem Gehäuse (10) und einem Halteelement (8) angeordnet ist. Das Halteelement (8) ist mit Hilfe einer Halteelementnase (8a) mit einer proximalen Gehäusenasenaussparung (10d) des Gehäuses (10) axialfest verbunden. Zudem ist ein Halteelementsteg (8b) zur drehfesten Verbindung mit einer korrespondierenden distalen Gehäusenut (10b) an dem Halteelement (8) vorgesehen. Die Injektionsvorrichtung umfasst zudem ein Einstellknopf (11), welcher zumindest teilweise in das Innere des hülsenförmigen Gehäuses (10) ragt und mit einem an dem Einstellknopf(10) angebrachten Einstellknopfring (11a) über eine Ring-/Nutverbindung mit dem Gehäuse (10) axialfest verbunden ist. An dem Einstellknopf (11) ist ein Einstellknopfschnapparm (11b) vorgesehen, welcher radial nach aussen ragt und mit einer an der Mantelinnenfläche des Gehäuses (10) vorgesehene proximale Gehäusenut oder Gehäuserampe (10e; beispielsweise in Figur 2b ersichtlich) derart zusammenwirken kann, dass der Einstellknopf (11) nur in eine Drehrichtung relativ zu dem Gehäuse (10) drehbar ist. Das Einrasten des Einstellknopfschnapparms (11b) mit der proximalen Gehäusenut oder Gehäuserampe (10e; beispielsweise in Figur 2b ersichtlich) kann ein akustisches Signal, insbesondere Klick-Geräusch auslösen. An der Mantelaussenfläche des Einstellknopfs (11) erstreckt sich in Längsrichtung ein Einstellknopfgriffsteg (11d) zur Einstellung oder Aufziehen der Dosis. Der Einstellknopfschnapparm (11b) kann in eine Dosierelementeinbuchtung (9d), die an der Mantelaussenfläche des Dosierelements (9) vorgesehen ist, radial nach innen elastisch auslenken. Der Einstellknopf (11) weist an dessen Mantelinnenfläche einen Einstellknopfsteg (11c) auf, welcher mit einer an der Mantelaussenfläche des Dosierelements (9) vorgesehene proximale Dosierelementnut (9a) eine drehfeste Verbindung bildet. An der Mantelinnenfläche des Dosierelements (9) ist eine Dosierelementnocke (9e, in Figur 4 ersichtlich) vorgesehen, welche bei Drehung des Dosierelements (9) über die drehfeste Verbindung des Einstellknopfs (11) entlang einer an der Mantelaussenfläche des Halteelements (8) angebrachte sägezahnförmige Halteelementsteuerkurve (8c) gleiten kann. Die Dosierelementnocke (9e; in Figur 4 ersichtlich) des Dosierelements (9) und die Halteelementsteuerkurve (8c) des Halteelements (8) können derart zusammenwirken, dass bei relativer Drehung des Dosierelements (9) zu dem Halteelement (8) das Dosierelement (9) axial in die proximale Richtung bewegbar ist. An der Mantelaussenfläche des Dosierelements (9) ist eine Dosierelementsteg (9b) vorgesehen, welcher mit einer sägezahnförmigen Gehäusesteuerkurve (10f, in Figur 4 ersichtlich), die an der Mantelinnenfläche des Gehäuses (10) angebracht ist, in einem Führungseingriff oder in einer Kulissenführung sein kann. Der Dosierelementsteg (9b) des Dosierelements (9) und die Gehäusesteuerkurve (10f, in Figur 4 ersichtlich) des Gehäuses (10) können derart zusammenwirken, dass bei relativer Drehung des Dosierelements (9) zu dem Gehäuse (10) verhindert wird, dass mehr als eine zu verabreichende festgelegte Dosis eingestellt werden kann. Das Dosierelement (9) weist eine Dosierelementaussparung (9c) auf, welche derart ausgelegt ist, dass eine Schnappnocke (8e; 8e'), welche auf einem Halteglied (8d; 8d') des Halteelements (8) radial nach aussen erstreckend ausgebildet ist, in diese Dosierelementaussparung (9c) ragen kann. Das Dosierelement (9) umfasst zwei Dosierelementaussparungen (9c), welche etwa im 180° Winkel in Umfangsrichtung des Dosierelements (9) versetzt zueinander angeordnet sind. Das Halteglied (8d; 8d') des Halteelements (8) kann als Schnapparm ausgebildet sein. Das Halteelement (8) weist ein erstes (8d) und ein zweites Halteglied (8d') oder einen ersten oder zweiten Halteelementschnapparm auf, wobei das erste Halteglied (8d) oder der erste Schnapparm eine erste nach aussen ragende Schnappnocke (8e) und eine erste nach innen ragende Schnappnocke (8f; beispielsweise in Figur 2a und Figur 2b ersichtlich) umfasst und das zweite Halteelementglied (8d') oder der zweite Schnapparm eine zweite nach aussen ragende Schnappnocke (8d') und eine zweite nach innen ragende Schnappnocke (8f, beispielsweise in Figur 2a und Figur 2b ersichtlich) umfasst. Die erste (8e) und die zweite nach aussen ragende Schnappnocke (8e') und die erste (8f) und die zweite nach innen ragende Schnappnocke (8f) sind in etwa gleicher axialen Höhe auf dem Halteelement (8) angeordnet. Die erste nach innen ragende Schnappnocke (8f) ist etwa diametral gegenüber der ersten nach aussen ragenden Schnappnocke (8e) an dem ersten Halteglied (8d) angeordnet, wobei die zweite nach innen ragende Schnappnocke (8f) etwa diametral gegenüber der zweiten nach aussen ragenden Schnappnocke (8e') an dem zweiten Halteglied (8d') vorgesehen ist. Das erste (8d) und das zweite Halteglied (8d') sind etwa im 90° Winkel in Umfangsrichtung des Halteelements (8) zueinander angeordnet. Zudem sind zwei erste (8d) und zwei zweite Halteglieder (8d') an dem Halteelement (8) vorgesehen, wobei die zwei ersten Halteglieder (8d) zueinander etwa im 180° Winkel in Umfangsrichtung des Halteelements (8) zueinander angeordnet sind und die zwei zweiten Halteglieder (8d') zueinander etwa im 180° Winkel in Umfangsrichtung des Halteelements (8) zueinander angeordnet sind. Beide ersten (8d) und beide zweiten Halteglieder (8d') umfassen je eine nach aussen ragende Schnappnocke (8e; 8e') und eine nach innen ragende Schnappnocke (8f; 8f). An der Mantelaussenfläche des Halteelements (8) sind mehrere Halteelementrippen (8g) angebracht, die mit einer an der Mantelinnenfläche des Dosierelements (9) vorgesehene distale Dosierelementmantelinnenfläche (9f) in Gleitkontakt sein können, um beim Ausschüttvorgang der festgelegten Dosis aus der Injektionsvorrichtung eine koaxiale Führung zwischen dem Halteelement (8) und dem Dosierelement (9) zu erzielen. Zudem können die Halteelementrippen (8g) dazu dienen, eine Führung zu bilden, um ein elastisches nach aussen Schwenken des ersten (8d) und/oder zweiten Halteglieds (8d') mit der ersten (8e) und/oder zweiten nach aussen ragenden Schnappnocke (8e') und mit der ersten (8f, beispielsweise in Figur 2a und Figur 2b ersichtlich) und/oder zweiten nach innen ragenden Schnappnocke (8f, beispielsweise in Figur 2a und Figur 2b ersichtlich) oder ein elastisches nach aussen Spannen des ersten (8d) und/oder zweiten Halteglieds (8d') mit der ersten (8e) und/oder zweiten nach aussen ragenden Schnappnocke (8e') und mit der ersten (8f, beispielsweise in Figur 2a und Figur 2b ersichtlich) und/oder nach innen ragenden Schnappnocke (8f', beispielsweise in Figur 2a und Figur 2b ersichtlich) zu erlauben. Dazu erstrecken sich die Halteelementrippen (8g) auf dem Halteelement parallel zu dem ersten (8d) und/oder zweiten Halteglied (8d'). Die Injektionsvorrichtung umfasst ferner ein Ausschüttelement (6). Das hülsenförmige Ausschüttelement (6) hat im Innem eine vorgespannte Feder (7) aufgenommen, welche am distalen Ende an einer distalen Stirnwand des Ausschüttelements (6) und am proximalen Ende an einer proximalen Stirnwand des Halteelements (8) abgestützt ist. Das hülsenförmige Ausschüttelement (6) umfasste eine erste Ausnehmung (6a), welche derart ausgebildet ist, dass die erste nach innen ragende Schnappnocke (8f', beispielsweise in Figur 2a und Figur 2b ersichtlich) des ersten Halteglieds (8d) aufnehmbar ist. Zudem weist das hülsenförmige Ausschüttelement (6) eine zweite Ausnehmung (6a') auf, welche derart ausgebildet ist, dass die zweite nach innen ragende Schnappnocke (8f', beispielsweise in Figur 2a und Figur 2b ersichtlich) des zweiten Halteglieds (8d') aufnehmbar ist. Die erste (6a) und die zweite Ausnehmung (6a') des Ausschüttelements (6) sind in Umfangsrichtung und in axialer Richtung teilweise versetzt zueinander angeordnet. Die erste (6a) und die zweite Ausnehmung (6a') sind derart teilweise axial zueinander versetzt vorgesehen, dass sie sich in Umfangsrichtung überlappen. Das Ausschüttelelement (6) umfasst zudem mehrere erste Ausnehmungen (6a), welche axial versetzt zueinander angeordnet sind und mehrere zweite Ausnehmungen (6a'), welche ebenfalls axial versetzt zueinander angeordnet sind. Zudem umfasst das Ausschüttelement (6) zwei erste Ausnehmungen (6a), welche etwa im 180° Winkel in Umfangsrichtung des Ausschüttelements (6) versetzt zueinander angeordnet sind, wobei das Ausschüttelement (6) jeweils mehrere erste Ausnehmungen (6a) vorsieht, die axial versetzt zueinander angeordnet sind. Ferner umfasst das Ausschüttelement (6) zwei zweite Ausnehmungen (6a'), welche etwa im 180° Winkel in Umfangsrichtung des Ausschüttelements (6) versetzt zueinander angeordnet sind, wobei das Ausschüttelement (6) jeweils mehrere zweite Ausnehmungen (6a') vorsieht, die axial versetzt zueinander angeordnet sind. Die erste (6a) und die zweite Ausnehmung (6a') sind etwa im 90° Winkel in Umfangsrichtung des Ausschüttelements (6) versetzt zueinander angeordnet. Das hülsenförmige Ausschüttelement (6) ist radial zwischen der vorgespannten Feder (7) und dem Halteelement (8) vorgesehen, wobei auf einem distalen Bereich des Ausschüttelements (6) ein distales und proximales Spannelement (3; 5) gelagert sind. Zwischen dem distalen und proximalen Spannelement (3; 5) ist eine vorgespannte Feder (4) angeordnet, wobei die vorgespannte Feder (4) durch eine axiale Relativbewegung zwischen dem distalen und proximalen Spannelement (3; 5) spannbar ist. Die Feder (4) ist zwischen einem Spannelementringsteg (3a) des distalen Spannelements (3) und einem Spannelementring (5a) des proximalen Spannelements (5) abgestützt. Das proximale Spannelement (5) umfasst einen proximalen Spannelementspanner (5b) und das distale Spannelement (3) weist einen Spannelementhaken (3b) auf. Der Spannelementhaken (3b) des distalen Spannelements (3) kann durch eine innere Öffnung des proximalen Spannelements (5) ragen und mit dem Spannelementring (5a) des proximalen Spannelements (5) verrasten, derart dass das distale Spannelement (3), die Feder (4) und das proximale Spannelement (5) eine Montageeinheit bilden können. Im zusammengesetzten Zustand der Injektionsvorrichtung kann der proximaler Spannelementring (5a) des proximalen Spannelements (5) gegen einen an der Mantelinnenfläche des Halteelements (8) vorgesehenen Halteelementanschlag (8h) anschlagen, wobei dieser Anschlagkontakt lösbar ist, um ein akustisches Signal, insbesondere ein Klick-Geräusch zu bilden. Das Halteelement (8) weist an der Mantelinnenfläche eine Halteelementnut (8j) mit einer proximalen Stirnseite auf, welche ein proximales Ende des proximalen Spannelementschnapper (5b) mit deren proximalen Stirnseite aufnehmen kann.

In der Figur 2a ist die erste Ausführungsform der Injektionsvorrichtung in einer Anfangsposition eines Dosierelements (9) im Längsschnitt dargestellt, wobei die Figur 2b die Injektionsvorrichtung gemäss Figur 2a zeigt, wobei die Injektionsvorrichtung um die Längsachse B-B um 90° gedreht ist. Das erste Halteglied (8d) ragt mit der ersten nach innen ragenden Schnappnocke (8f) in die erste Ausnehmung (6a) des Ausschüttelements (6). Die erste nach innen ragende Schnappnocke (8f) wird über die erste nach aussen ragende Schnappnocke (8e) des ersten Halteglieds (8d) von dem Dosierelement (9) in Eingriff mit der ersten Ausnehmung (6a) des Ausschüttelements (6) gehalten, derart dass das erste Halteglied (8d) mit der nach innen ragenden Schnappnocke (8f) daran gehindert wird, radial elastisch nach aussen ausgelenkt zu werden, um ausser Eingriff mit der ersten Ausnehmung (6a) des Ausschüttelements (6) zu gelangen. Die Feder (7), welche von dem Ausschüttelement (6) aufgenommen ist, ist auf der distalen Seite an dem Ausschüftelement (6) und auf der proximalen Seite von dem Halteelement (8) abgestützt und beaufschlagt das Ausschüftelement (6) mit einer Federkraft in die distale Richtung. Eine distale Stirnseite des Verbindungsstegs zweier in axialer Richtung versetzt zueinander angeordneten ersten Ausnehmungen (6a) des Ausschüttelements (6) ist in Anschlagkontakt mit einer proximalen Stirnseite der nach innen ragenden Schnappnocke (8f) des ersten Halteglieds (8d) und verhindert eine relative Bewegung des Ausschüttelements (6) zu dem Gehäuse (10) in die distale Richtung zur Ausschüttung der festgelegten Dosis. Die zweite nach innen ragende Schnappnocke (8f') des zweiten Halteglieds (8d') ragt elastisch entspannt in die zweite Ausnehmung (6a') des Ausschüttelements (6). Die Dosierelementaussparung (9c) des Dosierelements (9) ist über der zweiten nach aussen ragenden Schnappnocke (8f) des zweiten Halteglieds (8d') angeordnet, wobei die zweite nach innen ragende Schnappnocke (8f') des zweiten Halteglieds (8d') ausser Eingriff mit der Dosierelementaussparung (9c) des Dosierelements (9) ist. Eine proximale Stirnseite des proximalen Spannelementrings (5a) des proximalen Spannelements (5) stösst gegen eine distale Stirnseite des Halteelementanschlags (8h) des Halteelements (8), um die Feder (4) zwischen dem distalen Spannelementringsteg (3a) des distalen Spannelements (3) und dem proximalen Spannelementring (5a) des proximalen Spannelements (5) zu spannen. Das distale Spannelement (3) ist über eine distale Spannelementaussparung (3c) und dem distaler Spannelementringsteg (3a) mit dem Karpulenhalter (1) axial- und drehfest verbunden. Das proximale Spannelement (5) ist auf dem Ausschüttelement (6) axial bewegbar gelagert, wobei der proximale Spannelementring (5a) in Gleitkontakt mit der Mantelinnenfläche des Halteelements (8) ist. Der eine proximale Spannelementschnapper (5b) ragt elastisch entspannt in die erste Ausnehmung (6a) und der andere proximale Spannelementschnapper (5b) ragt elastisch entspannt in die zweite Ausnehmung (6a') des Ausschüttelements (6).

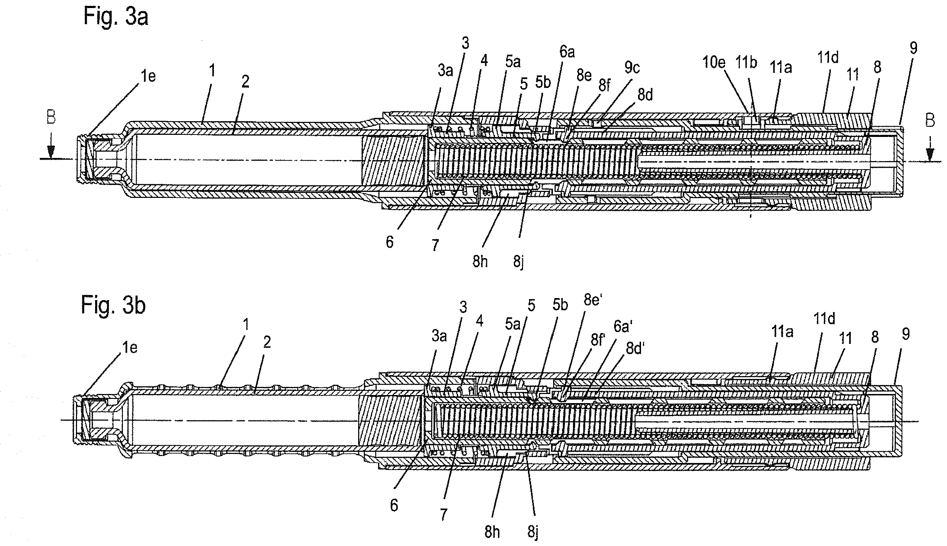

In der Figur 3a ist die erste Ausführungsform der Injektionsvorrichtung in einer aufgezogenen Position des Dosierelements (9) im Längsschnitt dargestellt, wobei die Figur 3b die Injektionsvorrichtung gemäss Figur 3a zeigt, wobei die Injektionsvorrichtung um die Längsachse B-B um 90° gedreht ist. Um das Injektionsgerät injektionsbereit zu machen, dreht der Benutzer den Einstellknopf (11) über den Einstellknopfgriffsteg (11d) relativ zu dem Gehäuse (10). Der Einstellknopfschnapparm (11b) des Einstellknopfs (11) gleitet über die proximale Gehäusenut oder die proximale Gehäuserampe (10e) des Gehäuses (10) und verhindert ein Drehen des Einstellknopfs (11) in die entgegengesetzte Richtung. Das Einrasten des Einstellknopfschnapparms (11b) des Einstellknopfs (11) hinter die proximale Gehäusenut oder die proximale Gehäuserampe (10e) des Gehäuses (10) erzeugt ein akustisches Geräusch, insbesondere ein Klick-Geräusch. Durch die drehfeste Verbindung zwischen dem Einstellknopfsteg (11c) des Einstellelements (11) und der proximalen Dosierelementnut (9a) des Dosierelements (9) wird die Drehbewegung auf das Dosierelement (9) übertragen, wobei das Dosierelement (9) mittels Führungseingriff oder Kulissenführung zwischen der Dosierelementnocke (9e) des Dosierelements (9) und der Halteelementsteuerkurve (8c) des Halteelements (8) in die proximale Richtung bewegt wird, wie in Figur 4 ersichtlich ist. Am Ende der Aufziehbewegung des Dosierelements (9) gelangt der Dosierelementsteg (9b) des Dosierelements (9) in Anschlagkontakt mit einer steilen Flanke der Gehäusesteuerkurve (10f) des Gehäuses (10), wobei dieser Anschlagkontakt die Aufziehbewegung beschränkt. Wie in Figur 4 gezeigt ist, befindet sich die Dosierelementnocke (9e) des Dosierelements (9) am Ende der Aufziehbewegung des Dosierelements (9) in axialer Flucht zu einer steilen Flanke der Halteelementsteuerkurve (8c) des Halteelements (8), derart dass durch die Begrenzung der Aufziehbewegung zwischen dem Dosieranschlagsteg (9b) des Dosierelements (9) und der Gehäusesteuerkurve (10f) des Gehäuses (10) zur Ausschüttung der festgelegten Dosis das Dosierelement (9) in die distale Richtung bewegt werden kann. In der aufgezogenen Position des Dosierelements (9) wird die erste nach innen ragende Schnappnocke (8e) des ersten Halteglieds (8d) immer noch über die erste nach aussen ragende Schnappnocke (8f) des ersten Halteglieds (8d) von dem Dosierelement (9) in Eingriff mit der ersten Ausnehmung (6a) des Ausschüttelements (6) gehalten, wobei durch die relative Drehbewegung des Dosierelements (9) die Dosierelementaussparung (9c) des Dosierelements (9) in axialer Flucht und in proximaler Richtung versetzt zur ersten nach aussen ragenden Schnappnocke (8e) des ersten Halteglieds (8d) zu liegen kommt. Zudem wird durch die relative Drehbewegung des Dosierelements (9) die zweite nach innen ragende Schnappnocke (8f) des zweiten Halteglieds (8d') über die zweite nach aussen ragende Schnappnocke (8e') von dem Dosierelement (9) in Eingriff mit der zweiten Ausnehmung (6a') des Ausschüttelements (6) gehalten.

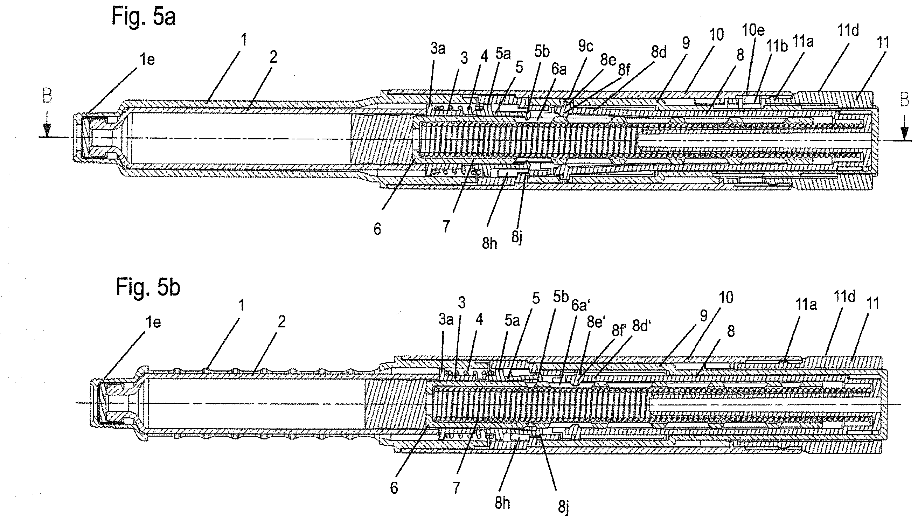

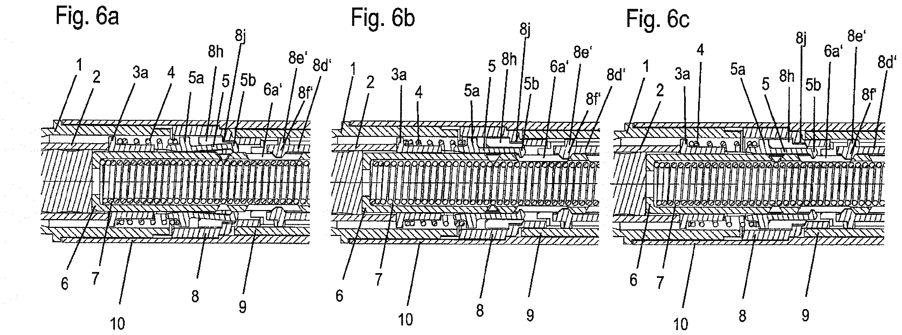

In der Figur 5a ist die erste Ausführungsform der Injektionsvorrichtung in einer injektionsausgelösten Position des Dosierelements (9) im Längsschnitt dargestellt, wobei die Figur 5b die Injektionsvorrichtung gemäss Figur 5a zeigt, wobei die Injektionsvorrichtung um die Längsachse B-B um 90° gedreht ist. Um die Injektion der festgelegten Dosis auszulösen, betätigt der Benutzer das Dosierelement (9), indem er das Dosierelement (9) axial relativ zu dem Halteelement (8) oder dem Gehäuse (10) in die distale Richtung bewegt, bis das proximale Ende des Dosierelements (9) an eine proximale Stirnseite des Halteelements (8) anstösst. Durch die axiale relative Bewegung des Dosierelements (9) zu dem Halteelement (8) gelangt die Dosierelementaussparung (9c) des Dosierelements (9) über die erste nach aussen ragende Schnappnocke (8e) des Halteelements (8), derart dass die erste nach aussen ragende Schnappnocke (8e) des Halteelements (8) aufgrund des axialen Drucks, welcher von dem Ausschüttelement (6) auf das erste Halteglied (8d) des Halteelements (8) wirkt, elastisch in die Dosierelementaussparung (9c) des Dosierelements (9) ausgelenkt wird. Dadurch gelangt die erste nach innen ragende Schnappnocke (8f) des ersten Halteglieds (8d) ausser Eingriff mit der ersten Ausnehmung (6a) des Ausschüttelements (6). Zudem löst sich der Anschlagkontakt zwischen der proximalen Stirnseite der ersten nach innen ragenden Schnappnocke (8f) und der distalen Stirnseite des Verbindungsstegs zweier in axialer Richtung versetzt zueinander angeordneten ersten Ausnehmungen (6a) des Ausschüttelements (6), sodass sich der Verbindungssteg relativ zu der ersten nach innen ragenden Schnappnocke (8f) in die distale Richtung bewegt. Das Ausschüttelement (6) bewegt sich während dem Ausschüttvorgang der festgelegten Dosis axial relativ zu dem Gehäuse (10) in die distale Richtung. Während dem Ausschüttvorgang stösst die proximale Stirnseite des proximalen Spannelementspanners (5b) gegen eine distale Stirnseite des Verbindungsstegs zweier in axialer Richtung versetzt zueinander angeordneten zweiten Ausnehmungen (6a') des Ausschüttelements (6), sodass das proximale Spannelement (5) relativ zu dem distalen Spannelement (3) mit dem Ausschüttelement (6) in die distale Richtung gleitet und folglich die Halteelementanschlag (8h) ausser Anschlagkontakt mit dem proximalen Spannelementring (5a) gelangt, wie in Figur 5b und Figur 6a ersichtlich ist. Dadurch wird die Feder (4) zwischen dem distalen Spannelement (3) und dem proximalen Spannelement (5) gespannt oder weiter gespannt. Das proximale Spannelement (5) bewegt sich axial relativ zu dem distalen Spannelement (3) bis der proximale Spannelementschnapper (5b) elastisch radial nach aussen in die Halteelementnut (8j) ausgelenkt wird. Die proximale Stimseite des proximalen Spannelementschnappers (5b) kommt dabei in Anschlagkontakt mit der proximalen Stirnseite der Halteelementnut (8j), wobei die Feder (4) im gespannten Zustand gehalten wird. Der Verbindungssteg, der zwischen zwei in axialer Richtung versetzt zueinander angeordneten zweiten Ausnehmungen (6a') des Ausschüttelements (6) vorgesehen ist, gleitet entlang des proximale Spannelementschnappers (5b), bis der proximale Spannelementschnapper (5b) in die proximalere zweite Ausnehmung (6a') des Ausschüttelements (6) elastisch radial nach innen einlenkt, wie in Figur 6b dargestellt ist. Die distale Stirnseite des Verbindungsstegs zweier in axialer Richtung versetzt zueinander angeordneten zweiten Ausnehmungen (6a') des Ausschüttelements (6) gelangt in Anschlagkontakt mit der proximalen Stirnseite der nach innen ragenden Schnappnocke (8f') des zweiten Halteglieds (8d') und verhindert, dass das Ausschüttelement (6) relativ zu dem Halteelement (8) weiter in die distale Richtung bewegt wird. Dadurch wird die Ausschüttbewegung gestoppt. Das zweite Halteglied (8d') des Halteelements (8) wird durch das Dosierelement (9) daran gehindert, elastisch radial nach aussen auszulenken, da die Dosierelementaussparung (9c) des Dosierelements (9) über dem ersten Halteglied (8d) des Halteelements (8) angeordnet ist. Wie in Figur 5a ersichtlich ist, gleitet während dem Ausschüttvorgang der Verbindungssteg zweier in axialer Richtung versetzt zueinander angeordneten ersten Ausnehmungen (6a) des Ausschüffelements (6) entlang der ersten nach innen ragende Schnappnocke (8f) des ersten Halteglieds (8d) des Haltelements (8) bis die erste nach innen ragende Schnappnocke (8f) in die proximalere erste Ausnehmung (6a) der beiden ersten Ausnehmungen (6a) des Ausschüttelements (6) elastisch einlenkt, wobei sich das erste Halteelement (8d) elastisch entspannt. Wie in Figur 6c gezeigt, wird durch das Einlenken des proximalen Spannelementschnapper (5b) in die proximalere zweite Ausnehmung (6a') des Ausschüttelements (6) der Anschlagkontakt zwischen der proximalen Stirnseite des proximalen Spannelementschnappers (5b) und der proximalen Stirnseite der Halteelementnut (8j) aufgelöst. Dadurch wirkt die Federkraft der Feder (4) derart auf das proximale Spannelement (5), dass das proximale Spannelement (5) relativ zu dem Halteelement (8) in die proximale Richtung axial bewegt wird, bis der proximale Spannelementring (5a) des proximalen Spannelements (5) gegen den Halteelementanschlag (8h) des Halteelements (8) anschlägt. Dieser Anschlagkontakt erzeugt ein akustisches Geräusch, insbesondere ein Klick-Geräusch. Das Injektionsende der festgelegten Dosis der Injektionsvorrichtung kann somit angezeigt werden.

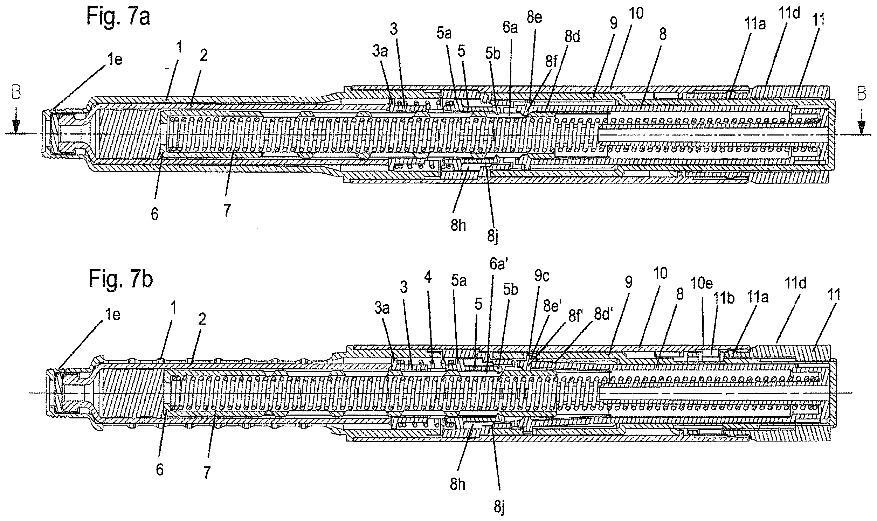

In der Figur 7a ist die erste Ausführungsform der Injektionsvorrichtung in einer komplett ausgeschütteten Position des Dosierelements (9) im Längsschnitt dargestellt, wobei die Figur 7b die Injektionsvorrichtung gemäss Figur 3a zeigt, wobei die Injektionsvorrichtung um die Längsachse B-B um 90° gedreht ist. Die Positionen der Einzelteile der Injektionsvorrichtung gemäss Figur 7a entsprechen den Positionen der Einzelteile der Injektionsvorrichtung gemäss Figur 2a, ausser dass das Ausschüttelement (6) so weit relativ zu dem Halteelement (8) axial in die distale Richtung vorgeschoben ist, dass die erste nach innen ragende Schnappnocke (8e) des Halteelements (8) sich in der proximalsten ersten Ausnehmung (6a) des Ausschüttelements (6) befindet. Das erste Halteglied (8d) des Halteelements (8) wird durch das Dosierelement (9) daran gehindert elastisch radial nach aussen auszulenken. Die zweite nach aussen ragende Schnappnocke (8e') des zweiten Halteglieds (8d') ragt in die Dosierelementaussparung (9c) des Dosierelements (9). Da keine weitere zweite Ausnehmung (6a') des Ausschüttelements (6), nämlich eine in die proximale Richtung der ersten Ausnehmung (6a) des Ausschüttelements (6) zweite Ausnehmung (6a') des Ausschüttelements (6), vorgesehen ist, wird das zweite Halteglied (6a') des Halteelements (6) daran gehindert, elastisch radial nach innen einzulenken. Die zweite nach aussen ragende Schnappnocke (8e') des zweiten Halteglieds (8d') ragt in der Dosierelementaussparung (9c) des Dosierelements (9) und verhindert das weitere Einstellen oder Aufziehen einer festgelegten Dosis. Die letzte festgelegte Dosis ist aus dem Injektionsgerät ausgeschüttet worden.

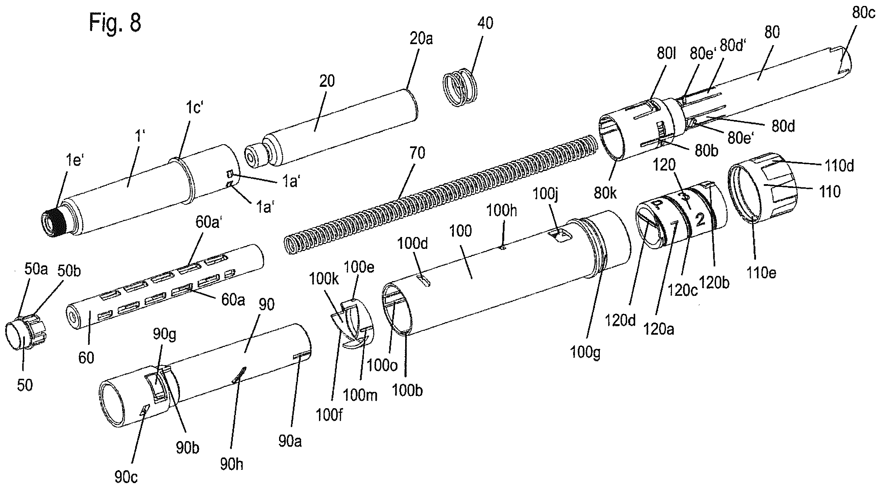

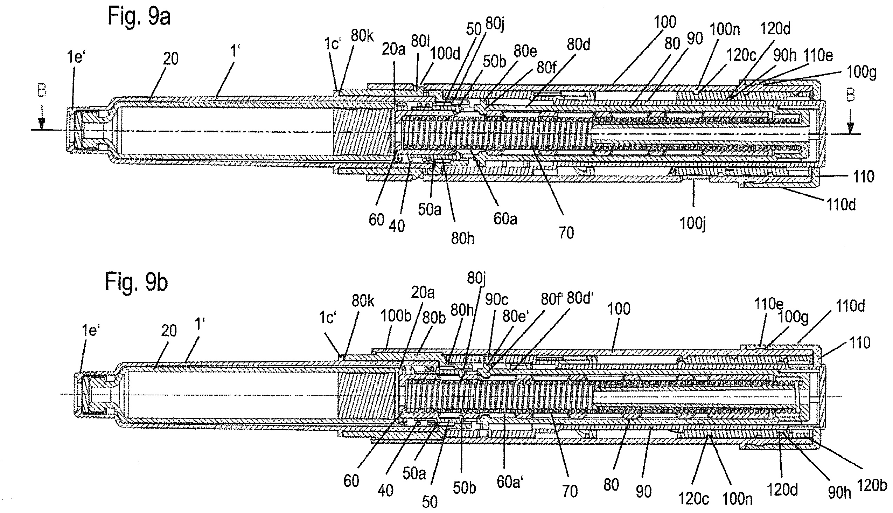

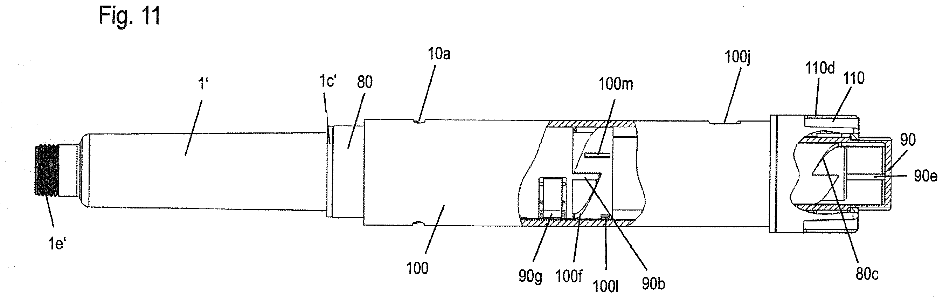

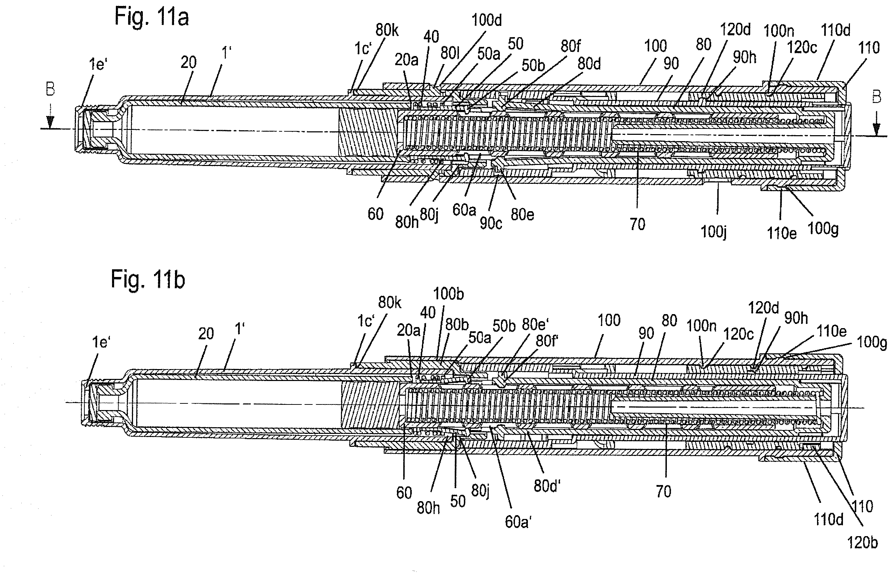

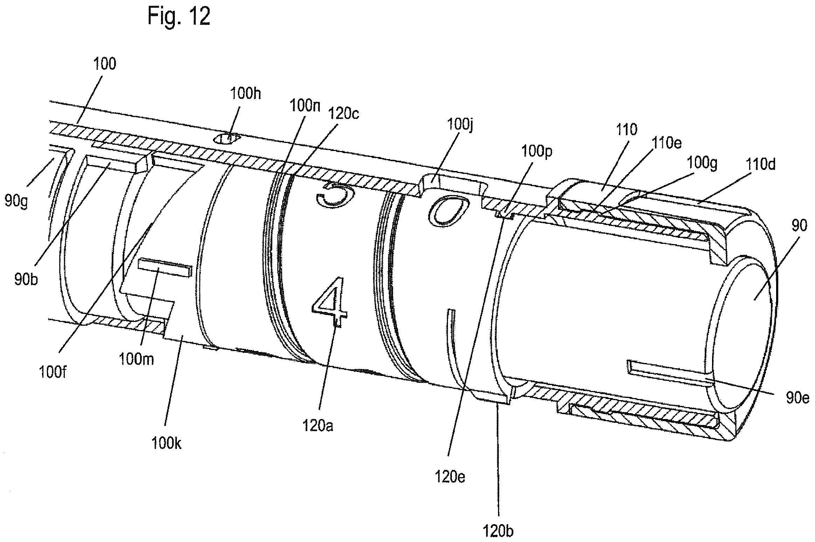

In der Figur 8 ist eine Explosionsansicht einer zweiten Ausführungsform einer erfindungsgemässen Injektionsvorrichtung dargestellt. Die Injektionsvorrichtung umfasst eine Karpule (20), welche von einem Karpulenhalter (1') aufgenommen ist. Der Karpulenhalter (1') ist über mindestens eine, in dieser Ausführungsform zwei Karpulenhalternasen (1a') mit mindestens einer, in dieser Ausführungsform zwei an der Mantelinnenfläche eines Halteelements (80) angeordneten Halteelementaussparungen (nicht ersichtlich) axial- und drehfest verbunden. Ein Karpulenhalteranschlag (1c') kann axial an eine distale Halteelementkante (80k) des Halteelements (80) anschlagen. Das distale Ende des Karpulenhalters (1') weist eine Nadelverbindungselement (1e') auf, welches zur lösbaren Befestigung einer Injektionsnadel dient. Die Injektionsvorrichtung umfasst ferner ein Dosierelement (90), das radial zwischen einem Gehäuse (100) und dem Halteelement (80) vorgesehen ist. Ein an dem Halteelement (80) vorgesehener Halteelementsteg (80b) ist zur drehfesten Verbindung mit einer korrespondierenden distalen Gehäusenut (100b) vorgesehen. Das Halteelement (80) ist über einen distalen Halteelementschnapparm (801) mit einer proximalen Gehäusenasenaussparung (100d) des Gehäuses (100) axialfest befestigt. Ferner ist ein Einstellknopf (11) vorgesehen, welcher über eine Einstellknopfringnut (110e) des Einstellknopfs (110) mit einem Gehäuseringsteg (100g) des Gehäuses (100) axialfest verbunden ist. An dem Einstellknopf (110) ist ein Einstellknopfgriffsteg (110d zur besseren Greifbarkeit des Einstellknopfs (110) durch den Benutzer vorgesehen. Der Einstellknopf (110) umfasst an der Mantelinnenfläche einen Einstellknopfsteg (nicht ersichtlich), welcher mit einer an der Mantelaussenfläche des Dosierelements (90) vorgesehenen proximalen Dosierelementnut (90a) eine drehfeste Verbindung eingeht. Das Dosierelement (90) umfasst einen Dosierelementschnapparm (90g), der mit einer an der Mantelinnenfläche des Gehäuses (100) vorgesehene distale Gehäuserampe (100o) derart zusammenwirken kann, dass das Dosierelement (90) nur in eine Drehrichtung relativ zu dem Gehäuse (100) drehen kann. Diese Zusammenwirkung zwischen dem Dosierelementschnapparm (90g) und der distale Gehäuserampe (100o) kann ein akustische Geräusch, insbesondere ein Klick-Geräusch erzeugen. Das Dosierelement (90) umfasst an dessen Mantelinnenfläche eine Dosierelementnocke (90e; in Figur 11 ersichtlich), die bei Drehung des Dosierelements (90) an einer sägezahnförmigen Halteelementkurve (80c) des Halteelements (80) entlang geführt werden kann. Dieser Führungseingriff oder die Kulissenführung zwischen der Dosierelementnocke (90e; in Figur 11 ersichtlich) und der Halteelementkurve (80c) bewirkt bei relativer Drehung des Dosierlements (90) zu dem Halteelement (80), dass das Dosierelement (90) axial in die proximale Richtung relativ zu dem Gehäuse (100) bewegbar ist. Das Dosierelement (90) weist einen Dosierelementsteg (90b) auf, welcher mit einer sägezahnförmigen Gehäusesteuerkurve (100f) eines Gehäuserings (100k) im Führungseingriff oder in der Kulissenführung ist. Der Gehäusering (100k) ist über eine Gehäuseringnase (100l) und einem Gehäuseringsteg (100m) mit einer Gehäuseringnasenaussparung (100h) und einem Gehäuseringnut (nicht ersichtlich) axial- und drehfest mit dem Gehäuse (100) verbunden. Der Dosierelementsteg (90b) des Dosierelements (90) und die Gehäusesteuerkurve (100f) des Gehäuserings (100k) können derart zusammenwirken, dass bei relativer Drehung des Dosierelements (90) zu dem Gehäuse (100) verhindert wird, dass mehr als eine zu verabreichende festgelegte Dosis eingestellt werden kann. Eine Dosierelementaussparung (90c) ist in dem Dosierelement (90) vorgesehen, welche derart ausgelegt ist, dass eine nach aussen ragende Halteelementschnappnocke (80e; 80e'), welche an einem Halteglied (80d; 80d') des Halteelements (80) angebracht ist, in diese Dosierelementaussparung (90c) ragen kann. Das Dosierelement (90) umfasst zwei Dosierelementaussparungen (90c), welche etwa im 180° Winkel in Umfangsrichtung des Dosierelements (90) versetzt zueinander angeordnet sind. Das Halteglied (80d; 80d') kann als Schnapparm ausgebildet sein. Die Injektionsvorrichtung umfasst ein erstes (80d) und ein zweites Halteglied (80d') oder Schnapparm, wobei das erste (80d) und das zweite Halteglied (80d') eine erste (80e) und eine zweite nach aussen ragende Schnappnocke (80e') und eine erste (80f; beispielsweise in Figur 9a und Figur 9b ersichtlich) und eine zweite nach innen ragende Schnappnocke (80f; beispielsweise in Figur 9a und Figur 9b ersichtlich) umfassen. Das erste (80d) und das zweite Halteglied (80d') sind als elastische Schwenkarme in dem Halteelement (80) ausgebildet. Die erste (80e) und die zweite nach aussen ragende Schnappnocke (80e') und die erste (80f) und die zweite nach innen ragende Schnappnocke (80f) sind etwa auch gleicher axialen Höhe auf dem Halteelement (80) angeordnet. Die erste nach innen ragende Schnappnocke (80f) ist etwa diametral gegenüber der ersten nach aussen ragenden Schnappnocke (80e) an dem ersten Halteglied (80d) angeordnet, wobei die zweite nach innen ragende Schnappnocke (80f') etwa diametral gegenüber der zweiten nach aussen ragenden Schnappnocke (80e') an dem zweiten Halteglied (80d') vorgesehen ist. Das erste (80d) und das zweite Halteglied (80d') sind etwa im 90° Winkel in Umfangsrichtung des Halteelements (80) versetzt zueinander angeordnet. Zudem sind zwei erste (80d) und zwei zweite Halteglieder (80d') an dem Halteelement (80) vorgesehen, wobei die zwei ersten Halteglieder (80d) zueinander etwa im 180° Winkel in Umfangsrichtung des Halteelements (80) zueinander angeordnet sind und die zwei zweiten Halteglieder (80d') zueinander etwa im 180° Winkel in Umfangsrichtung des Halteelements (80) zueinander angeordnet sind. Beide ersten (80d) und beide zweiten Halteglieder (80d') umfassen je eine nach aussen ragende Schnappnocke (80e; 80e') und eine nach innen ragende Schnappnocke (80f; 80f'). Die Injektionsvorrichtung umfasst ferner ein Ausschüttelement (6), welches eine vorgespannte Feder (70) aufgenommen hat. In dem Ausschüttelement ist eine erste Ausnehmung (60a) und eine zweite Ausnehmung (60a') vorgesehen. Die erste Ausnehmung (60a) ist geeignet die erste nach innen ragende Schnappnocke (80f; beispielsweise in Figur 9a und Figur 9b ersichtlich) und die zweite Ausnehmung (60a') ist geeignet die zweite nach innen ragende Schnappnocke (80f'; beispielsweise in Figur 9a und Figur 9b ersichtlich). Die erste (60a) und die zweite Ausnehmung (60a') des Ausschüffelements (60) sind in Umfangsrichtung und in axialer Richtung teilweise versetzt zueinander angeordnet Zudem sind die erste (60a) und die zweite Ausnehmung (60a') derart teilweise axial zueinander versetzt in dem Ausschüttelement (60) angeordnet, dass sie sich in Umfangsrichtung überlappen. Das Ausschüttelelement (6) umfasst zudem mehrere erste Ausnehmungen (60a), welche axial versetzt zueinander angeordnet sind und mehrere zweite Ausnehmungen (60a'), welche ebenfalls axial versetzt zueinander angeordnet sind. Die erste (60a) und die zweite Ausnehmung (60a') sind etwa im 90° Winkel in Umfangsrichtung des Ausschüttelements (60) versetzt zueinander angeordnet. Zudem umfasst das Ausschüttelement (60) zwei erste Ausnehmungen (60a), welche etwa im 180° Winkel in Umfangsrichtung des Ausschüttelements (60) versetzt zueinander angeordnet sind, wobei das Ausschüttelement (60) jeweils mehrere erste Ausnehmungen (60a) vorsieht, die axial versetzt zueinander angeordnet sind. Ferner umfasst das Ausschüftelement (60) zwei zweite Ausnehmungen (60a'), welche etwa im 180° Winkel in Umfangsrichtung des Ausschüffelements (60) versetzt zueinander angeordnet sind, wobei das Ausschüttelement (60) jeweils mehrere zweite Ausnehmungen (60a') vorsieht, die axial versetzt zueinander angeordnet sind. Auf dem Ausschüttelement (60) ist ein proximales Spannelement (50) mit einem proximalen Spannelementring (50a) und einem proximalen Spannelementschnapper (50b) axial beweglich gelagert. Zwischen dem proximalen Spannelementring (50a) und einer Karpulenkante (20a) ist eine Feder (40) vorgesehen, welche durch eine axiale Verschiebung des proximalen Spannelements (50) relativ zu dem Gehäuse (100) in die distale Richtung spannbar ist. Der an dem proximalen Spannelement (50) vorgesehener proximaler Spannelementring (50a) kann gegen ein an dem Halteelement (80) vorgesehener Halteelementanschlag (80h; beispielsweise in Figur 9a oder Figur 9b ersichtlich) anschlagen, um ein akustisches Signal auszulösen, insbesondere ein Klick-Geräusch zu erzeugen. Zudem weist die Mantelinnenfläche des Halteelements (80) eine Halteelementnut (80j; beispielsweise in Figur 9a oder Figur 9b ersichtlich) auf, welche das distale Ende des proximalen Spannelementschnappers (50b) aufnehmen kann. Die Injektionsvorrichtung umfasst ferner eine Anzeigehülse (120) mit mindestens einer Anzeigeziffer (120a) zur Anzeige einer abgegebenen und/oder einer noch zur Verfügung stehenden festgelegten Dosis und/oder einer aufgezogenen festgelegten Dosis. Die Anzeigeziffer (120a) der Anzeigehülse (120) können durch eine Gehäuseaussparung (100j) des Gehäuses (100) ersichtlich sein. Es kann auch eine auf der Anzeigehülse (120) angebrachte Markierung und/oder ein auf der Anzeigehülse (120) vorgesehener Zwischenraum zweier Anzeigeziffern (120a) oder zweier Markierungen durch die Aussparung (100j) des Gehäuses (100) zur Anzeige einer abgegebenen und/oder einer noch zur Verfügung stehenden festgelegten Dosis und/oder einer aufgezogenen festgelegten Dosis ersichtlich sein. Das Dosierelement (90) weist an der Mantelaussenfläche ein Dosierelementaussengewinde (90h) auf, welches im Gewindeeingriff mit einem an der Mantelinnenfläche der Anzeigehülse (120) vorgesehenen Anzeigehülseinnengewinde (120d) stehen kann. Zudem umfasst die Anzeigehülse (120) an der Mantelaussenfläche ein Anzeigehülseaussengewinde (120c), welches im Gewindeeingriff mit einem an der Mantelinnenfläche des Gehäuse angebrachten Gehäuseinnengewindes (100n; beispielsweise in Figur 12 ersichtlich) stehen kann. Die beiden Gewindeverbindungen der Anzeigehülse (120) sind gegenläufig und mit unterschiedlicher Gewindesteigung ausgebildet, wobei die Gewindesteigung der Gewindeverbindung zwischen der Anzeigehülse (120) und dem Gehäuse (100) flacher ausgebildet ist, als die die Gewindesteigung der Gewindeverbindung zwischen der Anzeigehülse (120) und dem Dosierelement (90). Am proximalen Ende des Gehäuseinnengewindes (100n) ist ein Gehäuseinnengewindeanfang (100p) vorgesehen und das proximale Ende der Anzeigehülsenaussengewinde (120c) weist ein Anzelgehülseaussengewindeende (120e) auf, wobei der Gehäuseinnengewindeanfang (100p) und das Anzelgehülseaussengewindeende (120e) in Anschagkontakt gelangen können, um das Einstellen oder Aufziehen einer weiteren festgelegten Dosis zu verhindern. Ferner umfasst die Anzeigehülse (120) ein Anzeigehülseschnapparm (120b), welcher mit einer proximalen Gehäusenut oder proximalen Gehäuserampe (nicht ersichtlich) derart zusammenwirken kann, dass die Anzeigehülse (120) nur in eine Drehrichtung drehbar ist. Die Anzeigehülse kann sich relativ zu dem Gehäuse (100) axial nur in die distale Richtung bewegbar ist. Dieses Zusammenwirken kann ein akustisches Signal, insbesondere ein Klick-Geräusch erzeugen.

In der Figur 9a ist die zweite Ausführungsform der Injektionsvorrichtung in einer Anfangsposition eines Dosierelements (90) im Längsschnitt dargestellt, wobei die Figur 9b die Injektionsvorrichtung gemäss Figur 9a zeigt, wobei die Injektionsvorrichtung um die Längsachse B-B um 90° gedreht ist. Die erste nach aussen ragende Schnappnocke (80e) des ersten Halteglieds (80d) ist derart in Anschlagkontakt mit der Mantelinnenfläche des Dosierelements (90), dass die erste nach innen ragende Schnappnocke (80f) des ersten Halteglieds (80d) in die erste Ausnehmung (60a) des Ausschüttelements (60) ragt und gegen die distale Stirnseite des Verbindungsstegs zweier in axialer Richtung versetzt zueinander angeordneten ersten Ausnehmungen (60a) anstösst derart, dass die erste nach innen ragende Schnappnocke (80f) nicht ausser Eingriff mit ersten Ausnehmung (60a) des Ausschüttelements (60) gelangt. Die Mantelfläche des Dosierelements (90) liegt über der ersten nach aussen ragenden Schnappnocke (80e) des ersten Halteglieds (80d) des Halteelements (80), um zu verhindern, dass die erste nach innen ragende Schnappnocke (80f) des ersten Halteglieds (80d) des Halteelements (80) ausser Eingriff mit der ersten Ausnehmung (60a) des Ausschüttelements (60) gelangen kann. Die vorgespannte Feder (70), welche in dem Ausschüttelement (60) vorgesehen ist, ist auf der distalen Seite an dem Ausschüttelement (60) und auf der proximalen Seite von dem Halteelement abgestützt und beaufschlagt das Ausschüttelement (60) mit einer Federkraft in die distale Richtung. Der Anschlagkontakt zwischen der ersten nach innen ragenden Schnappnocke (80f) des ersten Halteglieds (80d) und der distalen Stirnseite des Verbindungsstegs zweier in axialer Richtung versetzt angeordneten ersten Ausnehmungen bewirkt eine Hemmung einer relativen Bewegung des Ausschüttelements (60) zu dem Gehäuse (10) in die distale Richtung. Die zweite nach innen ragende Schnappnocke (80f) des zweiten Halteglieds (80d') befindet sich elastisch entspannt in der zweiten Ausnehmung (60a') des Ausschüttelements (60). Die Dosierelementaussparung (90c) des Dosierelements (90) liegt über der zweiten nach aussen ragenden Schnappnocke (80e') des zweiten Halteglieds (80d'), wobei die zweite nach aussen ragende Schnappnocke (80e') des zweiten Halteglieds (80d') ausser Eingriff mit der Dosierelementaussparung (90c) des Dosierelements (90) ist. Eine proximale Stirnseite des proximalen Spannelementschnappers (50b) des proximalen Spannelements (50) hat einen Anschlagkontakt mit einer distalen Stimseite des Verbindungsstegs zweiter in axialer Richtung versetzt zueinander angeordneten zweiten Ausnehmungen (60a') des Ausschüttelements (60). Dadurch wird die Feder (40) zwischen der Karpulenkante (20a) und dem proximalen Spannelementring (50a) vorgespannt gehalten. Das proximale Spannelement (50) ist auf dem Ausschüttelement (60) axial bewegbar gelagert. Der eine proximale Spannelementschnapper (50b) ragt elastisch entspannt in die erste Ausnehmung (60a) und der andere proximale Spannelementschnapper (50b) ragt elastisch entspannt in die zweite Ausnehmung (60a') des Ausschüttelements.

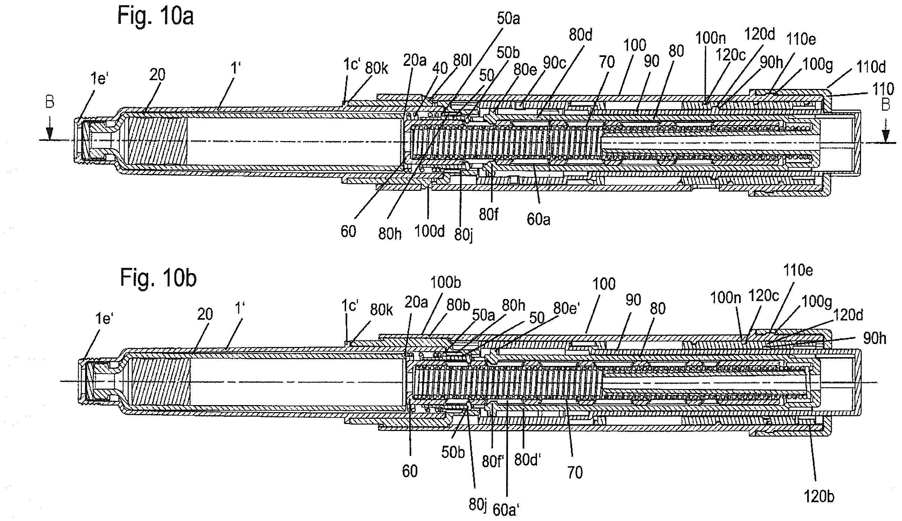

In der Figur 10a ist die zweite Ausführungsform der Injektionsvorrichtung in einer aufgezogenen Position des Dosierelements (90) im Längsschnitt dargestellt, wobei die Figur 10b die Injektionsvorrichtung gemäss Figur 10a zeigt, wobei die Injektionsvorrichtung um die Längsachse B-B um 90° gedreht ist. Zur Bereitmachung des Injektionsgeräts, dreht der Benutzer den Einstellknopf (110) mit Hilfe des Einstellknopfstegs (110d) relativ zu dem Gehäuse (100). Die drehfeste Verbindung zwischen der proximalen Dosierelementnut (90a) des Dosierelements (90) und dem Einstellknopfsteg des Einstellknopfs (110) bewirkt, dass die Drehbewegung des Einstellknopfs (110) auf das Dosierelement (90) übertragen wird. Durch den Führungseingriff oder die Kulissenführung zwischen der Dosierelementnocke (90e) des Dosierelements (90) und Halteelementsteuerkurve (80c) des Halteelements (80) bewegt sich das Dosierelement (90) relativ zu dem Gehäuse (100) in die proximale Richtung, wie in Figur 11 gezeigt wird. In Figur 11 ist zudem ersichtlich, dass am Ende der Aufziehbewegung des Dosierelements (90) der Dosierelementsteg (90b) des Dosierelements (90) in Anschlagkontakt mit einer steilen Flanke der Gehäusesteuerkurve (100f) des Gehäuserings (100k) kommt. Dieser Anschlagekontakt bewirkt eine Beschränkung der Aufziehbewegung derart, dass keine weitere festgelegte Dosis aufgezogen werden kann. Um eine weitere festgelegte Dosis einzustellen oder aufzuziehen, muss vorerst die bereits eingestellte oder aufgezogene festgelegte Dosis ausgeschüttet werden, indem das Dosierelement (90) relativ zu dem Gehäuse (100) axial in die distale Richtung bewegt wird. Dazu befindet sich die Dosierelementnocke (90e) des Dosierelements (90) in der axialen Flucht zu einer steilen Flanke der Halteelementsteuerkurve (80c) des Halteelements (80) in der aufgezogenen Position des Dosierelements (90). Ferner liegt die Dosierelementaussparung (90c) des Dosierelements (90) in axialer Flucht zu der ersten nach aussen ragenden Schnappnocke (80f) des ersten Halteglieds (80d). Die zweite nach innen ragende Schnappnocke (80f) des zweiten Halteglieds (80d') wird mit Hilfe der zweiten nach aussen ragenden Schnappnocke (80e') des zweiten Halteglieds (80d') in Eingriff mit der zweiten Ausnehmung (60a') des Ausschüttelements (60) gehalten. Während der Aufziehbewegung des Injektionsgeräts wird durch Drehung des Dosierelements (90) relativ zu dem Gehäuse (100) die Anzeigehülse (120) über die Gewindeverbindung zwischen dem Gehäuseinnengewinde (100n) des Gehäuses und dem Anzeigehülseaussengewinde (120c) der Anzeigehülse (120) und über die Gewindeverbindung zwischen dem Anzeigehülseinnengewinde (120d) der Anzeigehülse (120) und dem Dosierelementaussengewinde (90h) des Dosierelements (90) axial in die distale Richtung geschraubt. Die helixförmig angeordneten Anzeigeziffern (120a) der Anzeigehülse (120) sind durch die Gehäuseaussparung (100j) des Gehäuses (100) ersichtlich, wobei die beiden Gewindeverbindungen und die Anzeigeziffern (120a) der Anzeigehülse (120) derart ausgelegt sind, dass in der Anfangsposition des Dosierelements (90) eine andere Anzeigeziffer (120a) als in der aufgezogenen Position des Dosierelements (90) beziehungsweise in der Anfangsposition ein Zwischenraum zweier Anzeigeziffern (120a) oder eine Markierung durch die Gehäuseaussparung (100j) des Gehäuses (100) ersichtlich ist. Der Anzeigehülseschnapparm (120b) der Anzeigehülse (120) wirkt derart mit der proximalen Gehäusenut oder der proximalen Gehäuserampe des Gehäuses (100) zusammen, dass die Anzeigehüse (120) nur in eine Drehrichtung drehen kann.

In der Figur 11a ist die zweite Ausführungsform der Injektionsvorrichtung in einer injektionsausgelösten Position des Dosierelements (90) im Längsschnitt dargestellt, wobei die Figur 11b die Injektionsvorrichtung gemäss Figur 11a zeigt, wobei die Injektionsvorrichtung um die Längsachse B-B um 90° gedreht ist. Um die festgelegte Dosis auszulösen, wird das Dosierelement (90) relativ zu dem Halteelement (80) oder dem Gehäuse (100) in die distale Richtung bewegt, bis das Dosierelement (90) in Anschlagkontakt mit dem gehäusefesten Halteelement (80) gelangt. Das Dosierelement (90) bewegt sich axial relativ zu dem Halteelement (80) oder Gehäuse (100) bis die Dosierelementaussparung (90c) des Dosierelements (90) in den Bereich der ersten nach aussen ragenden Schnappnocke (80e) des ersten Halteglieds (80d) gelangt, sodass die erste nach aussen ragende Schnappnocke (80e) des ersten Halteglieds (80d) in die Dosierelementaussparung (90c) des Dosierelements (90) elastisch auslenken kann und das Ausschüttelement (60) frei gibt. Die axiale relativ Bewegung des Ausschüttelements (60) zu dem Gehäuse (100) oder dem Halteelement (80) führt dazu, dass die festgelegte Dosis aus der Injektionsvorrichtung ausgeschüttet wird, wobei die relative Bewegung durch einen Anschlagkontakt zwischen einer proximalen Stirnseite der zweiten nach innen ragenden Schnappnocke (80f') des zweiten Halteglieds (80d') und einer distalen Stirnseite des Verbindungsstegs zweier in axialer Richtung versetzt zueinander angeordneten zweiten Ausnehmungen (60a') des Ausschüttelements (60) gestoppt wird. Das Dosierelement (90) verhindert ein elastisches radial nach aussen Auslenken der zweiten nach innen ragenden Schnappnocke (80f') des zweiten Halteglieds (80d') aus der zweiten Ausnehmung (60a') des Ausschüttelements (60). Während der Ausschüttbewegung gleitet der Verbindungssteg zweiter in axialer Richtung versetzt zueinander angeordneten ersten Ausnehmungen (60a) des Ausschüttelements (60) entlang der ersten nach innen ragende Schnappnocke (80f) des ersten Halteglieds (80d) des Halteelements (80), bis die erste nach innen ragende Schnappnocke (80f) in eine erste Ausnehmung (60a) des Ausschüttelements (60) elastisch einlenkt. Ferner wird während der Ausschüttbewegung der festgelegten Dosis aus der Injektionsvorrichtung durch den Anschlagkontakt zwischen der proximalen Stirnseite des proximalen Spannelementschnapper (50b) und der distalen Stimseite des Verbindungsstegs zweiter in axialer Richtung versetzt zueinander angeordneten zweiten Ausnehmungen (60a') des Ausschüttelements (60) und durch die axiale relative Bewegung zwischen dem Ausschüttelement (60) und der Karpule (20) oder dem Halteelement (80) oder dem Gehäuse (100), die Feder (40) gespannt oder weiter vorgespannt, bis der proximale Spannelementschnapper (50b) oder zumindest ein Teil von dem proximalen Spannelementschnapper (50b) elastisch in die Halteelementnut (80j) des Halteelements radial nach aussen ausgelenkt wird. Dieser Anschlagkontakt bewirkt ein Halten der vorgespannten Feder (40). Bei weiterer axialer Relativbewegung des Ausschüttelements (60) gleitet der proximale Spannelementschnapper (50b) über den Verbindungssteg zweier in axialer Richtung versetzt zueinander angeordneten zweiten Ausnehmungen (60a') des Ausschüttelements (60), bis der proximale Spannelementschnapper (50b) in die proximal angeordnete zweite Ausnehmung (60a') der beiden in axialer Richtung versetzt zueinander angeordneten zweiten Ausnehmungen (60a') des Ausschüttelements (60) elastisch radial nach innen einlenkt. Dadurch wirkt die Federkraft der Feder (40) derart auf das proximale Spannelement (50b), dass der proximale Spannelementring (50a) des proximalen Spannelements (50b) gegen den Halteelementanschlag (80h) des Halteelements (80) anschlägt und ein akustisches Geräusch, insbesondere ein Klick-Geräusch erzeugt. Somit kann das Injektionsende der festgelegten Dosis der Injektionsvorrichtung angezeigt werden. Während der Ausschüttbewegung des Injektionsgeräts wird durch die axiale Verschiebung des Dosierelements (90) relativ zu dem Gehäuse (100) ein axialer Antrieb auf die Gewindeverbindung zwischen dem Gehäuseinnengewinde (100n) des Gehäuses (100) und dem Anzeigehülseaussengewinde (120c) der Anzeigehülse (120) und auf die Gewindeverbindung zwischen dem Anzeigehülseinnengewinde (120d) der Anzeigehülse (120) und dem Dosierelementaussengewinde (90h) des Dosierelements (90) ausgeübt, sodass die Anzeigehülse (120) axial in die distale Richtung geschraubt wird. Die Bewegung der Anzeigehülse (120) kann durch die Gehäuseaussparung (100j) des Gehäuses (100), insbesondere die Anzeigehülseziffern (120a) und/oder die Zwischenräume zwischen den Anzeigehülseziffern (120a) und/oder Markierungen gesichtet werden. Der Anzeigehülseschnapparm (120b) der Anzeigehülse (120) gelangt dabei über die proximalen Gehäusenut oder die proximalen Gehäuserampe des Gehäuses (100), sodass Zurückschrauben der Anzeigehülse (120) relativ zu dem Gehäuse (100) in die proximale Richtung verhindert werden kann.

In der Figur 12 ist eine Detailansicht des proximalen Endes der Injektionsvorrichtung der zweiten Ausführungsform in einer komplett ausgeschütteten Position des Dosierelements (90) dargestellt, wobei ein Teil des Gehäuses (100) und ein Teil des Einstellknopfs (110) ausgespart sind, sodass das Innere der Injektionsvorrichtung ersichtlich ist. Nachdem die letzte Dosis aus der Injektionsvorrichtung ausgeschüttet worden ist, ist der Gehäuseinnengewindeanfang (100p) des Gehäuseinnengewindes (100n) derart in der Nähe des Anzeigehülsenaussengewindeendes (120e) des Anzeigehülseaussengewindes (120c) angeordnet, dass beim Einstellen oder Aufziehen einer weiteren festgelegten Dosis, der Gehäuseinnengewindeanfang (100p) gegen das Anzelgehülseaussengewindeende (120e) anschlägt. Folglich kann keine weitere oder zumindest keine weitere vollständige festgelegte Dosis über die drehfeste Verbindung zwischen dem Dosierelement (90) und dem Einstellknopf (120) und über die beiden Gewindeverbindungen zwischen dem Gehäuse (100) und der Anzeigehülse (120) und zwischen der Anzeigehülse (120) und dem Dosierelement (90) aufgezogen werden.

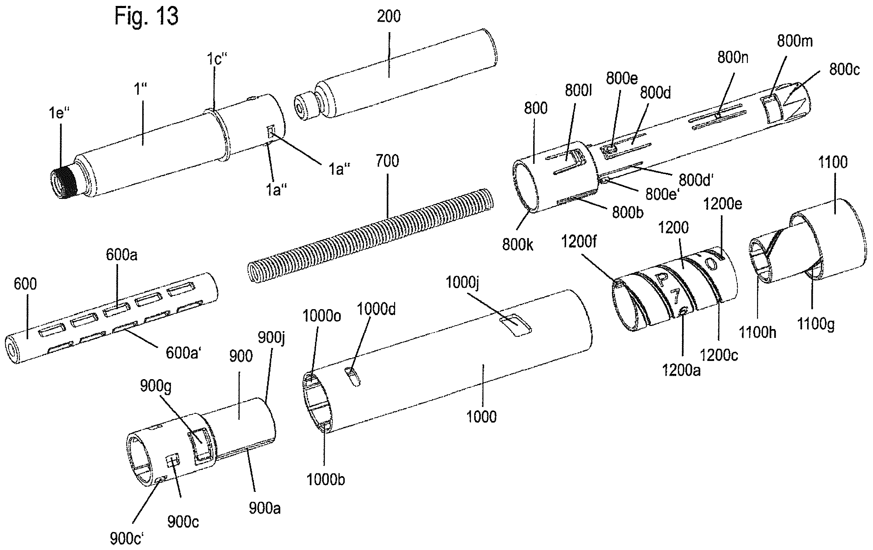

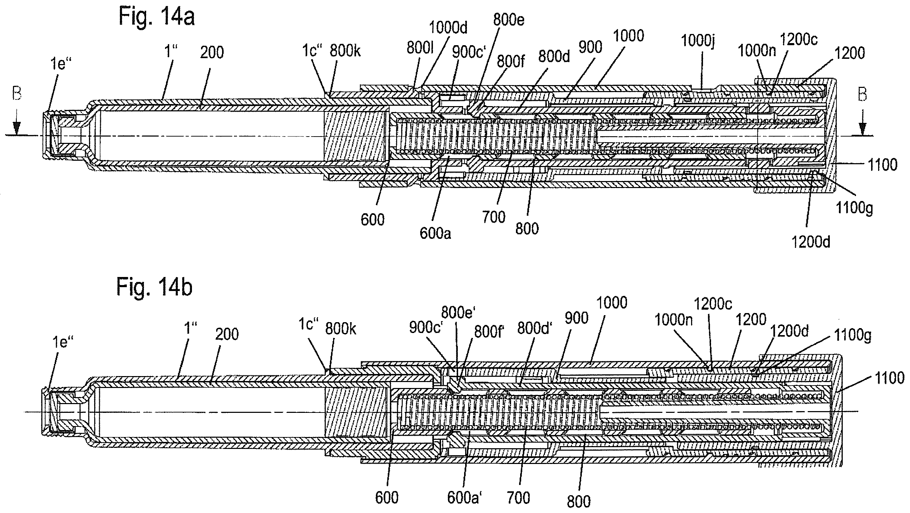

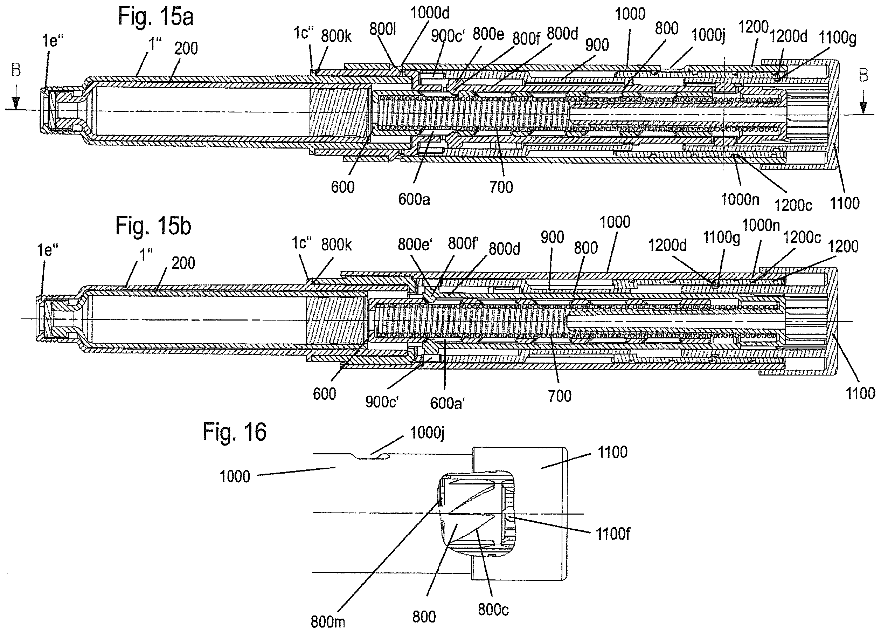

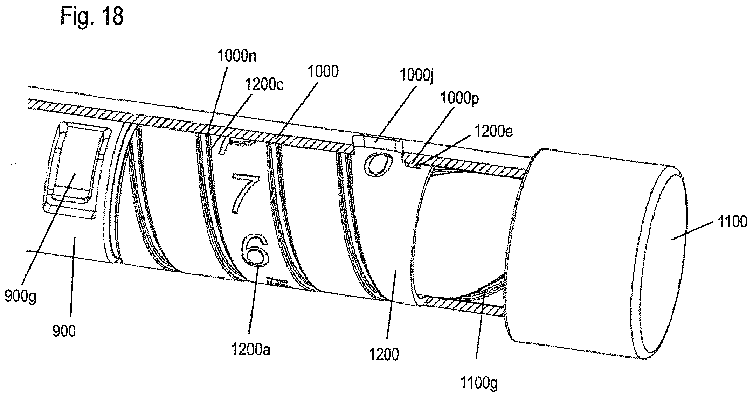

In der Figur 13 ist eine Explosionsansicht einer dritten Ausführungsform der erfindungsgemässen Injektionsvorrichtung dargestellt. Eine Karpule (200) ist von einem Karpulenhalter (1") aufgenommen, wobei der Karpulenhalter (1") über mindestens eine, in dieser Ausführungsform zwei Karpulehalternasen (1a") mit mindestens einer, in dieser Ausführungsform zwei an der Mantelinnenfläche eines Halteelements (800) vorgesehenen Halteelementaussparungen (nicht ersichtlich) axial- und drehfest befestigt ist. Eine distale Halteelementkante (800k) kann axial gegen einen Karpulenhalteranschlag (1c") anstossen. Ein Nadelverbindungselement (1e") zur lösbaren Befestigung einer Injektionsnadel ist an dem distalen Ende des Karpulenhalters (1") vorgesehen. Die Injektionsvorrichtung weist ferner ein Dosierelement (900) auf, wobei das Dosierelement (900) radial zwischen einem Gehäuse (1000) und dem Halteelement (800) angeordnet ist. Das Halteelement (800) ist mit Hilfe eines Halteelementstegs (800b) an dem Halteelement (800) mit dem Gehäuse (1000) über eine distale Gehäusenut (1000b) drehfest verbunden. Die axialfeste Verbindung zwischen dem Halteelement (800) und dem Gehäuse (1000) wird über eine Verbindung zwischen einem distalen Halteelementschnapparm (800l) und einer proximalen Gehäusenasenaussparung (1000d) vollzogen. Die Injektionsvorrichtung weist ferner einen Einstellknopf (1100) auf, der über Gewindeverbindungen mit dem Gehäuse (1000) verbunden ist. Eine Gewindeverbindung umfasst ein Einstellknopfaussengewinde (1100g) des Einstellknopfs (1100) und ein Anzeigenhülseinnengewinde (1200d, beispielsweise in Figur 14a oder Figur 14b ersichtlich) einer Anzeigehülse (1200) der Injektionsvorrichtung. An der Mantelinnenfläche des Einstellknopfs (1100) ist eine Einstellknopflängsnut oder eine Einstellknopframpe (1100h) vorgesehen, welche mit einem proximalen Halteelementschnapparm (800m) des Halteelements (800) derart zusammenwirkt, dass der Einstellknopf (1100) nur in eine Drehrichtung relativ zu dem Halteelement (800) drehbar ist. Das Zusammenwirken des proximalen Halteelementschnapparm (800m) des Halteelements (800) mit der Einstellknopflängsnut oder der Einstellknopframpe (1100h) des Einstellknopfs (1100) kann ein akustisches Geräusch, insbesondere ein Klick-Geräusch erzeugen. Der Einstellknopf (1100) umfasst an dessen Mantelinnenfläche eine Einstellknopfnocke (1100f; in Figur 16 ersichtlich), die bei Drehung des Einstellknopfs (1100) relativ zu dem Gehäuse (1000) an einer sägezahnförmigen Halteelementkurve (800c) des Halteelements (800) entlang geführt werden kann. Dieser Führungseingriff oder die Kulissenführung zwischen der Einstellknopfnocke (1100f; in Figur 16 ersichtlich), und der Halteelementkurve (800c) bewirkt bei relativer Drehung des Einstellknopfs (1100) zu dem Halteelement (800), dass der Einstellknopf (1100) in die proximale Richtung relativ zu dem Gehäuse (1000) ausgelenkt wird. Das Dosierelement (900) weist eine erste (900c) und eine zweite Dosierelementaussparung (900c') auf, welche derart ausgelegt sind, dass eine erste nach aussen ragende Halteelementschnappnocke (800e) eines ersten Halteglieds (800d) in die erste Dosierelementaussparung (900c) ragen kann und eine zweite nach aussen ragende Halteelementschnappnocke (800e') eines zweiten Halteglieds (800d'), die in die zweite Dosierelementaussparung (900c') ragen kann. Das erste (800d) und das zweite Halteglied (800d') können als Schnapparm oder als Schnappzunge ausgebildet sein und sind in Umfangrichtung und in axialer Richtung versetzt zueinander auf dem Halteelement (800) angeordnet. Das erste (800d) und das zweite Halteglied (800d') sind etwa im 90° Winkel in Umfangsrichtung des Halteelements (800) versetzt zueinander angeordnet. Die erste nach innen ragende Schnappnocke (800f; beispielsweise in Figur 14a und Figur 14b ersichtlich) ist etwa diametral gegenüber der ersten nach aussen ragenden Schnappnocke (800e) an dem ersten Halteglied (800d) angeordnet, wobei die zweite nach innen ragende Schnappnocke (800f'; beispielsweise in Figur 14a und Figur 14b ersichtlich) etwa diametral gegenüber der zweiten nach aussen ragenden Schnappnocke (800e') an dem zweiten Halteglied (800d') vorgesehen ist. Zudem sind zwei erste (800d) und zwei zweite Halteglieder (800d') an dem Halteelement (800) vorgesehen, wobei die zwei ersten Halteglieder (800d) zueinander etwa im 180° Winkel in Umfangsrichtung des Halteelements (800) zueinander angeordnet sind und die zwei zweiten Halteglieder (800d') zueinander etwa im 180° Winkel in Umfangsrichtung des Halteelements (800) zueinander angeordnet sind. Beide ersten (800d) und beide zweiten Halteglieder (800d') umfassen je eine nach aussen ragende Schnappnocke (800e; 800e') und eine nach innen ragende Schnappnocke (800f; 800f). Die erste (900c) und die zweite Dosierelementaussparung (900c') des Dosierelements (900c) sind in Umfangsrichtung und in axialer Richtung versetzt zueinander angeordnet. Die erste (900c) und die zweite Dosierelementaussparung (900c') sind etwa im 45° Winkel in Umfangsrichtung des Dosierelements (900) versetzt zueinander angeordnet. Zudem sind vier erste (900c) und vier zweite Dosierelementaussparung (900c') jeweils etwa im 90° Winkel in Umfangsrichtung des Dosierelements (900) versetzt zueinander angeordnet. Die Injektionsvorrichtung umfasst ferner ein Ausschüttelement (600), welches eine vorgespannte Feder (700) aufgenommen hat. In dem Ausschüttelement (600) ist eine erste Ausnehmung (600a) und eine zweite Ausnehmung (600a') vorgesehen. Die erste (600a) und die zweite Ausnehmung (600a') des Ausschüttelements (600) sind in Umfangrichtung versetzt zueinander angeordnet, wobei mehrere erste (600a) und mehrere zweite Ausnehmungen (600a) vorgesehen sind, die jeweils in axialer Richtung versetzt zueinander angeordnet sind. Die erste (600a) und die zweite Ausnehmung (600a) sind etwa im 90° Winkel in Umfangsrichtung des Ausschüttelements (600) versetzt zueinander angeordnet. Die erste Ausnehmung (600a) ist geeignet die erste nach innen ragende Schnappnocke (800f; beispielsweise in Figur 14a und Figur 14b ersichtlich) und die zweite Ausnehmung (600a') ist geeignet die zweite nach innen ragende Schnappnocke (800f'; beispielsweise in Figur 14a und Figur 14b ersichtlich). Zudem umfasst das Ausschüttelement (600) zwei erste Ausnehmungen (600a), welche etwa im 180° Winkel in Umfangsrichtung des Ausschüttelements (600) versetzt zueinander angeordnet sind, wobei das Ausschüttelement (600) jeweils mehrere erste Ausnehmungen (600a) vorsieht, die axial versetzt zueinander angeordnet sind. Ferner umfasst das Ausschüttelement (600) zwei zweite Ausnehmungen (600a'), welche etwa im 180° Winkel in Umfangsrichtung des Ausschüttelements (600) versetzt zueinander angeordnet sind, wobei das Ausschüttelement (600) jeweils mehrere zweite Ausnehmungen (600a') vorsieht, die axial versetzt zueinander angeordnet sind. Die Injektionsvorrichtung umfasst ferner eine Anzeigehülse (1200) mit mindestens einer Anzeigeziffer (1200a) und/oder einer Markierung und/oder einem Zwischenraum zur Anzeige einer abgegebenen und/oder einer noch zur Verfügung stehenden festgelegten Dosis. Die Anzeigeziffer (1200a) der Anzeigehülse (1200) kann durch eine Gehäuseaussparung (1000j) des Gehäuses (1000) ersichtlich sein. Ferner kann eine auf der Anzeigehülse (1200) vorgesehene Markierung und/oder einen Zwischenraum zweier Anzeigeziffern (1200a) oder Markierungen zur Anzeige einer abgegebenen und/oder einer noch zur Verfügung stehenden festgelegten Dosis durch die Gehäuseaussparung (1000j) des Gehäuses (1000) ersichtlich sein. Die Anzeigehülse (1200) umfasst das Anzeigehülseinnengewinde (1200d; beispielsweise in Figur 14a und Figur 14b ersichtlich), welches mit dem Einstellknopfaussengewinde (1100g) des Einstellknopfs im Gewindeeingriff ist. Ferner umfasst die Anzeigehülse (1200) ein Anzeigehülseaussengewinde (1200c), welches mit einem Gehäuseinnengewinde (1000n; beispielsweise in Figur 14a und Figur 14b ersichtlich) im Gewindeeingriff ist. Die Gewindesteigung der Gewindeverbindung zwischen der Anzeigehülse (1200) und dem Einstellknopf (1100) ist steiler ausgebildet als die Gewindesteigung der Gewindeverbindung zwischen der Anzeigehülse (1200) und dem Gehäuse (1100). Die Steigung der Halteelementkurve (800c) des Halteelements (800) ist gleich der Steigung des Einstellknopfaussengewindes (1100g) des Einstellknopfs (1100). Am proximalen Ende des Gehäuseinnengewindes (1000n; beispielsweise in Figur 18 ersichtlich) ist ein Gehäuseinnengewindeanfang (1000p; beispielsweise in Figur 18 ersichtlich) vorgesehen und das proximalen Ende der Anzeigehülsenaussengewinde (1200c) weist ein Anzelgehülseaussengewindeende (1200e; beispielsweise in Figur 18 ersichtlich) auf, wobei der Gehäuseinnengewindeanfang (1000p; beispielsweise in Figur 18 ersichtlich) und das Anzelgehülseaussengewindeende (1200e; beispielsweise in Figur 18) in Anschagkontakt gelangen können, um das Ausschütten einer weiteren festgelegten Dosis zu verhindern. Die Anzeigehülse (1200) weist zudem an deren Mantelinnenfläche einen Anzeigehülsesteg (1200f) auf, welcher in Eingriff mit einer proximalen Dosierelementnut (900a) zur drehfesten Verbindung ist. Eine Drehung der Anzeigehülse (1200) bewirkt somit eine Drehung des Dosierelements (900), wobei während der Drehung ein Zusammenwirken eines Dosierelementschnapparm (900g) an dem Dosierelement (900) mit einer distalen Gehäuserampe (1000o) an dem Gehäuse (1000) dazu führt, dass das Dosierelement (900) nur in eine Drehrichtung gedreht werden kann. Damit eine axiale Verschiebung des Dosierelements (900) relativ zu dem Gehäuse (1000) verhindert wird, ist an dem Halteelement (800) eine Halteelementnocke (800n) vorgesehen, welche gegen eine proximale Dosierelementkante (900j) des Dosierelements axial anstösst. Die Halteelementnocke (800n) ist über einen Federsteg zwecks einfacherer Montage der Injektionsvorrichtung an dem Halteelement (800) angebracht.

In der Figur 14a ist die dritte Ausführungsform der Injektionsvorrichtung in einer Anfangsposition eines Einstellknopfs (1100) im Längsschnitt dargestellt, wobei die Figur 14b die Injektionsvorrichtung gemäss Figur 14a zeigt, wobei die Injektionsvorrichtung um die Längsachse B-B um 90° gedreht ist. Die erste nach aussen ragende Schnappnocke (800e) des ersten Halteglieds (800d) des Halteelements (800) ist in Anschlagkontakt mit der Mantelinnenfläche des Dosierelements (900), wobei die erste nach innen ragende Schnappnocke (800f) des ersten Halteglieds (800d) in die erste Ausnehmung (600a) des Ausschüttelements (600) ragt und gegen eine distale Stirnseite des Verbindungsstegs zweier in axialer Richtung versetzt angeordneten ersten Ausnehmungen (600a) anstösst. Die erste nach innen ragende Schnappnocke (800f) ist in Eingriff mit ersten Ausnehmung (600a) des Ausschüttelements (600). Das Ausschüttelement (600), welches durch die vorgespannte Feder (700) mit einer Kraft in die distale Richtung beaufschlagt wird, wird durch den Anschlagkontakt zwischen der ersten nach innen ragenden Schnappnocke (800f) und dem Verbindungssteg zweier in axialer Richtung versetzt zueinander angeordneten ersten Ausnehmungen (600a) des Ausschüftelements (600) an einer relativen axialen Bewegung zu dem Gehäuse (1000) in die distale Richtung gehindert. Die zweite Dosierelementaussparung (900c') des Dosierelements (900) liegt über der zweiten nach aussen ragenden Schnappnocke (800e') des zweiten Halteglieds (80d'), wobei die zweite nach aussen ragende Schnappnocke (800e') des zweiten Halteglieds (800d') ausser Eingriff mit der zweiten Dosierelementaussparung (900c') des Dosierelements (900) ist. Die zweite nach innen ragende Schnappnocke (800f') des zweiten Halteglieds (800d') ragt elastisch entspannt in die zweite Ausnehmung (600a') des Ausschüttelements (600).

In der Figur 15a ist die dritte Ausführungsform der Injektionsvorrichtung in einer aufgezogenen Position des Einstellknopfs (1100) im Längsschnitt dargestellt, wobei die Figur 15b die Injektionsvorrichtung gemäss Figur 15a zeigt, wobei die Injektionsvorrichtung um die Längsachse B-B um 90° gedreht ist. Um die festgelegte Dosis aufzuziehen oder einzustellen, wird der Einstellknopf (1100) relativ zu dem Gehäuse (1000) oder dem Halteelement (800) gedreht. Da die Einstellknopfnocke (1100f), wie in Figur 16 ersichtlich ist, in einem Führungseingriff oder einer Kulissenführung mit der Halteelementsteuerkurve (800e) des Halteelements (800) ist, wird durch die relative Drehung des Einstellknopfs (1100) zu dem Gehäuse (1000) oder dem Halteelement (800) der Einstellknopf (1100) in die proximale Richtung geschraubt, bis die Einstellknopfnocke (1100f; beispielsweise in Figur 16 ersichtlich) in axialer Flucht zu einer steilen Flanke der der Halteelementsteuerkurve (800c) des Halteelements (800) gelangt und der proximale Halteelementschnapparm (800m) des Halteelements (800) in die Einstellknopflängsnut oder Einstellknopframpe (1100h) des Einstellknopfs (1100) rastet. Da die Steigung der Halteelementsteuerkurve (800c) des Halteelements (800) gleich gross ist wie die Steigung der Gewindeverbindung des Anzeigehülseinnengewindes (1200d) und des Einstellknopfaussengewindes (1100g), dreht die Anzeigehülse (1200) während der Einstellbewegung oder der Aufziehbewegung des Einstellknopfs (1100) nicht relativ zu dem Gehäuse (1000) oder dem Halteelelement (800). Da während der Einstellbewegung oder Aufziehbewegung des Einstellknopfs (1100) weder das Halteelement (800) noch das Dosierelement (900) eine relative Bewegung zu dem Gehäuse (1000) vollführt, verbleibt während der Einstellbewegung oder der Aufziehbewegung des Einstellknopfs (1200) die erste nach aussen ragende Schnappnocke (800e) des ersten Halteglieds (800d) des Halteelements (800) in Anschlagkontakt mit der Mantelinnenfläche des Dosierelements (900), wobei die erste nach innen ragende Schnappnocke (800f) des ersten Halteglieds (800d) in die erste Ausnehmung (600a) des Ausschüftelements (600) ragt und gegen die distale Stirnseite des Verbindungsstegs zweier in axialer Richtung versetzt angeordneten ersten Ausnehmungen anstösst. Zudem verbleibt die erste nach innen ragende Schnappnocke (800f) in Eingriff mit ersten Ausnehmung (600a) des Ausschüttelements (600), wobei die zweite nach innen ragende Schnappnocke (800f) des zweiten Halteglieds (800d') elastisch entspannt in die zweite Ausnehmung (600a') des Ausschüftelements (600) ragt. Zudem liegt weiterhin die zweite Dosierelementaussparung (900c') des Dosierelements (900) über der zweiten nach aussen ragenden Schnappnocke (800e') des zweiten Halteglieds (80d'), wobei die zweite nach aussen ragende Schnappnocke (800e') des zweiten Halteglieds (800d') ausser Eingriff mit der zweiten Dosierelementaussparung (900c') des Dosierelements (900) ist.

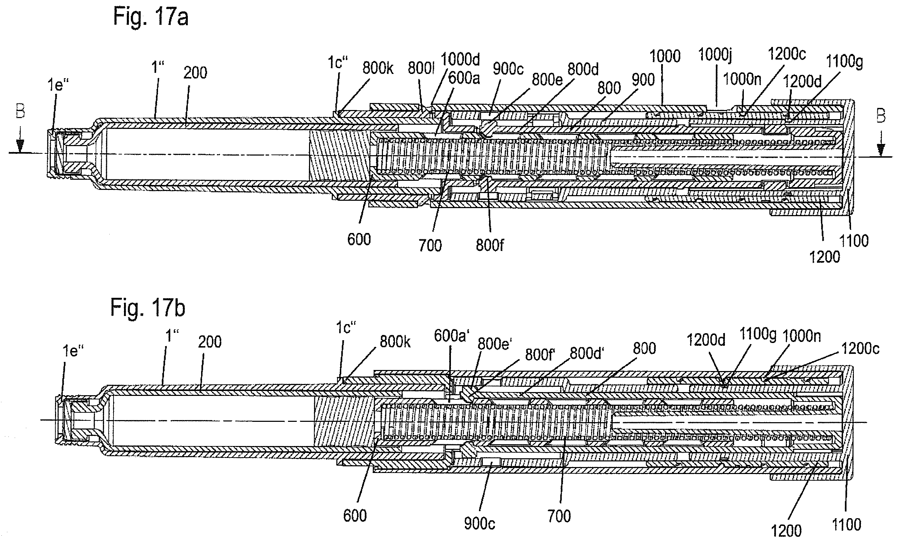

In der Figur 17a ist die dritte Ausführungsform der Injektionsvorrichtung in einer injektionsausgelösten Position des Einstellknopfs (1100) im Längsschnitt dargestellt, wobei die Figur 17b die Injektionsvorrichtung gemäss Figur 17a zeigt, wobei die Injektionsvorrichtung um die Längsachse B-B um 90° gedreht ist. Um die festgelegte Dosis auszulösen, wird der Einstellknopf (1100) axial relativ zu dem Gehäuse (1000) in die distale Richtung bewegt. Aufgrund des axialen Drucks, welcher von dem Einstellknopf (1100) über die beiden Gewindeverbindung zwischen der Anzeigehülse (1200) und dem Einstellknopf (1100) und zwischen der Anzeigehülse (1200) und dem Gehäuse (1000) auf die Anzeigehülse (1200) in distale Richtung wirkt, dreht sich die Anzeigehülse (1200). Die Anzeigehülse (1200) bewegt sich drehend relativ zu dem Gehäuse (1000) in die distale Richtung. Die drehfeste Verbindung zwischen der Anzeigehülse (1200) und Dosierelement (900), nämlich die Verbindung zwischen der proximalen Dosierelementnut (900a) und dem Anzeigehülsesteg (1200f), bewirkt, dass während dem Ausschüttvorgang der festgelegten Dosis das Dosierelement (900) relativ zu dem Gehäuse (1000) oder dem Halteelement (800) dreht. Das Dosierelement (900) dreht relativ zu dem Gehäuse (1000) oder dem Halteelement (800) um etwa einen 45° Winkel, sodass einerseits eine erste Dosierelementaussparung (900c) über die erste nach aussen ragende Schnappnocke (800e) des ersten Halteglieds (800d) des Halteelements (800) und andererseits eine andere erste Dosierelementaussparung (900c) axial versetzt zu der zweiten nach aussen ragenden Schnappnocke (800e') des ersten Halteglieds (800d') des Halteelements (800) zu liegen kommt. Die Mantelfläche des Dosierelements (900) liegt über der zweiten nach aussen ragenden Schnappnocke (800e') des zweiten Halteglieds (800d') des Halteelements (800), wobei das Dosierelement über der zweiten nach aussen ragenden Schnappnocke (800e') verhindert, dass die zweite nach innen ragende Schnappnocke (800f') des zweiten Halteglieds (800d') ausser Eingriff mit der zweiten Aussparung (600a') des Ausschüttelements (600) gelangt.

In der Figur 18 ist eine Detailansicht des proximalen Endes der Injektionsvorrichtung der dritten Ausführungsform in einer komplett ausgeschütteten Position des Einstellknopfs (1100) dargestellt, wobei ein Teil des Gehäuses (1000) und ein Teil des Einstellknopfs (1100) ausgespart sind, sodass das Innere der Injektionsvorrichtung ersichtlich ist. Nachdem die letzte Dosis aus der Injektionsvorrichtung ausgeschüttet worden ist, liegt der Gehäuseinnengewindeanfang (1000p) des Gehäuseinnengewindes (1000n) in der Nähe des Anzeigehülsenaussengewindeende (1200e) des Anzeigehülseaussengewindes (1200c). Während der Einstellbewegung oder Aufziehbewegung des Einstellknopfs (1100) einer weiteren festgelegten Dosis, dreht sich die Anzeigehülse (1200) nicht relativ zu dem Gehäuse (1000) oder dem Halteelement (900), wobei der Einstellknopf (1100) relativ zu dem Gehäuse (1000) oder dem Halteelement (900) in die proximale Richtung geschraubt wird. Somit bewegt sich die Anzeigehülse (1200) während dieser Bewegung nicht axial in die distale Richtung relativ zu dem Gehäuse (1000) oder dem Halteelement (900). Während der Auslösebewegung des Einstellknopfs (1100) zur Abgabe einer weiteren festgelegten Dosis, schlägt der Gehäuseinnengewindeanfang (1000p) des Gehäuses (1000) gegen das Anzeigehülseaussengewindeende (1200e) an, sodass die Anzeigehülse (1200) keine relative Drehung zu dem Gehäuse (1000) oder dem Halteelement (900) vollziehen kann und folglich das Dosierelement (900) ebenfalls nicht relativ zu dem Gehäuse (1000) oder dem Halteelement (900) gedreht wird. Folglich kann keine weitere festgelegte Dosis aus der Injektionsvorrichtung ausgeschüttet werden.

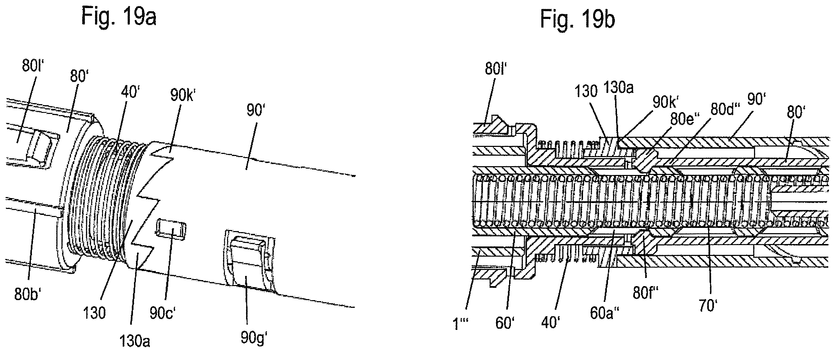

In den Figuren 19a bis 21a ist ein Mechanismus zur Anzeige des Injektionsendes in den verschiedenen Stadien dargestellt, wobei die Mechanik dieser Anzeige beispielsweise in ein Injektionsgerät gemäss der dritten Ausführungsform eingebaut werden kann.

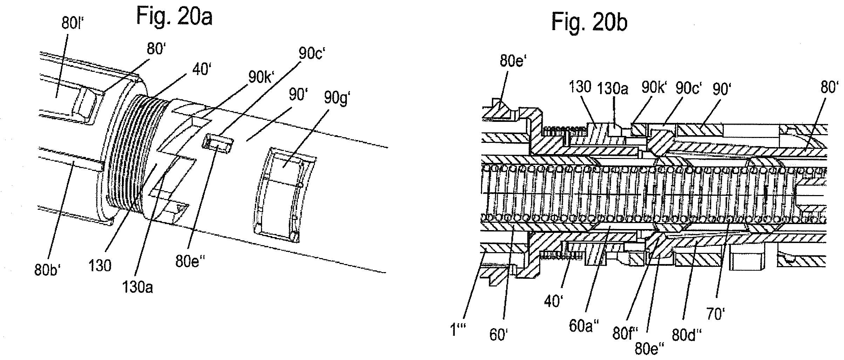

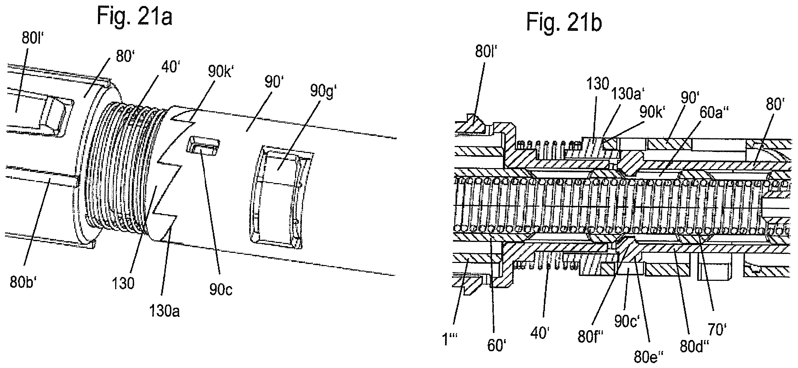

Das Injektionsgerät kann eine Feder (40') aufweisen, welche distal an einem gehäusefesten Halteelement (80') und proximal an einem Klick-Element (130) abgestützt ist. Das Halteelement (80') ist über einen distalen Halteelementschnapparm (80l') und über einen Halteelementsteg (80b') axial- und drehfest mit einem Gehäuse verbunden. Das Halteelement (80') ist zudem mit einem Karpulenhalter (1''') axial- und drehfest fixiert. Das Klick-Element (130) ist drehfest mit dem Halteelement (80') verbunden. Das Klick-Element (130) ist axial bewegbar auf dem Halteelement (80') gelagert. Das Klick-Element (130) wird durch eine Federkraft gegen eine distale Stirnseite des Dosierelements (90') vorgespannt. An einem distalen Ende des Dosierelements (90') ist eine sägezahnförmige Dosierelementsteuerkurve (90k') und an dem proximalen Ende des Klick-Elements (130) ist eine sägezahnförmige Klick-Elementsteuerkurve (130a) vorgesehen. Die sägezahnförmige Dosierelementsteuerkurve (90k') des Dosierelements (90') und die sägezahnförmige Klick-Elementsteuerkurve (130a) des Klick-Elements (130) sind komplementär zueinander ausgebildet und können ineinander greifen. In der Anfangsposition des Einstellknopfs greift die sägezahnförmige Dosierelementsteuerkurve (90k') in die Klick-Elementsteuerkurve (130a) ein, wie in Figur 19a und in Figur 19b dargestellt ist. Die Mantelfläche des Dosierelements liegt über der ersten nach aussen ragenden Schnappnocke (80e") des ersten Halteglieds (80d") des Halteelements (80') derart, dass die erste nach innen ragende Schnappnocke (80f") des ersten Halteglieds (80d") des Halteelements (80') nicht ausser Eingriff mit einer ersten Ausnehmung (60a") eines Ausschüftelements (60') gelangen kann. Das mit einer vorgespannten Feder (70') in die distale Richtung vorgespannte Ausschüttelement (60') wird durch den Anschlagkontakt zwischen einer proximalen Stirnfläche des ersten nach innen ragenden Schnappnocke (80f') des ersten Halteglieds (80d") des Halteelements (80') und einer distalen Fläche eines Verbindungsstegs zweier in axialer Richtung versetzt zueinander angeordneten ersten Ausnehmungen (60a") des Ausschüttelements (60) an einer axialen relativen Bewegung zu dem Gehäuse oder dem Halteelement (80') in die distale Richtung gehindert. Während dem Ausschüttvorgang der festgelegten Dosis aus der Injektionsvorrichtung, wie in Figur 20a und in Figur 20b dargestellt, dreht das Dosierelement (90') relativ zu dem Halteelement (90'). Das Dosierelement (90') kann aufgrund der Verbindung zwischen einem Dosierelementschnapparm (90g') zu einer Gehäuserampe des Gehäuses nur in eine Drehrichtung gedreht werden. Aufgrund des sägezahnförmigen Eingriffs zwischen dem Klick-Element (130) und dem Dosierelement (90') und der drehfesten Verbindung zwischen dem Klick-Element (130) und dem Halteelement (80') kann das Klick-Element (130) axial relativ zu dem Gehäuse oder dem Halteelement (90') in die distale Richtung bewegt werden, sodass die Feder (40') weiter vorgespannt wird. Durch die relative Drehung des Dosierelements (90') gelangt die erste Dosierelementaussparung (90c') über die erste nach aussen ragende Schnappnocke (80e") des ersten Halteglieds (80d") des Halteelements (80), wobei die erste nach aussen ragende Schnappnocke (80e") elastisch in die erste Dosierelementaussparung (90c') ausgelenkt wird und die erste nach innen ragende Schnappnocke (80f") des ersten Halteglieds (80d") des Halteelements (80) ausser Eingriff mit der ersten Ausnehmung (60a') des Ausschüttelements (60') gelangt. Dieses Auslenken der ersten nach aussen ragenden Schnappnocke (80e") in die erste Dosierelementaussparung (90c') bewirkt, dass das Klickelement in der distalen Position gehalten werden kann. Der Verbindungssteg zweier in axialer Richtung versetzt zueinander erster Ausnehmungen (60a') gleitet entlang der ersten nach innen ragenden Schnappnocke (80f") des ersten Halteglieds (80d') des Halteelements (80) bis die erste nach innen ragende Schnappnocke (80f") in proximalere erste Ausnehmung (60a') des Ausschüttelements (60') elastisch einlenkt, wie in der Figur 21a und in der Figur 21 b gezeigt. Das Klick-Element (130) wird nicht mehr in der distalen Position gehalten und kann durch die Kraft der vorgespannten Feder (40') mit der Klick-Elementsteuerkurve (130a) gegen die Dosierelementsteuerkurve (90k') des Dosierelements (90') anstossen. Dieser Anschlag kann ein akustisches Signal, insbesondere eine Klick-Geräusch auslösen. Dieses akustische Signal zeigt das Injektionsende der festgelegten Dosis an. Das Ausschüttelement (60') wird aufgrund eines Anschlagskontakt zwischen einer zweiten nach innen ragenden Schnappnocke eines zweiten Halteglieds (nicht ersichtlich) des Haltelements (80) und einer distalen Stirnseite eines Verbindungsstegs zweiter in axialer Richtung versetzt zueinander angeordneten zweiten Ausnehmungen (nicht ersichtlich) des Ausschüttelements (60') an einer weiteren axialen Relativbewegung zu dem Gehäuse oder dem Haltelement (80') gehindert.

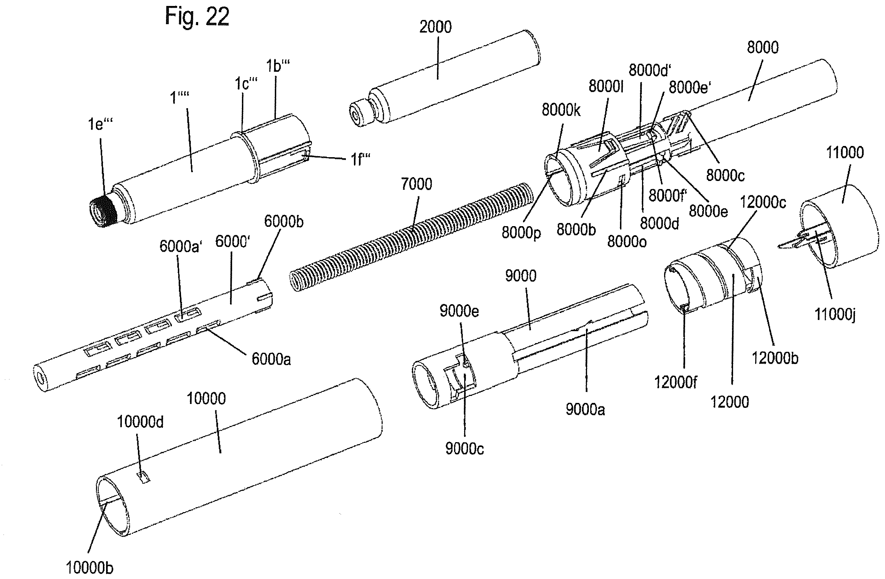

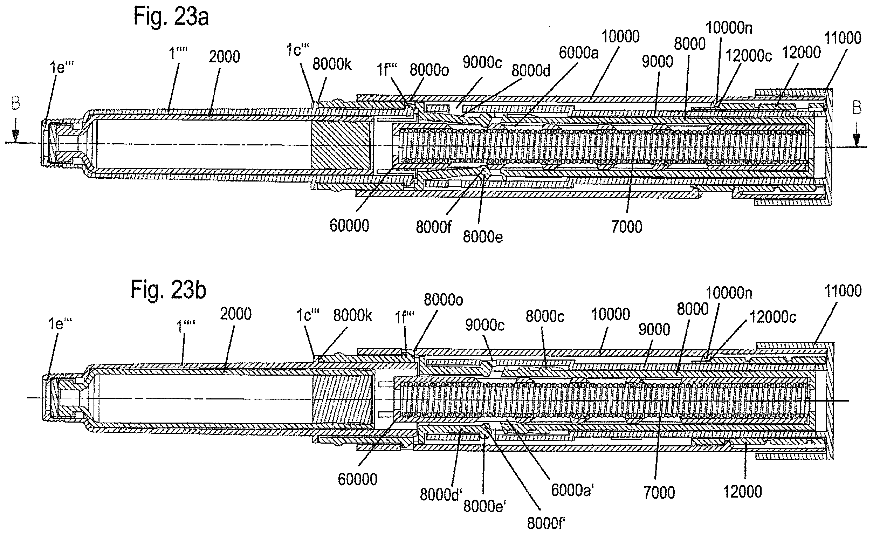

In der Figur 22 ist eine Explosionsansicht einer vierten Ausführungsform der erfndungsgemässen Injektionsvorrichtung dargestellt. Ein Karpulenhalter (1"") hat eine Karpule (2000) aufgenommen, wobei der Karpulenhalter (1'''') über einen Karpulenhalterschnapparm (1f") und einen Karpulenhaltersteg (1b''') mit einer an der Mantelinnenfläche eines Halteelements (8000) vorgesehene Halteelementaussparungen (8000o) und einer an der Mantelinnenfläche des Haltelements (8000) vorgesehenen distalen Halteelementnut (8000p) axial- und drehfest verbunden ist. Ein Karpulenhalteranschlag (1c''') kann gegen eine distale Halteelementkante (8000k) axial anstossen. Ein Nadelverbindungselement (1e''') zur lösbaren Befestigung einer Injektionsnadel ist an dem distalen Ende des Karpulenhalters (1'''') vorgesehen. Die Injektionsvorrichtung weist ferner ein Dosierelement (9000) auf, wobei das Dosierelement (9000) radial zwischen einem Gehäuse (10000) und dem Halteelement (8000) angeordnet ist. Das Halteelement (8000) ist mit Hilfe eines Halteelementstegs (8000b) an dem Halteelement (8000) mit dem Gehäuse (10000) über eine distale Gehäusenut (10000b) drehfest verbunden. Die axialfeste Verbindung zwischen dem Halteelement (8000) und dem Gehäuse (10000) wird über eine Verbindung zwischen einem distalen Halteelementschnapparm (8000l) und einer proximalen Gehäusenasenaussparung (10000d) vollzogen. Die Injektionsvorrichtung weist ferner einen Einstellknopf (11000) auf, der über ein Einstellknopfsicherungselement (11000j) des Einstellknopfs (11000) mit einem innerhalb des Dosierelements (9000) angeordneten Dosierelementgegensicherungselement (nicht ersichtlich) axial- und drehfest gesichert ist. Die Injektionsvorrichtung umfasst ferner eine Anzeigehülse (12000), welche eine Gewindeverbindung zu dem Gehäuse (10000) aufweist. Dazu hat die Anzeigehülse (12000) ein Anzeigehüseaussengewinde (12000c), welches in Eingriff mit einem Gehäuseinnengewinde (10000n; beispielsweise in Figur 23a oder Figur 23b ersichtlich) des Gehäuses (10000) ist. Die Anzeigehülse (1200) weist zudem an deren Mantelinnenfläche einen Anzeigehülsensteg (12000f) auf, welcher in Eingriff mit einer proximalen Dosierelementnut (9000a) des Dosierelements (9000) zur drehfesten Verbindung ist. Eine Drehung des Dosierelements (9000) bewirkt somit eine Drehung der Anzeigehülse (12000). Die Anzeigehülse (12000) weist zudem einen Anzeigehülseschnapparm (12000b) auf, welcher mit einer proximalen Gehäusenut oder proximalen Gehäuserampe (nicht ersichtlich) derart zusammenwirken kann, dass die Anzeigehülse (12000) nur in eine Drehrichtung drehbar ist. Dieses Zusammenwirken kann ein akustisches Geräusch, insbesondere ein Klick-Geräusch erzeugen, um das Einstellen oder das Aufziehen der festgelegten Dosis akustisch anzuzeigen. Alternativ kann die Anzeigehülse (12000) zur visuellen Anzeige des Einstellens oder des Aufziehens der festgelegten Dosis eine Anzeigeziffer und/oder eine Markierung und/oder einen Zwischenraum (nicht dargestellt) aufweisen, welche durch eine Gehäuseaussparung (nicht dargestellt) des Gehäuses (10000) visuell ersichtlich sein kann. Das Dosierelement (9000) weist eine Dosierelementnocke (9000e) auf, weiche in einem Führungseingriff oder in einer Kulissenführung mit einer Halteelementsteuerkurve (8000c) des Halteelements (8000) ist. Die Halteelementsteuerkurve (8000c) des Halteelements (8000) ist derart ausgebildet, dass durch eine relative Drehung des Dosierelements (9000) das Dosierelement (9000) relativ zu dem Gehäuse (10000) axial in die proximale Richtung bewegbar ist. In dem Dosierelement (9000) ist eine Dosierelementaussparung (9000c) vorgesehen, welche geeignet ist, eine erste (8000e) und/oder eine zweite nach aussen ragende Schnappnocke (8000e') eines ersten (8000d) und eines zweiten Halteglieds (8000d') des Halteelements (8000) aufzunehmen. Das erste (8000d) und das zweite Halteglied (8000d') können als Schnapparm oder als Schnappzunge ausgebildet sein. Das erste (8000d) und das zweite Halteglied (8000d') sind etwa im 90° Winkel in Umfangsrichtung des Halteelements (8000) versetzt zueinander angeordnet. Eine erste nach innen ragende Schnappnocke (8000f; beispielsweise in Figur 23a und Figur 23b ersichtlich) ist etwa diametral gegenüber die ersten nach aussen ragenden Schnappnocke (8000e) an dem ersten Halteglied (8000d) angeordnet, wobei eine zweite nach innen ragende Schnappnocke (8000f; beispielsweise in Figur 23a und Figur 23b ersichtlich) etwa diametral gegenüber der zweiten nach aussen ragenden Schnappnocke (8000e') an dem zweiten Halteglied (8000d') vorgesehen ist. Zudem sind zwei erste (8000d) und zwei zweite Halteglieder (8000d') an dem Halteelement (8000) vorgesehen, wobei die zwei ersten Halteglieder (8000d) zueinander etwa im 180° Winkel in Umfangsrichtung des Halteelements (8000) zueinander angeordnet sind und die zwei zweiten Halteglieder (8000d') zueinander etwa im 180° Winkel in Umfangsrichtung des Halteelements (8000) zueinander angeordnet sind. Beide ersten (8000d) und beide zweiten Halteglieder (8000d') umfassen je eine nach aussen ragende Schnappnocke (8000e; 8000e') und eine nach innen ragende Schnappnocke (8000f; 8000f'). Die erste (8000e) und die zweite nach aussen ragende Schnappnocke (8000e') und die erste (8000f) und die zweite nach innen ragende Schnappnocke (8000f') können als Schnapphaken ausgebildet sein. Die Injektionsvorrichtung umfasst ferner ein Ausschüttelement (6000), welches eine vorgespannte Feder (7000) aufgenommen hat. In dem Ausschüttelement (6000) ist eine erste Ausnehmung (6000a) und eine zweite Ausnehmung (6000a') vorgesehen. Die erste (6000a) und die zweite Ausnehmung (6000a') des Ausschüttelements (6000) sind in Umfangrichtung und in axialer Richtung versetzt zueinander angeordnet, wobei mehrere erste (6000a) und mehrere zweite Ausnehmungen (6000a) vorgesehen sind, die jeweils in axialer Richtung versetzt zueinander angeordnet sind. Die erste (6000a) und die zweite Ausnehmung (6000a) sind etwa im 90° Winkel in Umfangsrichtung des Ausschüftelements (6000) versetzt zueinander angeordnet. Die erste Ausnehmung (6000a) ist geeignet die erste nach innen ragende Schnappnocke (8000f; beispielsweise in Figur 23a und Figur 23b ersichtlich) und die zweite Ausnehmung (6000a') ist geeignet die zweite nach innen ragende Schnappnocke (8000f'; beispielsweise in Figur 23a und Figur 23b ersichtlich) aufzunehmen. Die erste (6000a) und die zweite (6000a') Ausnehmung des Ausschüttelements (6000) überlappen in axialer Richtung zueinander. Zudem umfasst das Ausschüttelement (6000) zwei erste Ausnehmungen (6000a), welche etwa im 180° Winkel in Umfangsrichtung des Ausschüttelements (6000) versetzt zueinander angeordnet sind, wobei das Ausschüttelement (6000) jeweils mehrere erste Ausnehmungen (6000a) vorsieht, die axial versetzt zueinander angeordnet sind. Ferner umfasst das Ausschüttelement (6000) zwei zweite Ausnehmungen (6000a'), welche etwa im 180° Winkel in Umfangsrichtung des Ausschüttelements (6000) versetzt zueinander angeordnet sind, wobei das Ausschüttelement (6000) jeweils mehrere zweite Ausnehmungen (6000a') vorsieht, die axial versetzt zueinander angeordnet sind. Das Ausschüttelement (6000) umfasst ferner einen Ausschüttelementsteg (6000b), der mit einer an der Mantelinnenfläche vorgesehenen Halteelementlängsnut (nicht ersichtlich) eine drehfeste Verbindung bildet.

In der Figur 23a ist die vierte Ausführungsform der Injektionsvorrichtung in einer Anfangsposition eines Dosierelements (9000) im Längsschnitt dargestellt, wobei die Figur 23b die Injektionsvorrichtung gemäss Figur 23a zeigt, wobei die Injektionsvorrichtung um die Längsachse B-B um 90° gedreht ist. Das erste Halteglied (8000d) wird mit der ersten nach innen ragende Schnappnocke (8000f) über die Mantelfläche des Dosierelements (9000) und die erste nach aussen ragende Schnappnocke (8000e) des ersten Halteglieds (8000d) des Halteelements (8000) elastisch in die erste Ausnehmung (6000a) des Ausschüttelements (6000) gespannt, sodass die erste nach innen ragende Schnappnocke (8000f) im Eingriff mit der ersten Ausnehmung (6000a) des Ausschüftelements (6000) ist. Eine proximale Stirnseite der ersten nach innen ragenden Schnappnocke (6000f) des ersten Halteglieds (8000d) des Halteelements (8000) ist in Anschlagkontakt mit einem Verbindungssteg zweier in axialer Richtung versetzt zueinander angeordneten ersten Ausnehmungen (6000a) des Ausschüttelements (6000), sodass das mit der Feder (7000) vorgespannte Ausschüttelement (6000) an einer axialen Relativbewegung zu dem Gehäuse (10000) oder dem Halteelement (8000) gehindert wird. Das zweite Halteglied (8000d') ist elastisch entspannt, sodass die zweite nach aussen ragende Schnappnocke (8000e') in die Dosierelementaussparung (9000c) des Dosierelements (9000) ragt und die zweite nach innen ragende Schnappnocke (8000f') ausser Eingriff mit der zweiten Ausnehmung (6000a') des Ausschüttelements (6000) ist. Die Dosierelementnocke (9000e) des Dosierelements (9000) ist in dem Führungseingriff oder in der Kulissenführung mit der Halteelementsteuerkurve (8000c) des Halteelements (8000).

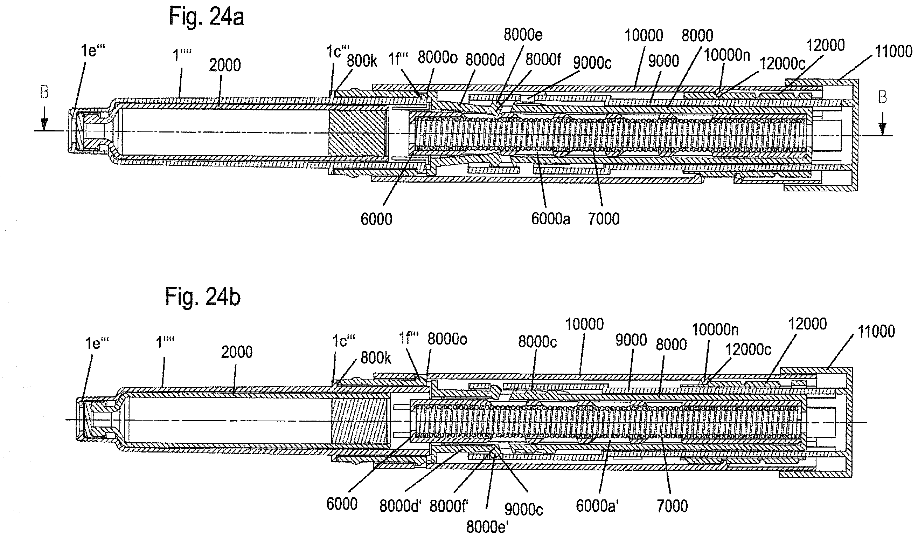

In der Figur 24a ist die vierten Ausführungsform der Injektionsvorrichtung in einer aufgezogenen Position des Dosierelements (9000) im Längsschnitt dargestellt, wobei die Figur 24b die Injektionsvorrichtung gemäss Figur 24a zeigt, wobei die Injektionsvorrichtung um die Längsachse B-B um 90° gedreht ist. Um das Injektionsgerät injektionsbereit zu machen, dreht der Benutzer den Einstellknopf (11000) relativ zu dem Gehäuse (10000). Durch die drehfeste Verbindung zwischen dem Einstellknopf (11000) und dem Dosierelement (9000) wird das Dosierelement (9000) und der Einstellknopf (11000) über den Führungseingriff oder der Kulissenführung zwischen der Dosierelementnocke (9000e) des Dosierelements (9000) und der Halteelementsteuerkurve (8000c) des gehäusefesten Halteelements (8000) relativ zu dem Gehäuse (10000) oder dem Halteelement (8000) in die proximale Richtung bewegt. Da die Halteelementsteuerkurve (8000c) des Halteelements (8000) schraubenförmig ausgebildet ist, werden der Einstellknopf (11000) und das Dosierelement (9000) in die proximale Richtung aus dem Gehäuse (10000) herausgeschraubt. Die Anzeigehülse (12000) wird aufgrund der drehfesten Verbindung zwischen dem Dosierelement (9000) und der Anzeigehülse (12000) und der Gewindeverbindung zwischen der Anzeigehülse (12000) und dem Gehäuse (10000) in die distale Richtung relativ zu dem Gehäuse (1000) geschraubt. In der aufgezogenen Position des Dosierelements (9000) wird die erste nach innen ragende Schnappnocke (8000e) des ersten Halteglieds (8000d) immer noch über die erste nach aussen ragende Schnappnocke (8000f) des ersten Halteglieds (8000d) von dem Dosierelement (9) in Eingriff mit der ersten Ausnehmung (6000a) des Ausschüftelements (6000) gehalten, wobei durch die relative Drehbewegung des Dosierelements (9000) die Dosierelementaussparung (9000c) des Dosierelements (9000) in axialer Flucht und in proximaler Richtung versetzt zur ersten nach aussen ragenden Schnappnocke (8000e) des ersten Halteglieds (8000d) zu liegen kommt. Zudem verbleibt die zweite nach aussen ragende Schnappnocke (8000f') des zweiten Halteglieds (8000d') in der Dosierelementaussparung (9000c) des Dosierelements (9000), sodass die zweite nach innen ragende Schnappnocke (8000f') des zweiten Halteelements (8000d') ausser Eingriff mit der zweiten Ausnehmung (6000a') des Ausschüttelements (6000) ist.

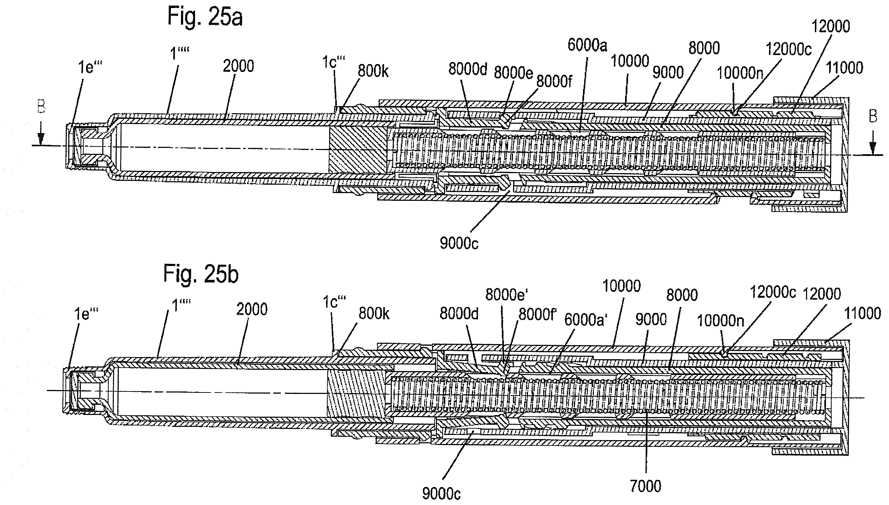

In der Figur 25a ist die vierte Ausführungsform der Injektionsvorrichtung in einer injektionsausgelösten Position des Dosierelements (9000) im Längsschnitt dargestellt, wobei die Figur 25b die Injektionsvorrichtung gemäss Figur 25a zeigt, wobei die Injektionsvorrichtung um die Längsachse B-B um 90° gedreht ist. Um die Injektion der festgelegten Dosis auszulösen, betätigt der Benutzer den Einstellknopf (1100), indem er den Einstellknopf (1100) axial relativ zu dem Gehäuse (10000) oder dem Halteelement (8000) axial in die distale Richtung bewegt. Durch diese axiale Bewegung gelangt die Dosierelementausparung (9000c) des Dosierelements (9000) über die erste nach aussen ragende Schnappnocke (8000e) des Halteelements (8000), derart dass die erste nach aussen ragende Schnappnocke (8000e) des Halteelements (8000) elastisch in die Dosierelementaussparung (9000c) des Dosierelements (9000) ausgelenkt wird, wobei sich das erste Halteglied (8000d) elastisch entspannt. Das Ausschüttelement (6000) bewegt sich während dem Ausschüttvorgang der festgelegten Dosis axial relativ zu dem Gehäuse (10000) in die distale Richtung. Während dem Ausschüttvorgang gelangt die Mantelfläche des Dosierelements (9000) über die zweite nach aussen ragende Schnappnocke (8000e') des zweiten Halteglieds (8000d') des Halteelements (8000), wobei das zweite Halteglied (8000d') mit der zweiten nach innen ragenden Schnappnocke (8000f') in die zweite Ausnehmung (6000a') des Ausschüttelements (6000) elastisch gespannt oder elastisch eingelenkt wird. Das Ausschüttelement (6000) bewegt sich so weit axial in die distale Richtung, bis eine proximale Stirnseite der zweiten nach innen ragenden Schnappnocke (8000f') gegen eine distale Stirnseite eines Verbindungsstegs zweier in axialer Richtung versetzt zueinander angeordneten zweiten Ausnehmungen (6000a') des Ausschüttelements (6000) in Anschlagkontakt gelangt. Während der Ausschüttbewegung dreht die Anzeigehülse (12000) nicht relativ zu dem Gehäuse (10000) oder dem Halteelement (8000). Wenn die letzte zur Verfügung stehende festgelegte Dosis aus der Injektionsvorrichtung ausgeschüttet ist, schlägt bei einem erneuten Einstellen oder Aufziehen einer festgelegten Dosis eine distale Stirnseite der Anzeigehülse (12000) gegen einen an der Mantelinnenfläche des Gehäuses (10000) angebrachten Anschlag, sodass keine weitere festgelegt Dosis eingestellt oder aufgezogen werden kann.

**Bezugszeichen:**

| | |
|---|---|
| 1; 1'; 1"; 1"'; 1"" | Karpulenhalter |
| 1a; 1a'; 1a" | Karpulenhalfernase |
| 1b; 1b'" | Karpulenhaltersteg |
| 1c; 1c'; 1c"; 1c'" | Karpulenhalteranschlag |
| 1d | Karpulenhalteraussparung |
| 1e; 1e'; 1e"; 1e'" | Nadelverbindungelement |
| 1f'" | Karpulenhalterschnapparm |
| 2; 20; 200; 2000 | Karpule |
| 20a | Karpulenkante |
| 3 | distales Spannelement |
| 3a | distaler Spannelementringsteg |
| 3b | distaler Spannelementhaken |
| 3c | distale Spannelementaussparung |
| 4; 40; 40' | Feder |
| 5; 50 | proximales Spannelement |
| 5a; 50a | proximaler Spannelementring |
| 5b; 50b | proximaler Spannelementschnapper |
| 6; 60; 60'; 600; 6000 | Ausschüttelement |
| 6a; 60a; 60a"; 600a; 6000a | erste Ausnehmung |
| 6a'; 60a'; 600a'; 6000a' | zweite Ausnehmung |
| 6000b | Ausschüttelementsteg |
| 7; 70; 70'; 700; 7000 | Feder |
| 8; 80; 80', 800; 8000 | Halteelement |
| 8a; 8000a | Halteelementnase |
| 8b; 80b; 80b'; 800b; 8000b | Halteelementsteg |
| 8c; 80c; 800c; 8000c | Halteeleementsteuerkurve |
| 8d; 80d; 80d"; 800d, 8000d | erstes Halteglied |
| 8d'; 80d'; 800d'; 8000d' | zweites Halteglied |
| 8e; 80e; 80e"; 800e; 8000e | erste nach aussen ragende Schnappnocke |
| 8e'; 80e'; 800e'; 8000e' | zweite nach aussen ragende Schnappnocke |
| 8f; 80f; 80f", 800f; 8000f | erste nach innen ragende Schnappnocke |
| 8f'; 80f; 800f'; 8000f' | zweite nach innen ragende Schnappnocke |
| 8g | Halteelementrippe |
| 8h; 80h | Halteelementanschlag |
| 8j; 80j | Halteelementnut |
| 80k; 800k; 8000k | Halteelementkante |
| 80l; 80l' 800l; 8000l | distale Halteelementschnapparm |
| 800m | proximale Halteelementschnapparm |
| 800n | Halteelementnocke |
| 8000o | Halteelementaussparung |
| 8000p | distale Halteelementnut |
| 9; 90; 90'; 900; 9000 | Dosierelement |
| 9a; 90a; 900a; 9000a | proximale Dosierelementnut |
| 9b; 90b | Dosierelementsteg |
| 9c; 90c; 9000c | Dosierelementaussparung |
| 90c; 900c | erste Dosierelementaussparung |
| 900c' | zweite Dosierelementaussparung |
| 9d | Dosierelementeinbuchtung |
| 9e, 90e; 9000e | Dosierelementnocke |
| 9f | distale Dosierelementmantelinnenfläche |
| 90g; 90g'; 900g | Dosierelementschnapparm |
| 90h | Dosierelementaussengewinde |
| 900j | proximale Dosierelementkante |
| 90k' | Dosierelementsteuerkurve |
| 10; 100; 1000; 10000 | Gehäuse |
| 10a | distale Gehäusenasenaussparung |
| 10b, 100b; 1000b; 10000b | distale Gehäusenut |
| 10c | distale Gehäusekante |
| 10d; 100d; 1000d; 10000d | proximale Gehäusenasenaussparung |
| 10e | proximale Gehäusenut, proximale Gehäuserampe |
| 10f; 100f | Gehäusesteuerkurve |
| 100g | Gehäuseringsteg |
| 100h | Gehäusringnasenaussparung |
| 100j; 1000j | Gehäuseaussparung |
| 100k | Gehäusering |
| 100l | Gehäuseringnase |
| 100m | Gehäuseringsteg |
| 100n; 1000n | Gehäuseinnengewinde |
| 1000; 1000o | distale Gehäuserampe |
| 100p; 1000p | Gehäuseinnengewindeanfang |
| 11; 110; 1100; 11000 | Einstellknopf |
| 11a | Einstellknopfring |
| 11b | Einstellknopfschnapparm |
| 11c | Einstellknopfsteg |
| 11d; 110d | Einstellknopfgriffsteg |
| 110e | Einstellknopfringnut |
| 1100f | Einstellknopfnocke |
| 1100g | Einstellknopfaussengewinde |
| 1100h | Einstellknopflängsnut oder Einstellknopframpe |
| 11000j | Einstellknopfsicherungselement |
| 120; 1200; 12000 | Anzeigehülse |
| 120a; 1200a | Anzeigeziffer |
| 120b; 12000b | Anzeigehülseschnapparm |
| 120c; 1200c; 12000c | Anzeigehülseaussengewinde |
| 120d; 1200d | Anzeigehülseinnengewinde |
| 120e; 1200e | Anzeigehülseaussengewindeende |
| 1200f; 12000f | Anzeigehülsesteg |
| 130 | Klick-Element |
| 130a | Klick-Elementsteuerkurve |

## Patentansprüche

1. Injektionsvorrichtung zur Aufdosierung und zur Ausschüttung einer festgelegten Dosis eines fluiden Produkts umfassend:
- ein Dosierelement (9; 90; 90'; 900; 9000) oder ein Einstellknopf (11; 110; 1100; 11000) zur Einstellung und zur Ausschüttung einer festgelegten Dosis mit einem Führungselement (9e; 90e; 1100f; 9000e)
- ein Halteelement (8; 80; 80'; 800, 8000) mit einem Führungsgegenelement (8c; 80c; 800c; 8000c) und einem ersten (8d; 80d; 80d"; 800d, 8000d) und einem zweiten Halteglied (8d'; 80d'; 800d'; 8000d'),
- ein Ausschüttelement (6; 60; 60'; 600; 6000) zur Ausschüttung einer festgelegten Dosis mit einer ersten Ausnehmungen (6a; 60a; 60a"; 600a; 6000a) zur Aufnahme des ersten Halteglieds (8d; 80d; 80d"; 800d, 8000d) oder zumindest eines Teils des ersten Halteglieds (8f; 80f; 800f; 8000f) des Halteelements (8; 80; 80', 800; 8000) und einer zweiten Ausnehmung (6a'; 60a'; 600a'; 6000a') zur Aufnahme des zweiten Halteglieds (8d'; 80d'; 800d'; 8000d') oder zumindest eines Teils des zweiten Halteglieds (8f; 80f; 800f'; 8000f') des Halteelements (8; 80; 80', 800; 8000),
- **dadurch gekennzeichnet, dass** die erste Ausnehmung (6a; 60a; 60a"; 600a; 6000a) des Ausschüftelements (6; 60; 60'; 600; 6000) mit dem ersten Halteglied (8d; 80d; 80d"; 800d, 8000d) oder mit zumindest einem Teil des ersten Halteglieds (8f; 80f; 800f; 8000f) des Halteelements (8; 80; 80', 800; 8000) und die zweite Ausnehmung (6a'; 60a'; 600a'; 6000a') des Ausschüttelement (6; 60; 60'; 600; 6000) mit dem zweiten Halteglied (8d'; 80d'; 800d'; 8000d') oder mit zumindest einem Teil des zweiten Halteglieds (8f'; 80f'; 800f'; 8000f') des Halteelements (8; 80; 80', 800; 8000) derart zusammen wirken, dass
- in einer Anfangsposition des Dosierelements (9; 90; 90'; 900; 9000) oder des Einstellknopfs (11; 110; 1100; 11000), in welcher das Dosierelement (9; 90; 90'; 900; 9000) oder der Einstellknopf (11; 110; 1100; 11000) in einer distalen Position ist, das erste Halteglied (8d; 80d; 80d"; 800d, 8000d) oder zumindest ein Teil des ersten Halteglieds (8f; 80f; 800f; 8000f) des Halteelements (8; 80; 80'; 800, 8000) mit der ersten Ausnehmung (6a; 60a; 60a"; 600a; 6000a) des Ausschüftelements (6; 60; 60'; 600; 6000) in Eingriff steht, wobei die Vorrichtung in der Anfangsposition des Dosierelements (9; 90; 90'; 900; 9000) oder des Einstellknopfs (11; 110; 1100; 11000) ferner eine vorgespannte Feder (7; 70; 70'; 700; 7000) aufweist, welche auf das Ausschüttelement (6; 60; 60'; 600; 6000) wirkt,
- wobei zur Einstellung oder zum Aufziehen der festgelegten Dosis der Injektionsvorrichtung das Dosierelement (9; 90; 90'; 900; 9000) oder der Einstellknopf (11; 110; 1100; 11000) von der distalen Position in eine proximale Position bewegt wird wobei
- in einer aufgezogenen Position des Dosierelements (9; 90; 90'; 900; 9000) oder des Einstellknopfs (11; 110; 1100; 11000), das erste Halteglied (8d; 80d; 80d"; 800d, 8000d) oder zumindest ein Teil des ersten Halteglieds (8f; 80f; 800f; 8000f) des Halteelements (8; 80; 80'; 800, 8000) mit der ersten Ausnehmung (6a; 60a; 60a"; 600a; 6000a) des Ausschüttelements (6; 60; 60'; 600; 6000) in Eingriff steht,
- wobei zur Ausschüttung der festgelegten Dosis der Injektionsvorrichtung das Dosierelement (9; 90; 90'; 900; 9000) oder der Einstellknopf (11; 110; 1100; 11000) von der proximalen Position in die distale Position bewegt wird wobei
- in einer ausgeschütteten Position des Dosierelements (9; 90; 90'; 900; 9000) oder des Einstellknopfs (11; 110; 1100; 11000) das zweite Halteglied (8d'; 80d'; 800d'; 8000d') oder zumindest ein Teil des zweiten Halteglieds des Halteelements (8f'; 80f'; 800f'; 8000f') mit der zweiten Ausnehmung des Ausschüftelements (6; 60; 60'; 600; 6000) in Eingriff steht, wobei in der Anfangsposition, in der aufgezogenen Position und in der ausgeschütteten Position des Dosierelements (9; 90; 90'; 900; 9000) oder des Einstellknopfs (11; 110; 1100; 11000) das Führungselement (9e; 90e; 1100f, 9000e) des Dosierelements (9; 90; 90'; 900; 9000) oder des Einstellknopfs (11; 110; 1100; 11000) in einem Führungseingriff oder in einer Kulissenführung mit dem Führungsgegenelement (8c; 80c; 800c; 8000c) des Halteelements (8; 80; 80', 800; 8000) steht,
wobei in der entsprechenden Position des Dosierelements die Ausnehmung des Dosierelements derart mit einem der Halteglieder oder zumindest einem Teil der Halteglieder des Halteelements zusammenwirken, dass das eine Halteglied oder zumindest ein Teil des Halteglieds des Halteelements zumindest teilweise in die Ausnehmung oder in die zumindest eine Ausnehmung ragt,
wobei das andere Halteglied des Halteelements gehindert wird, in die andere Aussparung oder in die zumindest eine andere Aussparung zu ragen.

2. Injektionsvorrichtung nach Anspruch 1, **dadurch gekennzeichnet, dass** die Injektionsvorrichtung ein Dosierelement (9; 90; 90'; 900; 9000) und ein Einstellknopf (11; 110; 1100; 11000) umfasst, wobei das Dosierelement (9; 90; 90'; 900; 9000) und der Einstellknopf (11; 110; 1100; 11000) mittelbar oder unmittelbar miteinander gekoppelt sind.

3. Injektionsvorrichtung nach einem der vorhergehenden Ansprüche, **dadurch gekennzeichnet, dass** das Dosierelement (9; 90; 90'; 900; 9000) ferner eine Aussparung (9c; 90c; 90c'; 900c, 900c'; 9000c) aufweist, welche derart ausgebildet ist, dass in der ausgeschütteten Position des Dosierelements (9; 90; 90'; 900; 9000) oder des Einstellknopfs (11; 110; 1100; 11000) das erste Halteglied (8d; 80d; 80d"; 800d, 8000d) oder zumindest ein Teil des ersten Halteglieds (8e; 80e; 80e"; 800e; 8000e) des Halteelements (8; 80; 80'; 800, 8000) mit der Aussparung (9c; 90c; 90c'; 900c, 900c'; 9000c) in Eingriff steht.

4. Injektionsvorrichtung nach einem der vorhergehenden Ansprüche, **dadurch gekennzeichnet, dass** das Dosierelement (9; 90; 90'; 900; 9000) in der Anfangsposition des Dosierelements (9; 90; 90'; 900; 9000) oder des Einstellknopfs (11; 110; 1100; 11000) mit dem ersten Halteglied (8d; 80d; 80d"; 800d, 8000d) oder mit zumindest einem Teil des ersten Halteglieds (8f; 80f; 800f; 8000f) des Halteelements (8; 80; 80'; 800, 8000) in Gleitkontakt steht.

5. Injektionsvorrichtung nach einem der vorhergehenden Ansprüche, **dadurch gekennzeichnet, dass** das Dosierelement (9; 90; 90'; 900; 9000) in der aufgezogenen Position des Dosierelements (9; 90; 90'; 900; 9000) oder des Einstellknopfs (11; 110; 1100; 11000) mit dem ersten Halteglied (8d; 80d; 80d"; 800d, 8000d) oder mit zumindest einem Teil des ersten Halteglieds (8f; 80f; 800f; 8000f) des Haltelements (8; 80; 80'; 800, 8000) in Gleitkontakt steht.

6. Injektionsvorrichtung nach einem der vorhergehenden Ansprüche, **dadurch gekennzeichnet, dass** das Dosierelement (9; 90; 90'; 900; 9000) oder der Einstellknopf (11; 110; 1100; 11000) relativ zu dem Halteelement (8; 80; 80'; 800, 8000) drehbar sind.

7. Injektionsvorrichtung nach einem der vorhergehenden Ansprüche, **dadurch gekennzeichnet, dass** das Ausschüttelement (6; 60; 60'; 600; 6000) hülsenförmig ausgebildet ist.

8. Injektionsvorrichtung nach einem der vorhergehenden Ansprüche, **dadurch gekennzeichnet, dass** die erste (6a; 60a; 60a"; 600a; 6000a) und die zweite Ausnehmung (6a'; 60a'; 600a'; 6000a') des Ausschüttelements (6; 60; 60'; 600; 6000) derart relativ zu dem ersten (8d; 80d; 80d"; 800d, 8000d) und dem zweiten Halteglied (8d'; 80d'; 800d'; 8000d') oder zu zumindest einem Teil des ersten (8f; 80f; 800f; 8000f) und des zweiten Halteglieds (8f'; 80f'; 800f'; 8000f') des Halteelements (8; 80; 80', 800; 8000) angeordnet sind, dass eine festgelegte Dosis aus der Vorrichtung ausschüttbar ist.

9. Injektionsvorrichtung nach einem der vorhergehenden Ansprüche, **dadurch gekennzeichnet, dass** die erste (6a; 60a; 60a"; 600a; 6000a) und die zweite Ausnehmung (6a'; 60a'; 600a'; 6000a') des Ausschüttelements (6; 60; 60'; 600; 6000) in Umfangsrichtung und vorzugsweise in axialer Richtung versetzt zueinander angeordnet sind.

10. Injektionsvorrichtung nach einem der vorhergehenden Ansprüche, **dadurch gekennzeichnet, dass** das Halteelement (8; 80; 80', 800; 8000) hülsenförmig ausgebildet ist.

11. Injektionsvorrichtung nach einem der vorhergehenden Ansprüche, **dadurch gekennzeichnet, dass** das erste (8d; 80d; 80d"; 800d, 8000d) und das zweite Halteglied (8d'; 80d'; 800d'; 8000d') oder zumindest ein Teil des ersten (8e; 80e; 80e"; 800e; 8000e; 8f; 80f; 800f; 8000f) und des zweiten Halteglieds (8e'; 80e'; 800e'; 8000e'; 8f'; 80f'; 800f'; 8000f') des Halteelements (8; 80; 80', 800; 8000) in Umfangsrichtung und vorzugsweise auf gleicher axialen Höhe oder auf unterschiedlicher axialen Höhe versetzt zueinander angeordnet sind.

12. Injektionsvorrichtung nach einem der vorhergehenden Ansprüche, **dadurch gekennzeichnet, dass** das erste (8d; 80d; 80d"; 800d, 8000d) und das zweite Halteglied (8d'; 80d'; 800d'; 8000d') oder zumindest ein Teil des ersten (8e; 80e; 80e"; 800e; 8000e; 8f; 80f; 800f; 8000f) und des zweiten Halteglieds (8e'; 80e'; 800e'; 8000e'; 8f'; 80f'; 800f'; 8000f') des Halteelements (8; 80; 80', 800; 8000) elastisch ausgebildet sind.

13. Injektionsvorrichtung nach einem der vorhergehenden Ansprüche, **dadurch gekennzeichnet, dass** das erste (8d; 80d; 80d"; 800d, 8000d) und das zweite Halteglied (8d'; 80d'; 800d'; 8000d') des Halteelements (8; 80; 80', 800; 8000) als Schnapparm oder Schnappzunge ausgebildet sind und vorzugsweise mindestens einen Schnapphaken oder Schnappnocke oder sonstigen Vorsprung (8e; 80e; 80e"; 800e; 8000e; 8f; 80f; 800f; 8000f; 8e'; 80e'; 800e'; 8000e';8f; 80f; 800f; 8000f') umfassen.

14. Injektionsvorrichtung nach einem der vorhergehenden Ansprüche, **dadurch gekennzeichnet, dass** das Führungselement (9e; 90e;1100f; 9000e) des Dosierelements (9; 90; 90'; 900; 9000) oder des Einstellknopfs (11; 110; 1100; 11000) als Führungsnocke und das Führungsgegenelement (8c; 80c; 800c; 8000c) des Halteelements (8; 80; 80', 800; 8000) als Kulissenführung oder das Führungselement (9e; 90e; 1100f; 9000e) des Dosierelements (9; 90; 90'; 900; 9000) oder des Einstellknopfs (11; 110; 1100; 11000) als Kulissenführung und das Führungsgegenelement (8c; 80c; 800c; 8000c) des Halteelements (8; 80; 80', 800; 8000) als Führungsnocke ausgebildet sind.

## Claims

1. Injection device for dosing and for dispensing a set of a fluid product, comprising:
- a dosing element (9; 90; 90'; 900; 9000) or an adjusting knob (11; 110; 1100; 11000) for adjusting and for dispensing a set dose with a guide element (9e; 90e; 1100f; 9000e)
- a holding element (8; 80; 80'; 800, 8000) with a guide counterelement (8c; 80c; 800c; 8000c) and a first (8d; 80d; 80d"; 800d, 8000d) and a second holding member (8d'; 80d'; 800d'; 8000d'),
- a dispensing element (6; 60; 60'; 600; 6000) for dispensing a set dose with a first recesses (6a; 60a; 60a"; 600a; 6000a) for receiving the first holding member (8d; 80d; 80d"; 800d, 8000d) or at least a part of the first holding member (8f; 80f; 800f; 8000f) of the holding element (8; 80; 80', 800; 8000) and a second recess (6a'; 60a'; 600a'; 6000a') for receiving the second holding member (8d'; 80d'; 800d'; 8000d') or at least a part of the second holding member (8f; 80f; 800f; 8000f) of the holding element (8; 80; 80', 800; 8000),
- **characterised in that** the first recess (6a; 60a; 60a"; 600a; 6000a) of the dispensing element (6; 60; 60'; 600; 6000) cooperates with the first holding member (8d; 80d; 80d"; 800d, 8000d) or with at least a part of the first holding member (8f; 80f; 800f; 8000f) the holding element (8; 80; 80', 800; 8000) and the second recess (6a'; 60a'; 600a'; 6000a') of the dispensing element (6; 60; 60'; 600; 6000) with the second holding member (8d'; 80d'; 800d'; 8000d') or with at least a part of the second holding member (8f'; 80f; 800f; 8000f) of the holding elements (8; 80; 80', 800; 8000) in such a way that
- the first holding member (8d; 80d; 80d"; 800d, 8000d) or at least a part of the first holding member (8f; 80f; 800f; 8000f) of the holding element (8; 80; 80'; 800, 8000) engages the first recess (6a; 60a; 60a"; 600a; 6000a) of the dispensing element (6; 60; 60'; 600; 6000) in a starting position of the dosing element (9; 90; 90'; 900; 9000) or of the adjusting knob (11; 110;
1100; 11000), in which the dosing element (9; 90; 90'; 900; 9000) or the adjusting knob (11; 110; 1100; 11000) are in a distal position, wherein the device further has a pre-tensioned spring (7; 70; 70'; 700; 7000), which acts on the dispensing element (6; 60; 60'; 600; 6000) in the starting position of the dosing element (9; 90; 90'; 900; 9000) or the adjusting knob (11; 110;1100; 11000),
- wherein the injection device of the dosing element (9; 90; 90'; 900; 9000) or the adjusting knob (11; 110; 1100; 11000) are moved from the distal position into a proximate position for adjusting or loading the set dose,
wherein the first holding member (8d; 80d; 80d"; 800d, 8000d) or at least a part of the first holding member (8f; 80f; 800f; 8000f) of the holding elements (8; 80; 80'; 800,8000) engage the first recess (6a; 60a; 60a"; 600a; 6000a) of the dispensing element (6; 60; 60'; 600; 6000) in a loaded position of the dosing element (9; 90; 90'; 900; 9000) or the adjusting knob (11; 110;1100; 11000),
- wherein the dosing element (9; 90; 90'; 900; 9000) or the adjusting knob (11; 110; 1100; 11000) is moved from the proximal position into the distal position for dispensing the set dose of the injection device, wherein
- the second holding member (8d'; 80d'; 800d'; 8000d') or at least a part of the second holding member of the holding element (8f'; 80f'; 800f'; 8000f') engages the second recess of the dispensing element (6; 60; 60'; 600; 6000) in a dispensing position of the dosing element (9; 90; 90'; 900; 9000) or the adjusting knob (11; 110; 1100; 11000), wherein the guide element (9e; 90e; 1100f; 9000e) of the dosing element (9; 90; 90'; 900; 9000) or the adjusting knob (11; 110; 1100; 11000) are in guiding engagement or sliding block guidance with the guide counterelement (8c; 80c; 800c; 8000c) of the holding element (8; 80; 80', 800; 8000) in the starting position, in the loaded position and in the dispensing position of the dosing elements (9; 90; 90'; 900; 9000) or the adjusting knob (11; 110; 1100; 11000),
wherein the recess of the dosing element cooperates with one of the holding members or at least a part of the holding member of the holding element in the corresponding position of the dosing elements in such a way that a holding member or at least a part of the holding member of the holding element at least partially projects into the recess or into the at least a recess, wherein the other holding member of the holding element is prevented from projecting into the other opening or into the at least one other opening.

2. Injection device according to claim 1, **characterised in that** the injection device comprises a dosing element (9; 90; 90'; 900; 9000) and an adjusting knob (11; 110; 1100; 11000), wherein the dosing element (9; 90; 90'; 900; 9000) and the adjusting knob (11; 110; 1100; 11000) are indirectly or directly coupled with each other.

3. Injection device according to one of the preceding claims, **characterised in that** the dosing element (9; 90; 90'; 900; 9000) further has an opening (9c; 90c; 90c'; 900c, 900c'; 9000c), which is designed in such a way that the first holding member (8d; 80d; 80d"; 800d, 8000d) or at least a part of the first holding member (8e; 80e; 80e"; 800e; 8000e) of the holding element (8; 80; 80'; 800, 8000) engages the opening (9c; 90c; 90c'; 900c, 900c'; 9000c) in the dispensing position of the dosing element (9; 90; 90'; 900; 9000) or the adjusting knob (11; 110; 1100; 11000).

4. Injection device according to one of the preceding claims, **characterised in that** the dosing element (9; 90; 90'; 900; 9000) is in gliding contact with the first holding member (8d; 80d; 80d"; 800d, 8000d) or with at least a part of the first holding member (8f; 80f; 800f; 8000f) of the holding element (8; 80; 80'; 800, 8000) in the starting position of the dosing element (9; 90; 90'; 900; 9000) or the adjusting knob (11; 110; 1100; 11000).

5. Injection device according to one of the preceding claims, **characterised in that** the dosing element (9; 90; 90'; 900; 9000) is in gliding contact with the first holding member (8d; 80d; 80d"; 800d, 8000d) or with at least a part of the first holding member (8f; 80f; 800f; 8000f) of the holding element (8; 80; 80'; 800, 8000) in the loaded position of the dosing element (9; 90; 90'; 900; 9000) or the adjusting knob (11; 110; 1100; 11000).

6. Injection device according to one of the preceding claims, **characterised in that** the dosing element (9; 90; 90'; 900; 9000) or the adjusting knob (11; 110; 1100; 11000) can be rotated relative to the holding element (8; 80; 80'; 800, 8000).

7. Injection device according to one of the preceding claims, **characterised in that** the dispensing element (6; 60; 60'; 600; 6000) is of a sleeve-shaped design.

8. Injection device according to one of the preceding claims, **characterised in that** the first (6a; 60a; 60a"; 600a; 6000a) and the second recess (6a'; 60a'; 600a'; 6000a') of the dispensing element (6; 60; 60'; 600; 6000) are arranged relative to the first (8d; 80d; 80d"; 800d, 8000d) and the second holding member (8d'; 80d'; 800d'; 8000d') or to at least a part of the first (8f; 80f; 800f; 8000f) and the second holding member (8f'; 80f'; 800f'; 8000f') of the holding element (8; 80; 80', 800; 8000) in such a way that a set dose can be dispensed from the device.

9. Injection device according to one of the preceding claims, **characterised in that** the first (6a; 60a; 60a"; 600a; 6000a) and the second recess (6a'; 60a'; 600a'; 6000a') of the dispensing element (6; 60; 60'; 600; 6000) are arranged offset from each other in a circumferential direction and preferably in an axial direction.

10. Injection device according to one of the preceding claims, **characterised in that** the holding element (8; 80; 80', 800; 8000) is of a sleeve-shaped design.

11. Injection device according to one of the preceding claims, **characterised in that** the first (8d; 80d; 80d"; 800d, 8000d) and the second holding member (8d'; 80d'; 800d'; 8000d') or at least a part of the first (8e; 80e; 80e"; 800e; 8000e; 8f; 80f; 800f; 8000f) and the second holding member (8e'; 80e'; 800e'; 8000e'; 8f'; 80f'; 800f'; 8000f') of the holding element (8; 80; 80', 800; 8000) are of an elastic design in circumference direction and preferably arranged offset to each other at the same axial height or at different axial heights.

12. Injection device according to one of the preceding claims, **characterised in that** the first (8d; 80d; 80d"; 800d, 8000d) and the second holding member (8d'; 80d'; 800d'; 8000d') or at least a part of the first (8e; 80e; 80e"; 800e; 8000e; 8f; 80f; 800f; 8000f) and the second holding member (8e'; 80e'; 800e'; 8000e';8f'; 80f'; 800f'; 8000f') of the holding element (8; 80; 80', 800; 8000) are of an elastic design.

13. Injection device according to one of the preceding claims, **characterised in that** the first (8d; 80d; 80d"; 800d, 8000d) and the second holding member (8d'; 80d'; 800d'; 8000d') of the holding element (8; 80; 80', 800; 8000) are designed as a snatch arm or a snatch tongue and preferably comprise at least one snatch hook or snatch cam or other projection (8e; 80e; 80e"; 800e; 8000e; 8f; 80f; 800f; 8000f; 8e'; 80e'; 800e'; 8000e'; 8f'; 80f'; 800f'; 8000f').

14. Injection device according to one of the preceding claims, **characterised in that** the guide element (9e; 90e; 1100f; 9000e) of the dosing element (9; 90; 90'; 900; 9000) or the adjusting knob (11; 110; 1100; 11000) are designed as a guiding cam and the guide counterelement (8c; 80c; 800c; 8000c) of the holding element (8; 80; 80', 800; 8000) is designed as a sliding block guidance or the guide element (9e; 90e; 1100f; 9000e) of the dosing element (9; 90; 90'; 900; 9000) or the adjusting knob (11; 110; 1100; 11000) as a sliding block guidance and the guide counterelement (8c; 80c; 800c; 8000c) of the holding element (8; 80; 80', 800; 8000) as a guiding cam.

## Revendications

1. Dispositif d'injection servant à doser et à distribuer une dose fixée d'un produit liquide comprenant :
- un élément de dosage (9 ; 90 ; 90' ; 900 ; 9000) ou un bouton de réglage (11 ; 110 ; 1100 ; 11000) servant à régler et à distribuer une dose fixée pourvu d'un élément de guidage (9e ; 90e ; 1100f ; 9000e),
- un élément de retenue (8 ; 80 ; 80' ; 800, 8000) pourvu d'un élément de guidage complémentaire (8c ; 80c ; 800c ; 8000c) et d'un premier (8d ; 80d ; 80d" ; 800d, 8000d) et d'un deuxième organe de retenue (8d' ; 80d' ; 800d' ; 8000d'),
- un élément de distribution (6 ; 60 ; 60' ; 600 ; 6000) servant à distribuer une dose fixée pourvu d'un premier évidement (6a ; 60a ; 60a" ; 600a ; 6000a) servant à recevoir le premier organe de retenue (8d ; 80d ; 80d" ; 800d, 8000d) ou au moins une partie du premier organe de retenue (8f ; 80f ; 800f ; 8000f) de l'élément de retenue (8 ; 80 ; 80', 800 ; 8000) et d'un deuxième évidement (6a' ; 60a' ; 600a' ; 6000a') servant à recevoir le deuxième organe de retenue (8d' ; 80d' ; 800d' ; 8000d') ou au moins une partie du deuxième organe de retenue (8f' ; 80f' ; 800f' ; 8000f') de l'élément de retenue (8 ; 80 ; 80', 800 ; 8000),
- **caractérisé en ce que** le premier évidement (6a ; 60a ; 60a" ; 600a ; 6000a) de l'élément de distribution (6 ; 60 ; 60' ; 600 ; 6000) coopère avec le premier organe de retenue (8d ; 80d ; 80d" ; 800d, 8000d) ou avec au moins une partie du premier organe de retenue (8f ; 80f ; 800f ; 8000f) de l'élément de retenue (8 ; 80 ; 80', 800 ; 8000) et le deuxième évidement (6a' ; 60a' ; 600a' ; 6000a') de l'élément de distribution (6 ; 60 ; 60' ; 600 ; 6000) coopère avec le deuxième organe de retenue (8d' ; 80d' ; 800d' ; 8000d') ou avec au moins une partie du deuxième organe de retenue (8f' ; 80f' ; 800f' ; 8000f') de l'élément de retenue (8 ; 80 ; 80', 800 ; 8000) de telle manière que
- dans une position de départ de l'élément de dosage (9 ; 90 ; 90' ; 900 ; 9000) ou du bouton de réglage (11 ; 110 ; 1100 ; 11000), dans laquelle l'élément de dosage (9 ; 90 ; 90' ; 900 ; 9000) ou le bouton de réglage (11 ; 110 ; 1100 ; 11000) est dans une position distale, le premier organe de retenue (8d ; 80d ; 80d" ; 800d, 8000d) ou au moins une partie du premier organe de retenue (8f ; 80f ; 800f ; 8000f) de l'élément de retenue (8 ; 80 ; 80' ; 800, 8000) est en prise avec le premier évidement (6a ; 60a ; 60a" ; 600a ; 6000a) de l'élément de distribution (6 ; 60 ; 60' ; 600 ; 6000), dans lequel le dispositif présente, dans la position de départ de l'élément de dosage (9 ; 90 ; 90' ; 900 ; 9000) ou du bouton de réglage (11 ; 110 ; 1100 ; 11000), en outre un ressort (7 ; 70 ; 70' ; 700 ; 7000) précontraint, qui agit sur l'élément de distribution (6 ; 60 ; 60' ; 600 ; 6000),
- dans lequel afin de régler ou de chauffer la dose fixée du dispositif d'injection, l'élément de dosage (9 ; 90 ; 90' ; 900 ; 9000) ou le bouton de réglage (11 ; 110 ; 1100 ; 11000) est déplacé depuis la position distale dans la position proximale, dans lequel
- dans une position tirée de l'élément de dosage (9 ; 90 ; 90' ; 900 ; 9000) ou du bouton de réglage (11 ; 110 ; 1100 ; 11000), le premier organe de retenue (8d ; 80d ; 80d" ; 800d, 8000d) ou au moins une partie du premier organe de retenue (8f ; 80f ; 800f ; 8000f) de l'élément de retenue (8 ; 80 ; 80' ; 800, 8000) est en prise avec le premier évidement (6a ; 60a ; 60a" ; 600a ; 6000a) de l'élément de distribution (6 ; 60 ; 60' ; 600 ; 6000),
- dans lequel afin de distribuer la dose fixée du dispositif d'injection, l'élément de dosage (9 ; 90 ; 90' ; 900 ; 9000) ou le bouton de réglage (11 ; 110 ; 1100 ; 11000) est déplacé depuis la position proximale dans la position distale, dans lequel
- dans une position de distribution de l'élément de dosage (9 ; 90 ; 90' ; 900 ; 9000) ou du bouton de réglage (11 ; 110 ; 1100 ; 11000), le deuxième organe de retenue (8d' ; 80d' ; 800d' ; 8000d') ou au moins une partie du deuxième organe de retenue de l'élément de retenue (8f' ; 80f' ; 800f' ; 8000f') est en prise avec le deuxième évidement de l'élément de distribution (6 ; 60 ; 60' ; 600 ; 6000), dans lequel dans la position de départ, dans la position tirée et dans la position de distribution de l'élément de dosage (9 ; 90 ; 90' ; 900 ; 9000) ou du bouton de réglage (11 ; 110 ; 1100; 11000), l'élément de guidage (9e ; 90e; 1100f ; 9000e) de l'élément de dosage (9 ; 90 ; 90' ; 900 ; 9000) ou du bouton de réglage (11 ; 110 ; 1100 ; 11000) est en prise de guidage ou dans un guidage coulissant avec l'élément de guidage complémentaire (8c ; 80c ; 800c ; 8000c) de l'élément de retenue (8 ; 80 ; 80' ; 800 ; 8000),
dans lequel dans la position correspondante de l'élément de dosage, l'évidement de l'élément de dosage coopère avec un des organes de retenue ou au moins avec une partie des organes de retenue de l'élément de retenue de telle manière qu'un organe de retenue ou au moins une partie de l'organe de retenue de l'élément de retenue dépasse au moins en partie dans l'évidement ou dans l'au moins un évidement, dans lequel l'autre organe de retenue de l'élément de retenue est empêché de dépasser dans l'autre renfoncement ou dans l'au moins un autre renfoncement.

2. Dispositif d'injection selon la revendication 1, **caractérisé en ce que** le dispositif d'injection comprend un élément de dosage (9 ; 90 ; 90' ; 900 ; 9000) et un bouton de réglage (11 ; 110 ; 1100 ; 11000), dans lequel l'élément de dosage (9 ; 90 ; 90' ; 900 ; 9000) et le bouton de réglage (11 ; 110 ; 1100 ; 11000) sont couplés l'un à l'autre de manière indirecte ou de manière directe.

3. Dispositif d'injection selon l'une quelconque des revendications précédentes, **caractérisé en ce que** l'élément de dosage (9 ; 90 ; 90' ; 900 ; 9000) présente en outre un renfoncement (9c, 90c ; 90c' ; 900c, 900c' ; 9000c), qui est réalisé de telle manière que dans la position de distribution de l'élément de dosage (9 ; 90 ; 90' ; 900 ; 9000) ou du bouton de réglage (11 ; 110 ; 1100 ; 11000), le premier organe de retenue (8d ; 80d ; 80d" ; 800d, 8000d) ou au moins une partie du premier organe de retenue (8e ; 80e ; 80e" ; 800e ; 8000e) de l'élément de retenue (8 ; 80 ; 80' ; 800, 8000) est en prise avec le renfoncement (9c ; 90c ; 90c' ; 900c, 900c' ; 9000c).

4. Dispositif d'injection selon l'une quelconque des revendications précédentes, **caractérisé en ce que** l'élément de dosage (9 ; 90 ; 90' ; 900 ; 9000) est, dans la position de départ de l'élément de dosage (9 ; 90 ; 90' ; 900 ; 9000) ou du bouton de réglage (11 ; 110 ; 1100 ; 11000), en contact glissant avec le premier organe de retenue (8d ; 80d ; 80d" ; 800d, 8000d) ou avec au moins une partie du premier organe de retenue (8f ; 80f ; 800f ; 8000f) de l'élément de retenue (8 ; 80 ; 80' ; 800, 8000).

5. Dispositif d'injection selon l'une quelconque des revendications précédentes, **caractérisé en ce que** l'élément de dosage (9 ; 90 ; 90' ; 900 ; 9000) est, dans la position tirée de l'élément de dosage (9 ; 90 ; 90' ; 900 ; 9000) ou du bouton de réglage (11 ; 110 ; 1100 ; 11000), en contact glissant avec le premier organe de retenue (8d ; 80d ; 80d" ; 800d, 8000d) ou avec au moins une partie du premier organe de retenue (8f ; 80f ; 800f ; 8000f) de l'élément de retenue (8 ; 80 ; 80' ; 800, 8000).

6. Dispositif d'injection selon l'une quelconque des revendications précédentes, **caractérisé en ce que** l'élément de dosage (9 ; 90 ; 90' ; 900 ; 9000) ou le bouton de réglage (11 ; 110 ; 1100; 11000) peuvent être tournés par rapport à l'élément de retenue (8 ; 80 ; 80' ; 800, 8000).

7. Dispositif d'injection selon l'une quelconque des revendications précédentes, **caractérisé en ce que** l'élément de distribution (6 ; 60 ; 60' ; 600 ; 6000) est réalisé de manière à présenter une forme de douille.

8. Dispositif d'injection selon l'une quelconque des revendications précédentes, **caractérisé en ce que** le premier (6a ; 60a ; 60a" ; 600a ; 6000a) et le deuxième évidement (6a' ; 60a' ; 600a' ; 6000a') de l'élément de distribution (6 ; 60 ; 60' ; 600 ; 600) sont disposés par rapport au premier (8d ; 80d ; 80d" ; 800d, 8000d) et au deuxième organe de retenue (8d' ; 80d' ; 800d' ; 8000d') ou par rapport à au moins une partie du premier (8f ; 80f ; 800f ; 8000f) et du deuxième organe de retenue (8f' ; 80f' ; 800f' ; 8000f') de l'élément de retenue (8 ; 80 ; 80' ; 800 ; 8000) de telle manière qu'une dose fixée peut être distribuée depuis le dispositif.

9. Dispositif d'injection selon l'une quelconque des revendications précédentes, **caractérisé en ce que** le premier (6a ; 60a ; 60a" ; 600a ; 6000a) et le deuxième évidement (6a' ; 60a' ; 600a' ; 6000a') de l'élément de distribution (6 ; 60 ; 60' ; 600 ; 6000) sont disposés dans la direction périphérique et de préférence de manière décalée l'un par rapport à l'autre dans une direction axiale.

10. Dispositif d'injection selon l'une quelconque des revendications précédentes, **caractérisé en ce que** l'élément de retenue (8 ; 80 ; 80', 800 ; 8000) est réalisé de manière à présenter une forme de douille.

11. Dispositif d'injection selon l'une quelconque des revendications précédentes, **caractérisé en ce que** le premier (8d ; 80d ; 80d" ; 800d, 8000d) et le deuxième organe de retenue (8d' ; 80d' ; 800d' ; 8000d') ou au moins une partie du premier (8e ; 80e ; 80e" ; 800e ; 8000e ; 8f ; 80f ; 800f ; 8000f) et du deuxième organe de retenue (8e' ; 80e' ; 800e' ; 8000e' ; 8f' ; 80f' ; 800f' ; 8000f') de l'élément de retenue (8 ; 80 ; 80' ; 800 ; 8000) sont disposés dans la direction périphérique et de préférence de manière décalée les uns par rapport aux autres à une hauteur axiale identique ou à différentes hauteurs axiales.

12. Dispositif d'injection selon l'une quelconque des revendications précédentes, **caractérisé en ce que** le premier (8d ; 80d ; 80d" ; 800d, 8000d) et le deuxième organe de retenue (8d' ; 80d' ; 800d' ; 8000d') ou au moins une partie du premier (8e ; 80e ; 80e" ; 800e ; 8000e ; 8f ; 80f ; 800f ; 8000f) et du deuxième organe de retenue (8e' ; 80e' ; 800e' ; 8000e' ; 8f' ; 80f' ; 800f' ; 8000f') de l'élément de retenue (8 ; 80 ; 80' ; 800 ; 8000) sont réalisés de manière élastique.

13. Dispositif d'injection selon l'une quelconque des revendications précédentes, **caractérisé en ce que** le premier (8d ; 80d ; 80d" ; 800d, 8000d) et le deuxième organe de retenue (8d' ; 80d' ; 800d' ; 8000d') de l'élément de retenue (8 ; 80 ; 80', 800 ; 8000) sont réalisés sous la forme d'un bras à déclic ou d'une languette à déclic et comprennent de préférence au moins un crochet à déclic ou une came à déclic ou toute autre partie faisant saillie (8e ; 80e ; 80e" ; 800e ;8000e ; 8f ; 80f ; 800f ; 8000f ; 8e' ; 80e' ; 800e' ; 8000e' ; 8f' ; 80f' ; 800f' ; 8000f').

14. Dispositif d'injection selon l'une quelconque des revendications précédentes, **caractérisé en ce que** l'élément de guidage (9e ; 90e ; 1100f ; 9000e) de l'élément de dosage (9 ; 90 ; 90' ; 900 ; 9000) ou du bouton de réglage (11 ; 110 ; 1100 ; 11000) est réalisé sous la forme d'une came de guidage et l'élément de guidage complémentaire (8c ; 80c ; 800c ; 8000c) de l'élément de retenue (8 ; 80 ; 80' ; 800 ; 8000) est réalisé sous la forme d'un guidage coulissant ou l'élément de guidage (9e ; 90e ; 1100f ; 9000e) de l'élément de dosage (9 ; 90 ; 90' ; 900 ; 9000) ou du bouton de réglage (11 ; 110 ; 1100 ; 11000) est réalisé sous la forme d'un guidage coulissant et l'élément de guidage complémentaire (8c ; 80c ; 800c ; 8000c) de l'élément de retenue (8 ; 80 ; 80' ; 800 ; 8000) est réalisé sous la forme d'une came de guidage.
